# EUROPÄISCHE PATENTSCHRIFT

(11) **EP 1 482 019 B1**
(45) Veröffentlichungstag und Bekanntmachung des Hinweises auf die Patenterteilung: **27.09.2006**
(21) Anmeldenummer: 04010357.4
(22) Anmeldetag: 30.04.2004
(51) Int. Cl.: C09K 19/34, C09K 19/30, C07D 309/20, C07D 309/32

(54) **Pyranderivate**
Pyran derivatives
Dérivés de pyrane

(30) Priorität: 27.05.2003 DE 10324312
(43) Veröffentlichungstag der Anmeldung: 01.12.2004
(73) Patentinhaber: Merck Patent GmbH, 64293 Darmstadt (DE)
(72) Erfinder: Poetsch, Eike Dr., 64367 Mühltal (DE); Binder, Werner, 64807 Dieburg (DE); Meyer, Volker, 64846 Gross-Zimmern (DE); Heckmeier, Michael Dr., 69502 Hemsbach (DE); Lüssem, Georg Dr., 85238 Petershausen (DE)

(56) Entgegenhaltungen:
- EP-A- 0 409 066
- DE-A- 3 306 960
- DE-A- 19 640 618
- US-A- 5 480 580

## Beschreibung

Die Erfindung betrifft Pyranderivate, Verfahren zur ihrer Herstellung und Derivatisierung sowie ihre Verwendung in flüssigkristallinen Medien.

Pyranderivate spielen eine bedeutende Rolle in Chemie und Pharmazie, unter anderem als Inhaltsstoffe von natürlichen und synthetischen Aromastoffen, in Arzneimitteln und in flüssigkristallinen Materialien. Allerdings ist der präparative Zugang zu vielen Pyranen, insbesondere solchen mit 2,5-Disubstitution, gegenwärtig limitiert und beschränkt sich oft auf die Derivatisierung von Kohlenhydraten, die Pyranoseringeinheiten aufweisen. Viele theoretisch denkbaren Pyranderivate sind bislang synthetisch nicht zugänglich.

Aus den Dokumenten DE 3306960 A1 und DE 19640618 A1 sind flüssigkristalline Verbindungen bekannt, die ein Tetrahydropyran, ein 2-Oxotetrahydropyran oder ein 2H-5,6-Dihydropyran beinhalten.

Der vorliegenden Erfindung liegt daher als eine Aufgabe zugrunde, neue Pyranderivate bereitzustellen, die über technisch nützliche Eigenschaften verfügen beziehungsweise als Ausgangsverbindungen zur effizienten Synthese weiterer Pyranderivate dienen können.

Gelöst wird diese Aufgabe durch Verbindungen der allgemeinen Formel I 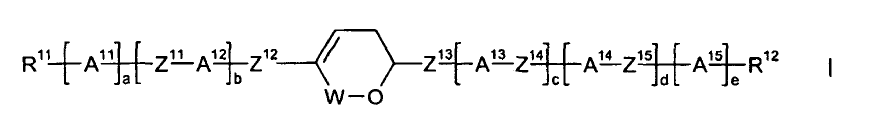 wobei
- a, b, c, d und e: jeweils unabhängig voneinander 0 oder 1 sind;
- W: -CH₂- oder-C(=O)- bedeutet;
- R¹¹: H, einen unsubstituierten oder einfach oder mehrfach gleich oder verschieden mit Halogen oder -CN substituierten Alkylrest mit 1 bis 15 Kohlenstoffatomen bedeutet, wobei in diesem Rest auch eine oder mehrere CH₂-Gruppen durch -C≡C-, -CH=CH-, -O-, -S-, -C(O)-O- und/oder -O-C(O)- so ersetzt sein können, dass Heteroatome (O, S) nicht direkt miteinander verknüpft sind;
- R¹²: H, Halogen, -CN, -NCS, Aralkyl, O-Aralkyl oder einen unsubstituierten oder einfach oder mehrfach gleich oder verschieden mit Halogen oder -CN substituierten Alkylrest mit 1 bis 15 Kohlenstoffatomen bedeutet, wobei in diesem Rest auch eine oder mehrere CH₂-Gruppen durch -C≡C-, -CH=CH-, -O-, -S-, -C(O)-O- und/oder -O-C(O)- so ersetzt sein können, dass Heteroatome (O, S) nicht direkt miteinander verknüpft sind;
- Z¹¹: eine Einfachbindung, -CH₂-, -CH₂CH₂-, -CF₂CH₂-, -CH₂CF₂-, -CF₂CF₂-, -CH=CH- oder -C≡C- bedeutet;
- Z¹²: eine Einfachbindung, -CH₂-, -CH₂CH₂-, -CF₂CH₂-, -CH₂CF₂- oder -CF₂CF₂- bedeutet;
- Z¹³, Z¹⁴ und Z¹⁵: jeweils unabhängig voneinander eine Einfachbindung, -CH₂CH₂-, -CF₂CH₂-, -CH₂CF₂-, -CF₂CF₂-, -CH=CH-, -C≡C-, -CH₂O-, -CF₂O-, -C(O)- oder -C(O)-O- bedeuten;
- A¹¹ und A¹²: unabhängig voneinander für 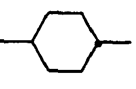 oder 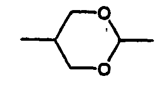 stehen;
- A¹³ und A¹⁴: unabhängig voneinander für 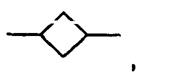 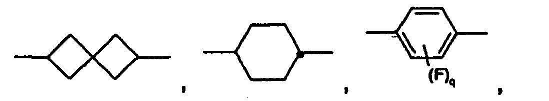 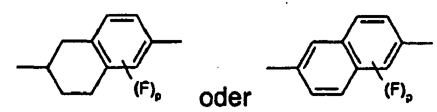 stehen;
- A¹⁵: für 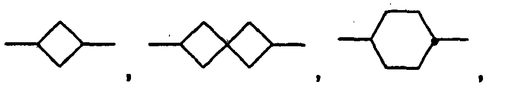 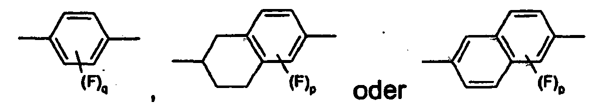 steht oder
- A¹⁵-R¹²: zusammen für 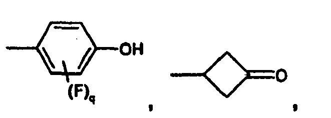 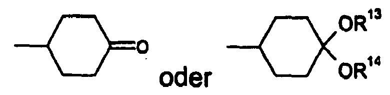 steht; oder
- Z¹³-[-A¹³-Z¹⁴-]_{c}-[-A¹⁴-Z¹⁵-]_{d}-[-A¹⁵-]ₑ-R¹²: für 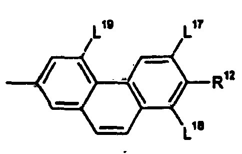 oder 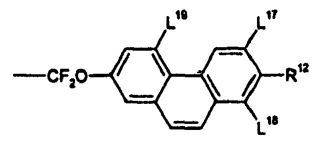 steht, wobei R¹² wie oben definiert ist und L¹⁷, L¹⁸ und L¹⁹ unabhängig voneinander H oder F bedeuten;
- q: 0, 1, 2, 3 oder 4 ist;
- p: 0, 1, 2 oder 3 ist;
- R¹³ und R¹⁴: unabhängig voneinander einen Alkanylrest mit 1 bis 7 Kohlenstoffatomen bedeuten oder zusammen für eine Alkylenbrücke mit 2 bis 7 Kohlenstoffatomen stehen;
mit der Maßgabe,
dass für den Fall der direkten Verknüpfung von Z¹³ und R¹² zu -Z¹³-R¹² R¹² H, Aralkyl, Alkanyl oder Alkenyl bedeutet, wenn Z¹³ -C(=O)-O- oder -C(=O)-bedeutet, und Z¹³ nicht -CH₂O oder -CF₂O- bedeutet;
dass für den Fall der direkten Verknüpfung von Z¹⁴ und R¹² zu -Z¹⁴-R¹² R¹² H, Aralkyl, Alkanyl oder Alkenyl bedeutet, wenn Z¹⁴ -C(=O)-O- oder -C(=O)-bedeutet, und Z¹⁴ nicht -CH₂O- oder -CF₂O- bedeutet;
dass für den Fall der direkten Verknüpfung von Z¹⁵ und R¹² zu -Z¹⁵-R¹² R¹² H, Aralkyl, Alkanyl oder Alkenyl bedeutet, wenn Z¹⁵ -C(=O)-O- oder -C(=O)-bedeutet, und Z¹⁵ nicht -CH₂O- oder -CF₂O- bedeutet;
dass R¹¹ nicht H bedeutet, wenn zugleich a und b beide null sind und Z¹² eine Einfachbindung bedeutet;
dass R¹¹ nicht CH₃ bedeutet, wenn zugleich a und b beide null sind, Z¹² und Z¹³ jeweils eine Einfachbindung bedeuten, W -CH₂- bedeutet und -[-A¹³-Z¹⁴-]_{c}-[-A¹⁴-Z¹⁵-]_{d}-[-A¹⁵-]ₑ-R¹² für unsubstituiertes Phenyl steht.

Die erfindungsgemäßen Verbindungen der Formel I finden auf Grund ihrer Eigenschaften Verwendung in flüssigkristallinen Medien beziehungsweise dienen als Ausgangsverbindungen zur effizienten Synthese weiterer Pyranverbindungen, insbesondere solchen mit mesogenen Eigenschaften. Bevorzugt sind die erfindungsgemäßen Verbindungen der Formel I mesogen, insbesondere flüssigkristallin.

Im Zusammenhang mit der vorliegenden Erfindung bedeutet der Ausdruck "Alkyl" - sofern er nicht an anderer Stelle dieser Beschreibung oder in den Ansprüchen abweichend definiert ist - einen geradkettigen oder verzweigten aliphatischen Kohlenwasserstoffrest mit 1 bis 15 (d.h. 1, 2, 3, 4, 5, 6, 7, 8, 9, 10, 11, 12, 13, 14 oder 15) Kohlenstoffatomen; dieser Rest ist unsubstituiert oder einfach oder mehrfach gleich oder verschieden mit Fluor, Chlor, Brom, Iod und/oder Cyano substituiert.

Sofern es sich bei diesem Alkylrest um einen gesättigten Rest handelt, wird er auch als "Alkanyl" bezeichnet (CₐH₂ₐ₊₁-, wobei a eine ganze Zahl von 1 bis 15 ist und ein oder mehrere Wasserstoffatome durch Halogen, insbesondere Fluor, und/oder Cyano ersetzt sein können). Ferner umfaßt der Ausdruck "Alkyl" auch unsubstituierte oder entsprechend mit F, Cl, Br, I und/oder -CN ein- oder mehrfach gleich oder verschieden substituierte Kohlenwasserstoffreste, in denen auch eine oder mehrere CH₂-Gruppen derart durch -O- ("Alkoxy", "Oxaalkyl"), -S- ("Thioalkyl"), -CH=CH-("Alkenyl"), -C≡C- ("Alkinyl"), -CO-O- oder -O-CO- ersetzt sein können, dass Heteroatome (O, S) nicht direkt miteinander verknüpft sind. Vorzugsweise ist Alkyl ein geradkettiger oder verzweigter, unsubstituierter oder substituierter Alkanyl-, Alkenyl- oder Alkoxyrest mit 1, 2, 3, 4, 5, 6, 7 oder 8 Kohlenstoffatomen. Sofern Alkyl einen Alkanylrest bedeutet, ist dieser bevorzugt Methyl, Ethyl, n-Propyl, i-Propyl, n-Butyl, i-Butyl, t-Butyl, n-Pentyl, Neopentyl, n-Hexyl, n-Heptyl, n-Octyl; CF₃, CHF₂, CH₂F; CF₂CF₃. Besonders bevorzugt ist der Alkanylrest geradkettig und unsubstituiert oder mit F substituiert.

Da in einem Alkylrest erfindungsgemäß eine oder mehrere CH₂-Gruppen durch -O- ersetzt sein können, umfaßt der Ausdruck "Alkyl" auch "Alkoxy"-beziehungsweise "Oxaalkyl"-Reste. Unter Alkoxy ist ein O-Alkyl-Rest zu verstehen, in dem das Sauerstoffatom direkt mit der durch den Alkoxyrest substituierten Gruppe oder dem substituierten Ring verbunden ist und Alkyl wie oben definiert ist; vorzugsweise ist Alkyl dann Alkanyl oder Alkenyl. Bevorzugte Alkoxyreste sind Methoxy, Ethoxy, Propoxy, Butoxy, Pentoxy, Hexoxy, Heptoxy und Octoxy, wobei jeder dieser Reste auch mit Halogen oder Cyano substituiert sein kann, vorzugsweise mit einem oder mehreren Fluoratomen. Besonders bevorzugt ist Alkoxy -OCH₃, -OC₂H₅, -O-n-C₃H₇. -O-n-C₄H₉, -O-t-C₄H₉, -OCF₃, -OCHF₂, -OCH₂F or -OCHFCHF₂.

Im Zusammenhang mit der vorliegenden Erfindung bedeutet der Ausdruck "Oxaalkyl" Alkylreste, in denen wenigstens eine nicht-terminale CH₂₋Gruppe durch -O- derart ersetzt ist, dass keine benachbarten Heteroatome (O, S) vorliegen. Vorzugsweise umfaßt Oxaalkyl geradkettige Reste der Formel -CₐH₂ₐ₊₁-O-(CH₂)_{b}-, wobei a und b jeweils unabhängig voneinander 1, 2, 3, 4, 5, 6, 7, 8, 9 oder 10 bedeuten; besonders bevorzugt ist a eine ganze Zahl von 1 bis 6 und b 1 oder 2.

Sofern in einem wie oben definerten Alkylrest eine oder mehrere CH₂₋Gruppen durch Schwefel ersetzt sind, liegt ein "Thioalkyl"-Rest vor. Vorzugsweise umfaßt "Thioalkyl" einen geradkettigen Rest der Formel CₐH₂ₐ₊₁-S-(CH₂)_{b}-, wobei a 1, 2, 3, 4, 5, 6, 7, 8, 9 oder 10 ist und b 0, 1, 2, 3, 4, 5, 6, 7, 8, 9 oder 10 ; besonders bevorzugt ist a eine ganze Zahl von 1 bis 6 und b 0, 1 oder 2. Der Thioalkylrest kann ebenfalls mit F, Cl, Br, I und/oder -CN substituiert sein und ist vorzugsweise unsubstituiert.

Im Zusammenhang der vorliegenden Erfindung bedeutet der Ausdruck "Alkenyl" einen wie oben definierten Alkylrest, in dem eine oder mehrere -CH=CH-Gruppen vorhanden sind. Sofern zwei -CH=CH-Gruppen in dem Rest vorhanden sind, kann dieser auch als "Alkadienyl" bezeichnet werden. Ein Alkenylrest kann 2 bis 15 (d.h. 2, 3, 4, 5, 6, 7, 8, 9, 10, 11, 12, 13, 14 oder 15) Kohlenstoffatome enthalten und ist verzweigtkettig oder vorzugsweise geradkettig. Der Rest ist unsubstituiert oder ein- oder mehrfach gleich oder verschieden mit F, Cl, Br, I und/oder CN substituiert. Ferner können eine oder mehrere CH₂-Gruppen jeweils unabhängig voneinander durch -O-, -S-, -C≡C-, -CO-O-, -OC-O- so ersetzt sein, dass Heteroatome (O, S) nicht direkt miteinander verbunden sind. Falls die CH=CH-Gruppe an beiden Kohlenstoffatomen einen anderen Rest als Wasserstoff trägt, etwa wenn sie eine nicht-terminale Gruppe ist, kann die CH=CH-Gruppe in zwei Konfigurationen vorliegen, nämlich als E-Isomer und als Z-Isomer. Im allgemeinen ist das E-Isomer (trans) bevorzugt. Vorzugsweise enthält der Alkenylrest 2, 3, 4, 5, 6 oder 7 Kohlenstoffatome und bedeutet Vinyl, 1E-Propenyl, 1E-Butenyl, 1E-Pentenyl, 1E-Hexenyl, 1 E-Heptenyl, 2-Propenyl, 2E-Butenyl, 2E-Pentenyl, 2E-Hexenyl, 2E-Heptenyl, 3-Butenyl, 3E-Pentenyl, 3E-Hexenyl, 3E-Heptenyl, 4-Pentenyl, 4Z-Hexenyl, 4E-Hexenyl, 4Z-Heptenyl, 5-Hexenyl und 6-Heptenyl. Besonders bevorzugte Alkenylreste sind Vinyl, 1 E-Propenyl und 3E-Butenyl.

Falls in einem Alkylrest eine oder mehrere CH₂-Gruppen durch -C≡Cersetzt sind, liegt ein Alkinylrest vor. Auch die Ersetzung von einer oder mehreren CH₂-Gruppen durch -CO-O- oder -O-CO- ist möglich. Dabei sind die folgenden dieser Reste bevorzugt: Acetyloxy, Propionyloxy, Butyryloxy, Pentanoyloxy, Hexanoyloxy, Acetyloxymethyl, Propionyloxymethyl, Butyryloxymethyl, Pentanoyloxymethyl, 2-Acetyloxyethyl, 2-Propionyloxyethyl, 2-Butyryloxyethyl, 2-Acetyloxypropyl, 3-Propionyloxypropyl, 4-Acetyloxybutyl, Methoxycarbonyl, Ethoxycarbonyl, Propoxycarbonyl, Butoxycarbonyl, Pentoxycarbonyl, Methoxycarbonylmethyl, Ethoxycarbonylmethyl, Propoxycarbonylmethyl, Butoxycarbonylmethyl, 2-(Methoxycarbonyl)ethyl, 2-(Ethoxycarbonyl)ethyl, 2-(Propoxycarbonyl)-ethyl, 3-(Methoxycarbonyl)-propyl, 3-(Ethoxy-carbonyl)-propyl oder 4-(Methoxycarbonyl)-butyl.

Falls in einem Alkylrest eine CH₂-Gruppe durch unsubstituiertes oder substituiertes -CH=CH- und eine benachbarte CH₂-Gruppe durch -CO-O- oder -O-CO- ersetzt ist, so kann dieser Rest geradkettig oder verzweigt sein.

Vorzugsweise ist er geradkettig und hat 4 bis 13 Kohlenstoffatome. Er bedeutet demnach besonders bevorzugt Acryloyloxymethyl, 2-Acryloyloxyethyl, 3-Acryloyloxypropyl, 4-Acryloyloxybutyl, 5-Acryloyloxypentyl, 6-Acryloyloxyhexyl, 7-Acryloyloxyheptyl, 8-Acryloyloxyoctyl, 9-Acryloyloxynonyl, 10-Acryloyloxydecyl, Methacryloyloxymethyl, 2-Methacryloyloxyethyl, 3-Methacryloyloxypropyl, 4-Methacryloyloxybutyl, 5-Methacryloyloxypentyl, 6-Methacryloyloxyhexyl, 7-Methacryloyloxyheptyl, 8-Methacryloyloxyoctyl und 9-Methacryloyloxynonyl.

Im Zusammenhang der vorliegenden Erfindung steht der Ausdruck "Aralkyl" für einen Aryl-Alkyl-Rest, d.h. für einen Rest, in dem ein Aryl-Substituent über eine Alkyl-Brücke mit einem Atom, einer Kette, einem anderen Rest oder einer funktionellen Gruppe verknüpft ist. Der Ausdruck "-O-Aralkyl" steht für einen Aryl-Alkoxy-Rest, d.h. für einen Rest, in dem ein Aryl-Alkyl-Rest über ein Sauerstoffatom mit einem Atom, einer Kette, einem anderen Rest oder einer funktionellen Gruppe verknüpft ist. Unter einem ArylSubstituenten versteht man dabei einen aromatischen Kohlenwasserstoff mit 6 bis 18 Kohlenstoffatomen, der gegebenenfalls mit Halogen, Nitro, Alkanyl- und/oder Alkoxy-Resten substituiert ist, insbesondere einen Phenyl- und einen Naphthylrest. Bei der Alkyl-Brücke handelt es sich vorzugsweise um einen gesättigten Kohlenwasserstoffrest, insbesondere um Methylen (-CH₂-) und Ethylen (-CH₂CH₂-). Bevorzugte Beispiele eines Aralkylrests sind Benzyl und Phenethyl. Bevorzugte Beispiele eines -O-Aralkylrests sind O-Benzyl (-O-CH₂Phenyl), O-Phenethyl (-O-CH₂CH₂Phenyl) und O-(p-Nitrobenzyl).

Erfindungsgemäß bedeutet "Alkylenbrücke" eine aliphatische Kohlenwasserstoffkette, die unverzweigt oder verzweigt ist und die Formel -CₙH₂ₙ₋aufweist, beispielsweise -CH₂CH₂-, -CH₂CH₂CH₂- oder -CH₂C(CH₃)₂CH₂-,

"Halogen" umfasst im Zusammenhang der vorliegenden Erfindung Fluor, Chlor, Brom und Iod.

Sofern Reste oder Substituenten der erfindungsgemäßen Pyranderivate beziehungsweise die erfindungsgemäßen Pyranderivate selbst als optisch aktive oder stereoisomere Reste, Substituenten beziehungsweise Verbindungen vorliegen können, weil sie beispielsweise ein asymmetrisches Zentrum aufweisen, so sind diese von der vorliegenden Erfindung mit umfaßt. Dabei ist es selbstverständlich, daß die erfindungsgemäßen Pyranderivate der allgemeinen Formeln I und III in isomerenreiner Form, zum Beispiel als reine Enantiomeren, Diastereomeren, E- beziehungsweise Z-Isomeren, trans- beziehungsweise cis-Isomeren, oder als Gemisch mehrerer Isomeren in jedem beliebigen Verhältnis, zum Beispiel als Racemat, E-/Z-Isomerengemisch oder als cis/trans-Isomerengemisch, vorliegen können.

Bevorzugt weisen die erfindungsgemäßen Verbindungen der Formel I einschließlich des zentralen Pyranrings nicht mehr als insgesamt vier Ringsysteme auf, so dass a+b+c+d+e ≤ 3 ist; besonders bevorzugt gilt: 1 ≤ a+b+c+d+e ≤ 3, d.h. neben dem Pyranring sind wenigstens ein weiteres und nicht mehr als drei weitere Ringsysteme vorhanden. Weiterhin ist bevorzugt, dass R¹¹ ein geradkettiger Alkenyl- oder besonders bevorzugt ein geradkettiger Alkanylrest mit 1 bis 7 Kohlenstoffatomen, insbesondere Methyl, und R¹² Halogen, insbesondere Fluor, oder ein gegebenenfalls mit Halogen, insbesondere Fluor, einfach oder mehrfach substituierter unverzweigter Alkanyl- oder Alkoxyrest mit 1 bis 5 Kohlenstoffatomen ist.

Verbindungen der Formel I mit verzweigten Flügelgruppen R¹¹ und/oder R¹² können gelegentlich bei Verwendung in flüssigkristallinen Medien wegen ihrer sehr guten Löslichkeit in den üblichen, flüssigkristallinen Basismaterialien von Bedeutung sein, insbesondere aber als chirale Dotierstoffe, wenn sie optisch aktiv sind. Smektische Verbindungen dieser Art eignen sich als Komponenten für ferroelektrische Materialien.

Verzweigte Gruppen dieser Art enthalten in der Regel nicht mehr als eine Kettenverzweigung. Bevorzugte verzweigte Reste R¹¹ beziehungsweise R¹² sind Isopropyl, 2-Butyl (= 1-Methylpropyl), Isobutyl (= 2-Methylpropyl), 2-Methylbutyl, Isopentyl (= 3-Methylbutyl), 2-Methylpentyl, 3-Methylpentyl, 2-Ethylhexyl, 2-Propylpentyl, Isopropoxy, 2-Methylpropoxy, 2-Methylbutoxy, 3-Methylbutoxy, 2-Methylpentyloxy, 3-Methylpentyloxy, 2-Ethylhexyloxy, 1-Methylhexyloxy, 1-Methylheptyloxy.

Eine bevorzugte Klasse erfindungsgemäßer Verbindungen der Formel I wird von Pyranderivaten gebildet, bei denen W in Formel I für eine Carbonylgruppe, d.h. für -C(=O)- steht. Es handelt sich dann um Lactone der allgemeinen Formel I-A: 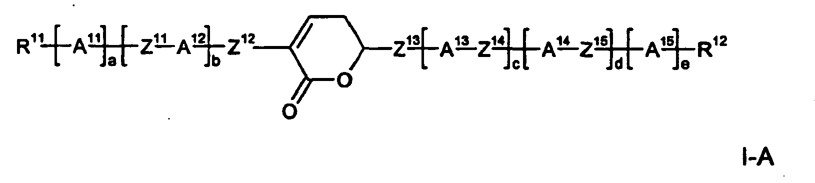 wobei a, b, c, d, e, R¹¹, R¹², Z¹¹, Z¹², Z¹³, Z¹⁴, Z¹⁵, A¹¹, A¹², A¹³, A¹⁴ und A¹⁵ die gleiche Bedeutung wie für Formel I oben besitzen.

Eine andere bevorzugte Klasse erfindungsgemäßer Verbindungen der Formel I wird von Pyranderivaten gebildet, bei denen W in Formel I für eine Methylengruppe, d.h. für -CH₂- steht. Es handelt sich dann um Dihydropyrane der allgemeinen Formel I-B: 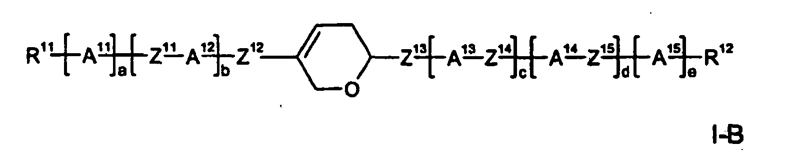 wobei a, b, c, d, e, R¹¹, R¹², Z¹¹, Z¹², Z¹³, Z¹⁴, Z¹⁵, A¹¹, A¹², A¹³, A¹⁴ und A¹⁵ die gleiche Bedeutung wie für Formel I oben besitzen.

Im Zusammenhang der vorliegenden Erfindung werden die erfindungsgemäßen Verbindungen der Formel I auch als "Pyranderivate" bezeichnet; der Ausdruck "Pyranderivate" umfasst sowohl Pyrane der Formel I-B als auch Lactone der Formel I-A.

Ferner sind solche Verbindungen der Formel I bevorzugte Ausführungsformen der Erfindung, in denen Z¹² eine Einfachbindung ist und der zentrale Pyranring entweder direkt mit R¹¹ (a = b = 0) oder mit dem Ring A¹¹ (a = 1; b = 0) beziehungsweise dem Ring A¹² (a = 0; b =1) verbunden ist (Formel I-C): 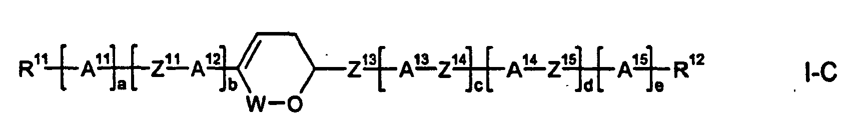 wobei a, b, c, d, e, W, R¹¹, R¹², Z¹¹, Z¹³, Z¹⁴, Z¹⁵, A¹¹, A¹², A¹³, A¹⁴ und A¹⁵ die gleiche Bedeutung wie für Formel I oben besitzen. Bevorzugte Untergruppen von Verbindungen der Formel I-C werden dabei von Verbindungen der Formel I-CA mit W gleich -C(=O)- und insbesondere von Verbindungen der Formel I-CB mit W gleich -CH₂- gebildet: 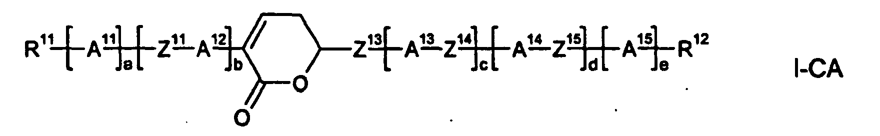 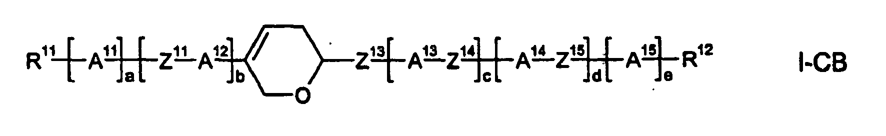 wobei a, b, c, d, e, R¹¹, R¹², Z¹¹, Z¹³, Z¹⁴, Z¹⁵, A¹¹, A¹², A¹³, A¹⁴ und A¹⁵ die gleiche Bedeutung wie für Formel I oben besitzen. Besonders bevorzugt sind dabei Verbindungen, bei denen in Formel I-CA beziehungsweise I-CB a = b = 0 gilt und R¹¹ Alkanyl bedeutet.

Außerdem sind bevorzugte Verbindungen der Erfindung solche, bei denen b 1 ist, Z¹¹ eine Einfachbindung bedeutet und A¹² für einen 1,4-Cyclohexylenring steht (Formel I-D): 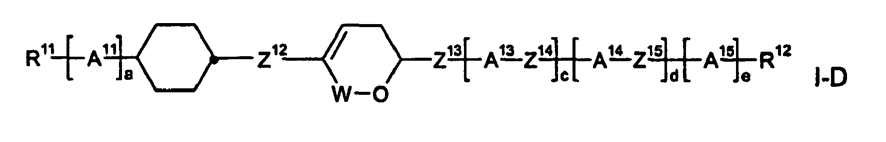 wobei a, c, d, e, W, R¹¹, R¹², Z¹², Z¹³, Z¹⁴, Z¹⁵, A¹¹, A¹³, A¹⁴ und A¹⁵ die gleiche Bedeutung wie für Formel I oben besitzen.

Bevorzugte Untergruppen von Verbindungen der Formel I-D werden dabei von Verbindungen der Formel I-DA mit W gleich -C(=O)- und insbesondere von Verbindungen der Formel I-DB mit W gleich -CH₂- gebildet.

Besonders bevorzugt sind dabei solche Verbindungen der Formel I-DB, bei denen ferner Z¹² eine Einfachbindung darstellt (Formel I-DBA): 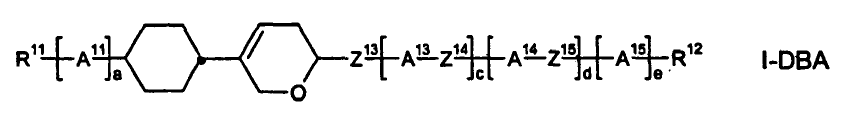 wobei a, c, d, e, R¹¹, R¹², Z¹³, Z¹⁴, Z¹⁵, A¹¹, A¹³, A¹⁴ und A¹⁵ die gleiche Bedeutung wie für Formel I oben besitzen. Es ist dabei ganz besonders bevorzugt, dass Z¹³ für eine Einfachbindung oder -CF₂O- steht, c und d beide zugleich null sind und A¹⁵ für einen gegebenenfalls mit Fluor einfach oder mehrfach substituierten 1,4-Phenylenring steht.

Eine weitere Gruppe bevorzugter erfindungsgemäßen Verbindungen wird von Verbindungen der Formel I-E gebildet, d.h. Verbindungen der Formel I mit a = 1 und A¹¹ = 1,4-Cyclohexylen: 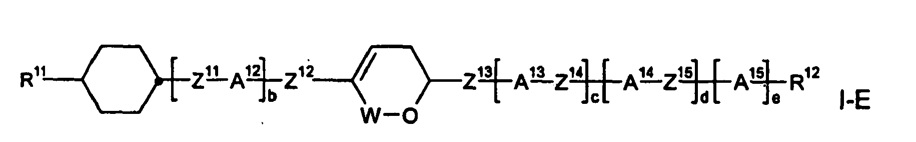 wobei b, c, d, e, W, R¹¹, R¹², Z¹¹, Z¹², Z¹³, Z¹⁴, Z¹⁵, A¹², A¹³, A¹⁴ und A¹⁵ die gleiche Bedeutung wie für Formel I oben besitzen. Bevorzugte Untergruppen von Verbindungen der Formel I-E werden dabei von Verbindungen der Formel I-EA mit W gleich -C(=O)- und insbesondere von Verbindungen der Formel I-EB mit W gleich -CH₂- gebildet.

Besonders bevorzugte Verbindungen der Formel I-EB sind dabei solche Verbindungen der Formeln I-EBA und I-EBB; ganz besonders bevorzugt sind Verbindungen der Formel I-EBC: 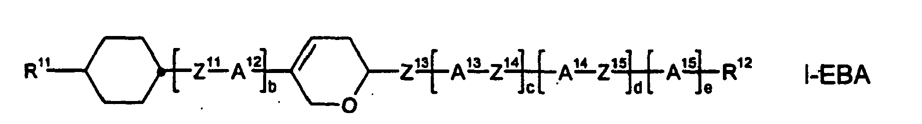 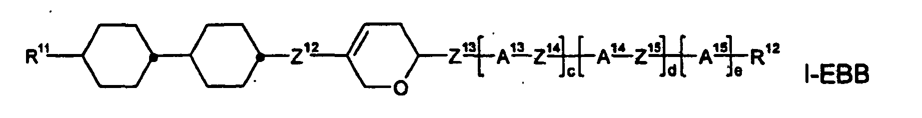 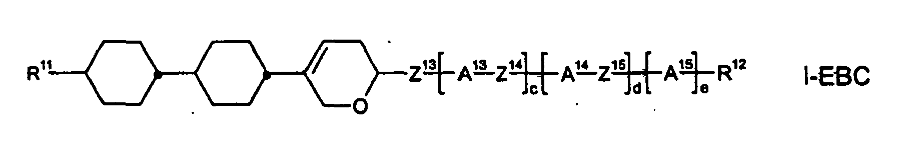 wobei b, c, d, e, R¹¹, R¹², Z¹¹, Z¹², Z¹³, Z¹⁴, Z¹⁵, A¹², A¹³, A¹⁴ und A¹⁵ die gleiche Bedeutung wie für Formel I oben besitzen. Bevorzugt ist dabei, dass Z¹³ für eine Einfachbindung oder -CF₂O- steht, c und d beide zugleich null sind und A¹⁵ für einen gegebenenfalls mit Fluor einfach oder mehrfach substituierten 1,4-Phenylenring steht.

Darüber hinaus ist es auch bevorzugt, dass Z¹³ in Formel I eine Einfachbindung darstellt (Formel I-F) oder für eine Carboxylfunktion (Formel I-G) oder eine Difluoroxymethylenbrücke (Formel I-H) steht: 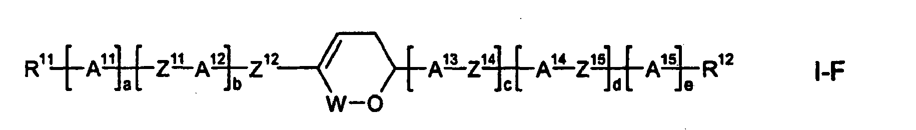 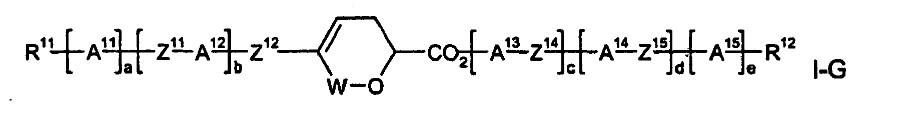 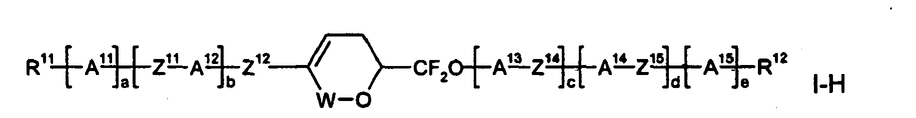 wobei a, b, c, d, e, W, R¹¹, R¹², Z¹¹, Z¹², Z¹⁴, Z¹⁵, A¹¹, A¹², A¹³, A¹⁴ und A¹⁵ die gleiche Bedeutung wie für die Formeln I, I-C, I-D beziehungsweise I-E oben besitzen. Bevorzugte Untergruppen von Verbindungen der Formel I-F. I-G und I-H werden dabei von Verbindungen der Formel I-FA, I-GA und I-HA mit W gleich -C(=O)- und insbesondere von Verbindungen der Formel I-FB, I-GB und I-HB mit W gleich -CH₂- gebildet. Dabei ist es besonders bevorzugt, dass Z¹² eine Einfachbindung darstellt. Wenn a und b beide zugleich null sind, so ist vorzugsweise R¹¹ ein Alkanylrest mit 1 bis sieben Kohlenstoffatomen. Wenn a 1 ist, steht A¹¹ vorzugsweise für einen 1,4-Cyclohexylenring. Wenn b 1 ist, so sind vorzugsweise Z¹¹ eine Einfachbindung und A¹² ebenfalls ein 1,4-Cyclohexylenring.

Es ist ferner bevorzugt, dass die erfindungsgemäßen Verbindungen der Formel I einen Ring A¹⁵ besitzen, wobei es besonders bevorzugt ist, dass dieser Ring ein gegebenenfalls in 3- und/oder 5-Position mit Fluor substituierter 1,4-Phenylenring ist (Formel I-J): 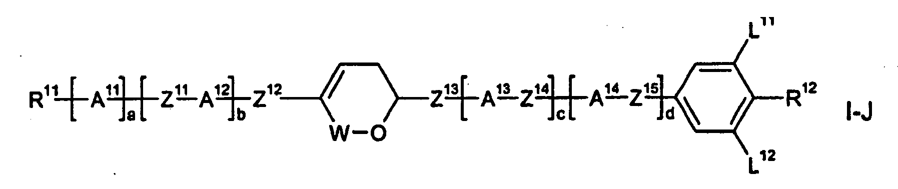 wobei a, b, c, d, W, R¹¹, R¹², Z¹¹, Z¹², Z¹³, Z¹⁴, Z¹⁵, A¹¹, A¹², A¹³ und A¹⁴ die gleiche Bedeutung wie für die Formeln I, I-C, I-D, I-E, I-F, I-G beziehungsweise I-H oben besitzen und L¹¹ und L¹² unabhängig voneinander H oder F bedeuten. Bevorzugte Untergruppen von Verbindungen der Formel 1-J werden dabei von Verbindungen der Formel I-JA mit W gleich -C(=O)- und insbesondere von Verbindungen der Formel 1-JB mit W gleich -CH₂- gebildet. R¹² ist hierbei besonders bevorzugt Halogen, insbesondere Fluor, oder ein gegebenenfalls mit Halogen, insbesondere Fluor, einfach oder mehrfach substituierter unverzweigter Alkanyl- oder Alkoxyrest mit 1 bis 5 Kohlenstoffatomen.

Eine weitere Gruppe bevorzugter erfindungsgemäßer Verbindungen der Formel I wird von Verbindungen gebildet, in denen A¹⁵-R¹² für 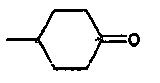 oder 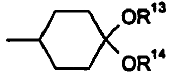 steht, wobei R¹³ und R¹⁴ wie oben für Formel I definiert sind, und die durch die Formeln I-K (Cyclohexanone) beziehungsweise I-L (Ketale) dargestellt werden: 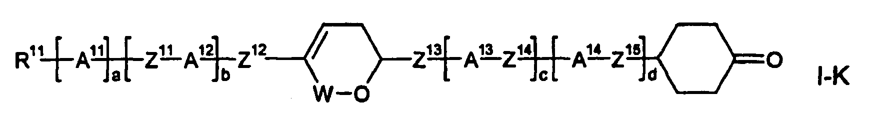 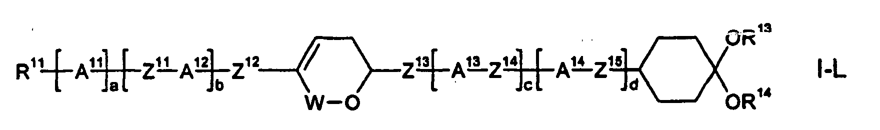 wobei a, b, c, d, W, R¹¹, Z¹¹, Z¹², Z¹³, Z¹⁴, Z¹⁵, A¹¹, A¹², A¹³ und A¹⁴ die gleiche Bedeutung wie für Formel I oben besitzen. Besonders bevorzugt sind c und d in den Formeln I-K und I-L zugleich null.

Eine weitere Gruppe bevorzugter erfindungsgemäßer Verbindungen der Formel I wird von Verbindungen gebildet, bei denen c 1 ist; Z¹⁴ eine Einfachbindung, -C(O)-O- oder -CF₂O- bedeutet; A¹³ für 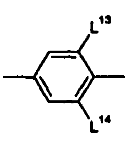 oder 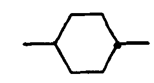 steht; und L¹³ und L¹⁴ unabhängig voneinander für H oder F stehen. Diese Verbindungen werden durch die Formeln I-M, I-N, I-O, I-P, I-Q und I-R dargestellt: 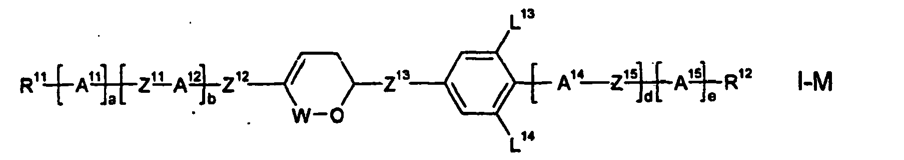 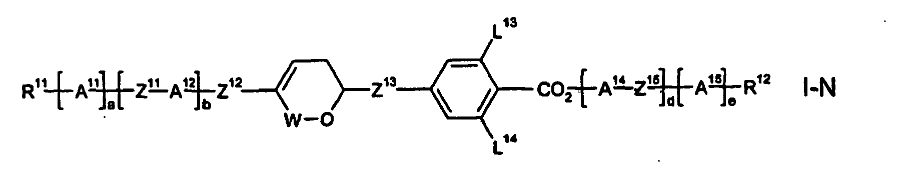 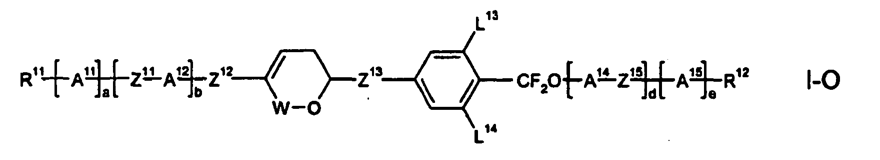 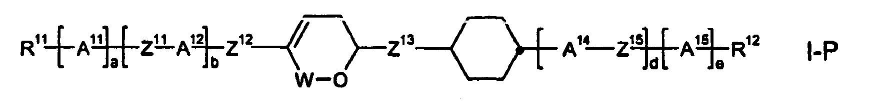 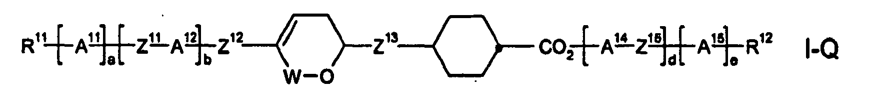 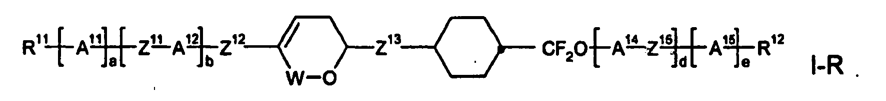 wobei a, b, d, e, R¹¹, R¹², Z¹¹, Z¹², Z¹³, Z¹⁵, A¹¹, A¹², A¹⁴ und A¹⁵ die gleiche Bedeutung wie für Formel I oben besitzen. Bevorzugte Untergruppen von Verbindungen der Formeln I-M, I-N, I-O, I-P, I-Q und I-R werden dabei von Verbindungen der Formeln I-MA, I-NA, I-OA, I-PA, I-QA und I-RA mit W gleich -C(=O)- und insbesondere von Verbindungen der Formeln I-MB, I-NB, I-OB, I-PB, I-QB und I-RB mit W gleich -CH₂- gebildet. Dabei ist es besonders bevorzugt, dass Z¹³ eine Einfachbindung darstellt, d null und e 1 ist und A¹⁵ für einen gegebenenfalls mit Fluor einfach oder mehrfach substituierten 1,4-Phenylenring steht.

Eine weitere Gruppe bevorzugter erfindungsgemäßer Verbindungen wird von Verbindungen der Formeln I-S und I-T gebildet, d.h. Verbindungen der Formel I, in denen d 1 ist; Z¹⁵ -CF₂O- bedeutet; A¹⁴ für 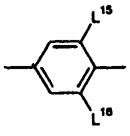 oder 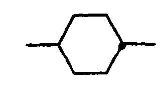 steht; und L¹⁵ und L¹⁶unabhängig voneinander H oder F bedeuten: 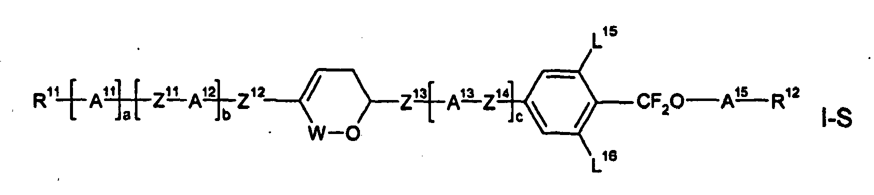 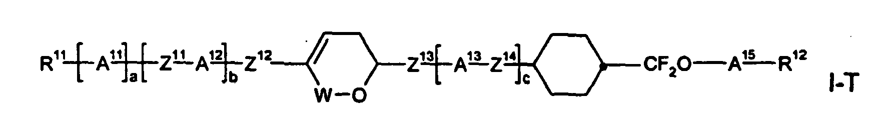 wobei a, b, c, R¹¹, R¹², Z¹¹, Z¹², Z¹³, Z¹⁴, A¹¹, A¹², A¹³ und A¹⁵ die gleiche Bedeutung wie für Formel I oben besitzen. Es ist besonders bevorzugt, dass A¹⁵ in den Formeln I-S und I-T für 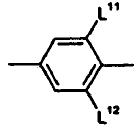 steht und Z¹³ eine Einfachbindung darstellt. Ferner ist es für die Verbindungen der Formel 1-S mit c = 1 bevorzugt, dass Z¹⁴ eine Einfachbindung darstellt und A¹³ für 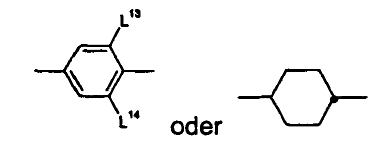 steht. Für die Verbindungen der Formel I-T mit c = 1 ist es bevorzugt, dass Z¹⁴ eine Einfachbindung darstellt und A¹³ für 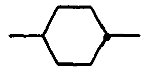 steht.

Bevorzugte Untergruppen von Verbindungen der Formeln I-S und I-T werden dabei von Verbindungen der Formeln I-SA und I-TA mit W gleich -C(=O)- und insbesondere von Verbindungen der Formeln I-SB und I-TB mit W gleich -CH₂- gebildet.

Ganz besonders bevorzugte erfindungsgemäße Verbindungen der Formel I-CB sind solche der Formeln I-CBI und I-CBII (die unter anderem auch Ausführungsformen von Verbindungen der Formeln I-F, I-J, I-P, I-Q, I-R und I-T (I-CBI) beziehungsweise I-F, I-J, I-M, I-N und I-O (I-CBII) darstellen):

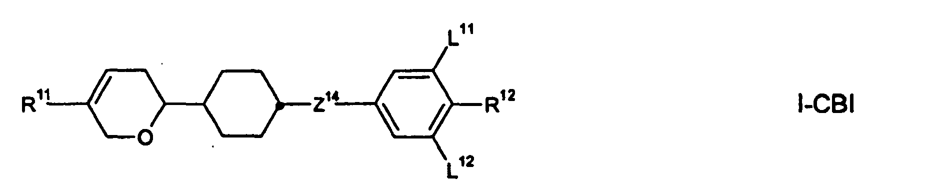

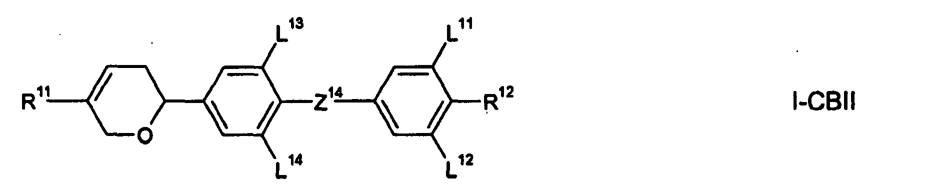

Dabei sind L¹³ und L¹⁴ unabhängig voneinander H oder F und R¹¹, R¹², L¹¹, L¹² und Z¹⁴ wie für Formel 1 oben definiert, wobei R¹¹ bevorzugt ein geradkettiger Alkenyl- (CₙH₂ₙ₋₁-) oder besonders bevorzugt Alkanylrest (CₙH₂ₙ₊₁-) mit 1 bis 7 Kohlenstoffatomen, insbesondere Methyl, ist, während R¹² bevorzugt Halogen, insbesondere Fluor, oder ein gegebenenfalls mit Halogen, insbesondere Fluor, einfach oder mehrfach substituierter unverzweigter Alkanyl- oder Alkoxyrest mit 1 bis 5 Kohlenstoffatomen ist und Z¹⁴ bevorzugt eine Einfachbindung, eine Carboxylgruppe oder eine Difluoroxymethylen-Brücke darstellt.

Beispielhafte Verbindungen der Formeln I-CBI und I-CBII werden durch die Formeln I-CBIa, I-CBIb, I-CBIc, I-CBIIa, I-CBIIb und I-CBIIc wiedergegeben, wobei die bevorzugte Bedeutung für R¹², L¹¹, L¹², L¹³ und L¹⁴ sowie n in den Tabellen 1 und 2 unten angegeben ist:

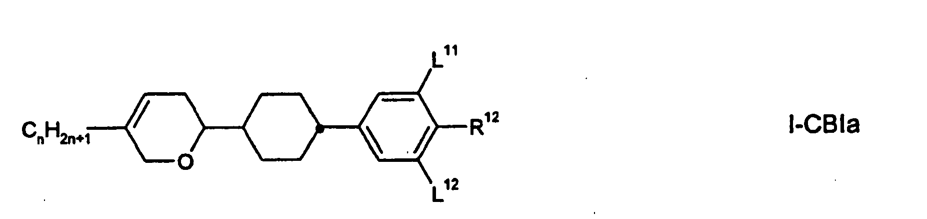

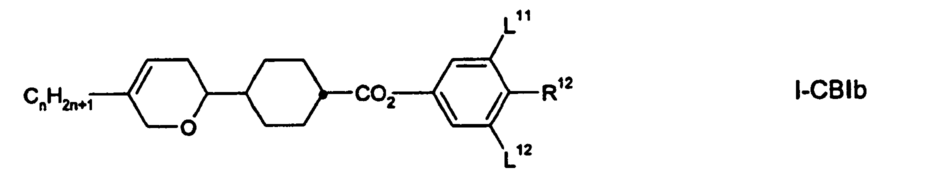

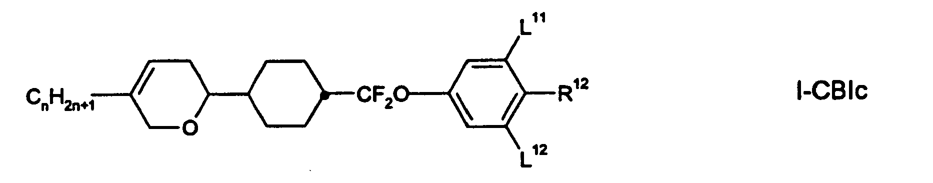

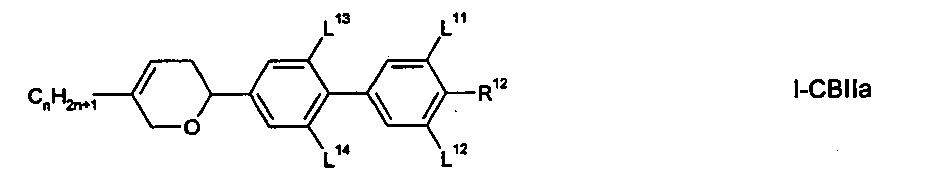

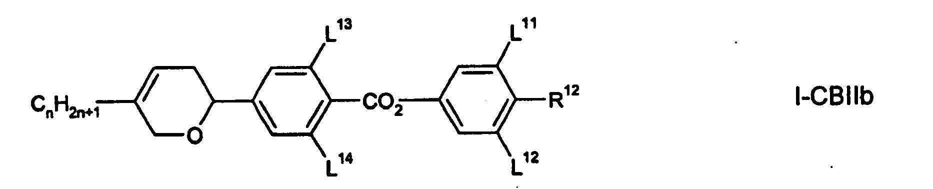

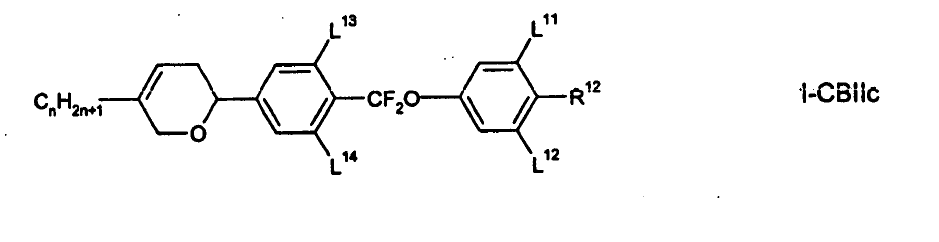

Eine weitere Gruppe ganz besonders bevorzugter Verbindungen der Formel I-CB sind solche der Formel I-CBIII: 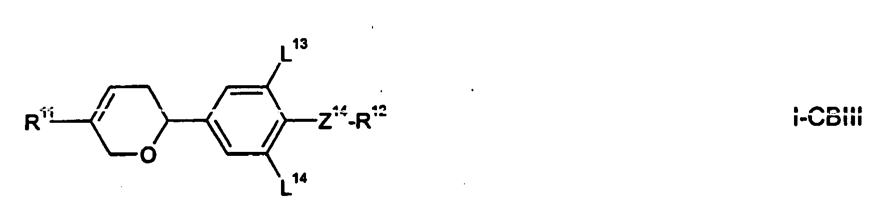 wobei R¹¹ und Z¹⁴-R¹² wie für Formel I oben definiert sind und L¹³ und L¹⁴ unabhängig voneinander H oder F bedeuten. Bevorzugt bilden Z¹⁴-R¹² dabei Substituenten, die eine weitere Funktionalisierung beziehungsweise Derivatisierung unter Bildung anderer erfindungsgemäßer Verbindungen der Formel I, z.B. durch Einführung weiterer Ringsysteme A¹⁴ und/oder A¹⁵, erleichtern. Besonders bevorzugt sind Z¹⁴-R¹² Alkoxy oder O-Aralkyl, insbesondere O-t-Butyl, O-Benzyl oder O-(p-Nitrobenzyl); CO₂H; CO₂Alkanyl oder CO₂Aralkyl, insbesondere CO₂-t-Butyl oder CO₂Benzyl; und (Einfachbindung)-Halogen, insbesondere -Br. R¹¹ ist bevorzugt ein geradkettiger Alkenyl- oder Alkanylrest mit bis zu 7 Kohlenstoffatomen.

Beispielhafte Verbindungen der Formel I-CBIII sind solche der Formeln I-CBIIIa, I-CBIIIb und I-CBIIIc:

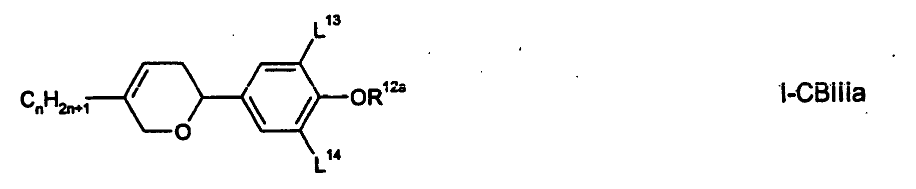

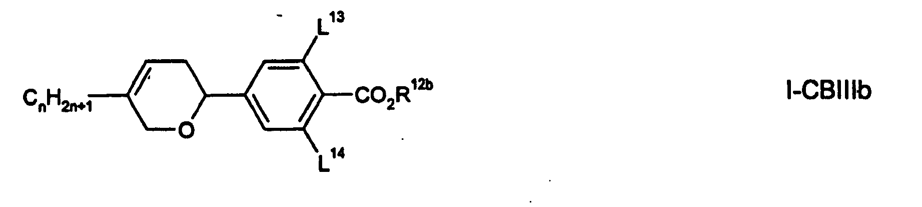

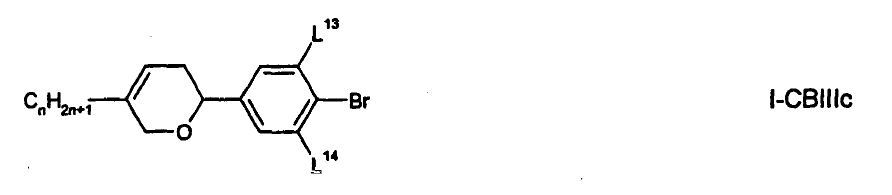

Dabei sind L¹³ und L¹⁴ unabhängig voneinander H oder F, R^{12a} t-Butyl, Benzyl oder p-Nitrobenzyl, R^{12b} H, Alkanyl mit 1 bis 5 Kohlenstoffatomen oder Aralkyl, insbesondere H, t-Butyl oder Benzyl, während CₙH₂ₙ₊₁ für einen geradkettigen Alkanylrest mit n = 1, 2, 3, 4, 5, 6 oder 7 steht.

Ganz besonders bevorzugte erfindungsgemäße Verbindungen der Formel I-DBA sind solche der Formeln I-DBAI, I-DBAII und I-DBAIII:

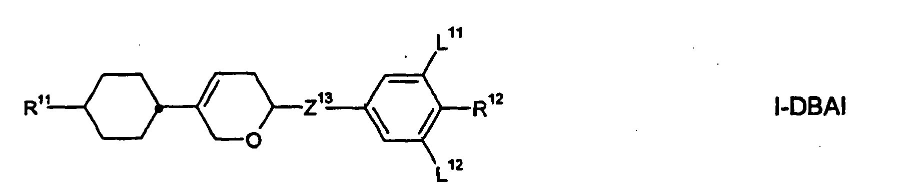

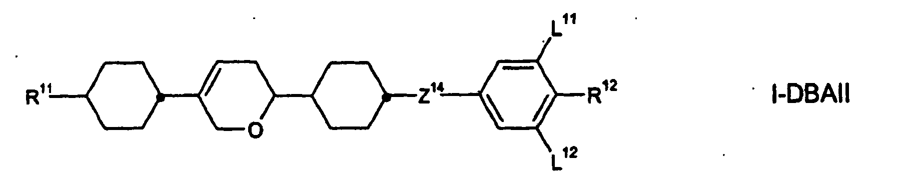

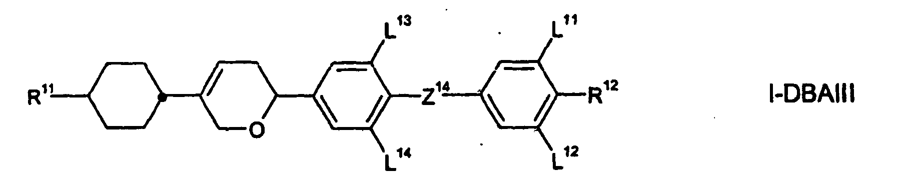

Dabei sind L¹³ und L¹⁴ unabhängig voneinander H oder F und R¹¹, R¹² , L¹¹, L¹², Z¹³ und Z¹⁴ wie für Formel I oben definiert, wobei R¹¹ bevorzugt H, ein geradkettiger Alkenyl- (CₙH₂ₙ₋₁-) oder besonders bevorzugt Alkanylrest (CₙH₂ₙ₊₁-) mit 1 bis 7 Kohlenstoffatomen, insbesondere Methyl, ist, während R¹² bevorzugt Halogen, insbesondere Fluor, oder ein gegebenenfalls mit Halogen, insbesondere Fluor, einfach oder mehrfach substituierter unverzweigter Alkanyl- oder Alkoxyrest mit 1 bis 5 Kohlenstoffatomen ist, Z¹³ bevorzugt für eine Einfachbindung oder CF₂O steht und Z¹⁴ bevorzugt eine Einfachbindung, eine Carboxylgruppe oder eine Difluoroxymethylen-Brücke darstellt.

Beispielhafte Verbindungen der Formeln I-DBAI, I-DBAII und I-DBAIII werden durch die Formeln I-DBAIa, I-DBAIb, I-DBAIIa, I-DBAIIb, I-DBAIIIa und I-DBAIIIb wiedergegeben, wobei die bevorzugte Bedeutung für R¹², L¹¹, L¹², L¹³ und L¹⁴ sowie n (n ist 0, 1, 2, 3, 4, 5, 6 oder 7) in den Tabellen 3 und 4 unten angegeben ist:

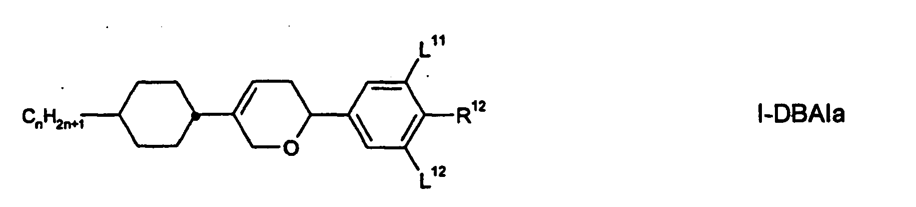

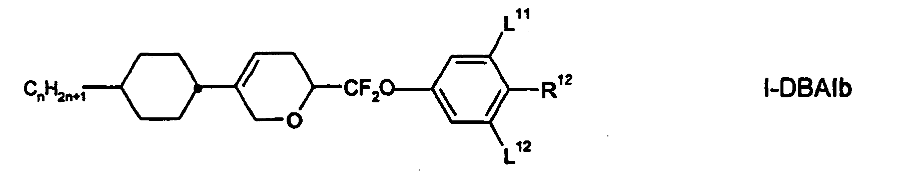

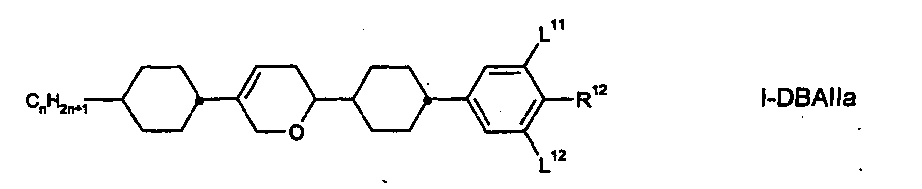

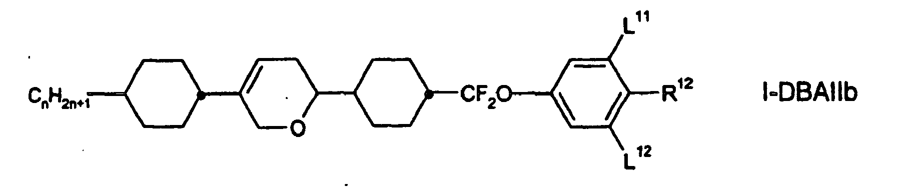

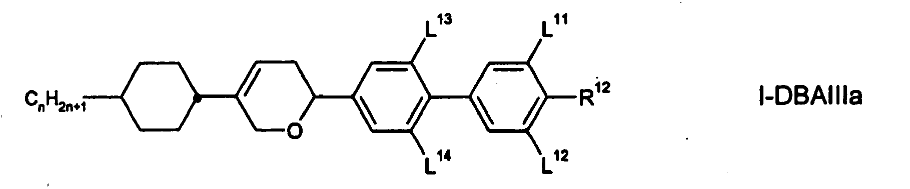

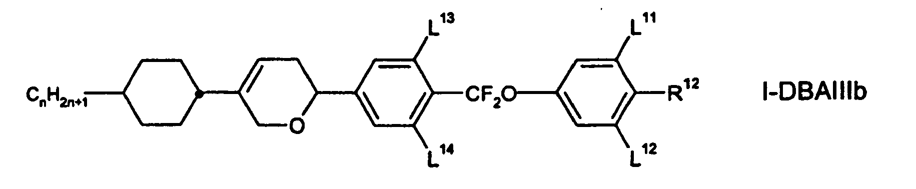

Ganz besonders bevorzugte erfindungsgemäße Verbindungen der Formel I-EBC sind solche der Formel I-EBCI:

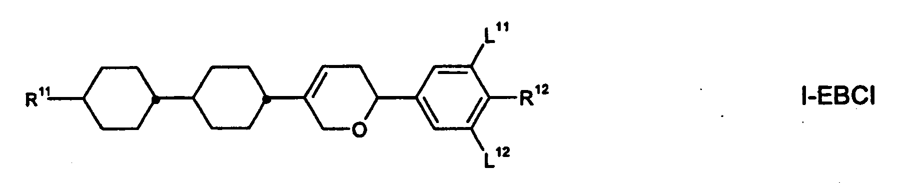

Dabei sind R¹¹ und R¹² wie für Formel I oben definiert, wobei R¹¹ bevorzugt H, ein geradkettiger Alkenyl- (CₙH₂ₙ₋₁-) oder insbesondere Alkanylrest (CₙH₂ₙ₊₁-) mit 1 bis 7 Kohlenstoffatomen ist, während R¹² bevorzugt Halogen, insbesondere Fluor, oder ein gegebenenfalls mit Halogen, insbesondere Fluor, einfach oder mehrfach substituierter unverzweigter Alkanyl- oder Alkoxyrest mit 1 bis 5 Kohlenstoffatomen ist.

Beispielhafte Verbindungen der Formeln I-EBCI werden durch die Formel I-EBCIa wiedergegeben, wobei die bevorzugte Bedeutung für R¹², L¹¹ und L¹² sowie n (n ist 0, 1, 2, 3, 4, 5, 6 oder 7) in Tabelle 3 unten angegeben ist:

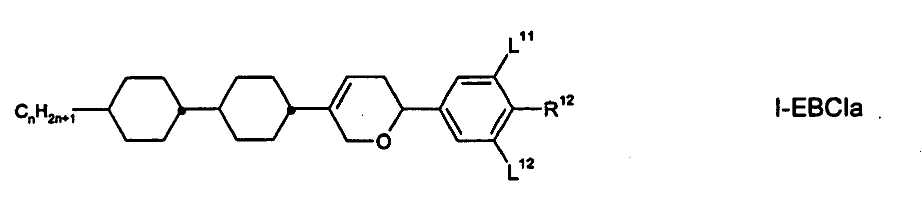

Ganz besonders bevorzugte erfindungsgemäße Verbindungen der Formel I-GB sind neben Verbindungen der Formel I-GBI auch solche der Formeln I-GBII und I-GBIII (die unter anderem auch Ausführungsformen von Verbindungen der Formel I-J darstellen):

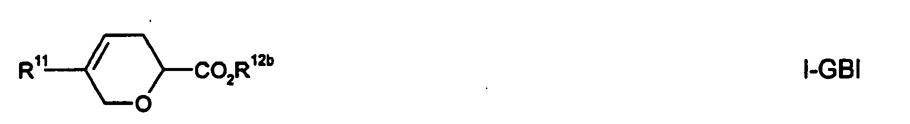

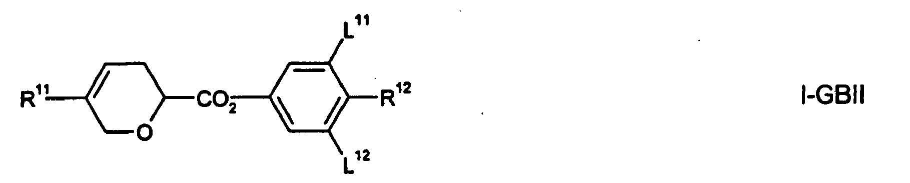

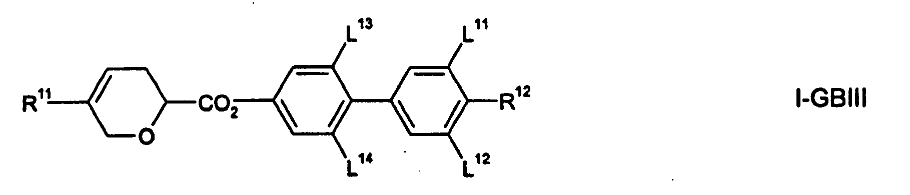

Dabei sind L¹³ und L¹⁴ unabhängig voneinander H oder F, R^{12b} wie R¹² für Formel I oben definiert und R¹¹, R¹², L¹¹ und L¹² wie für Formel I oben definiert, wobei R¹¹ bevorzugt ein geradkettiger Alkenyl- (CₙH₂ₙ₋₁-) oder insbesondere Alkanylrest (CₙH₂ₙ₊₁-) mit 1 bis 7 Kohlenstoffatomen ist, während R¹² für I-GBII und I-GBIII bevorzugt Halogen, insbesondere Fluor, oder ein gegebenenfalls mit Halogen, insbesondere Fluor, einfach oder mehrfach substituierter unverzweigter Alkanyl- oder Alkoxyrest mit 1 bis 5 Kohlenstoffatomen ist; für I-GBI ist R^{12b} bevorzugt H, Alkanyl mit 1 bis 5 Kohlenstoffatomen oder Aralkyl, insbesondere H, tert.-Butyl oder Benzyl.

Beispielhafte Verbindungen der Formel I-GBI werden durch die Formel I-GBIa wiedergegeben, wobei die bevorzugte Bedeutung für R^{12b} sowie n (n ist 1, 2, 3, 4, 5, 6 oder 7) in Tabelle 5 unten angegeben ist. Beispielhafte Verbindungen der Formeln I-GBII und I-GBIII werden durch die Formeln I-GBlla und I-GBllla wiedergegeben, wobei die bevorzugte Bedeutung für R¹², L¹¹, L¹² , L¹³ und L¹⁴ sowie n (n ist 1, 2, 3, 4, 5, 6 oder 7) in den Tabellen 1 und 2 unten angegeben ist:

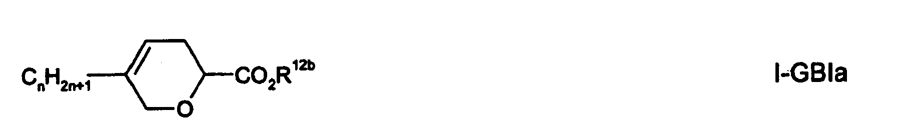

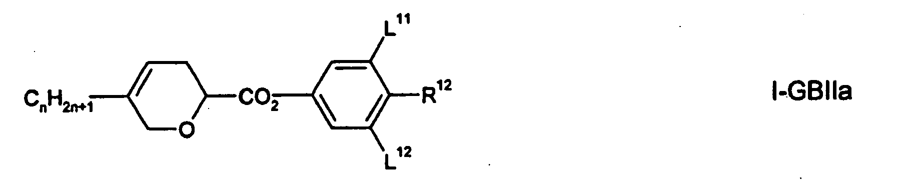

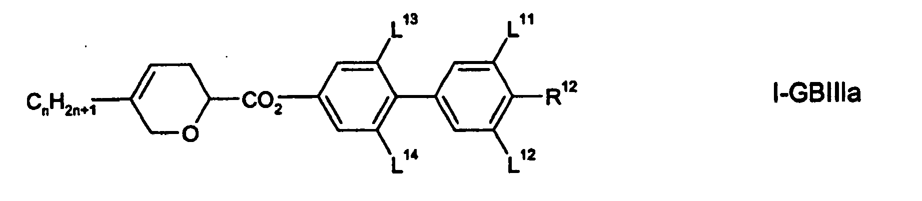

Ganz besonders bevorzugte erfindungsgemäße Verbindungen der Formel I-HB sind solche der Formeln I-HBI und I-HBII (wobei die Verbindungen der Formeln I-HBI und I-HBII unter anderem auch Ausführungsformen von Verbindungen der Formel I-J sind):

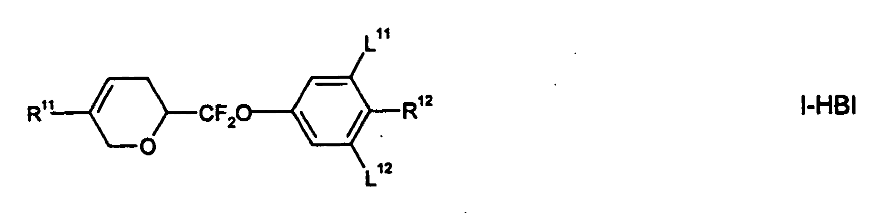

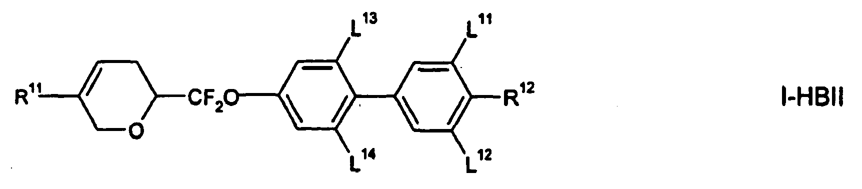

Dabei sind L¹³ und L¹⁴ unabhängig voneinander H oder F und R¹¹, R¹², L¹¹ und L¹² wie für Formel I oben definiert, wobei R¹¹ bevorzugt ein geradkettiger Alkenyl- (CₙH₂ₙ₋₁-) oder insbesondere Alkanylrest (CₙH₂ₙ₊₁-) mit 1 bis 7 Kohlenstoffatomen ist, während R¹² bevorzugt Halogen, insbesondere Fluor, oder ein gegebenenfalls mit Halogen, insbesondere Fluor, einfach oder mehrfach substituierter unverzweigter Alkanyl- oder Alkoxyrest mit 1 bis 5 Kohlenstoffatomen ist.

Beispielhafte Verbindungen der Formeln I-HBI und I-HBII werden durch die Formeln I-HBIa und I-HBIIa wiedergegeben, wobei die bevorzugte Bedeutung für R¹², L¹¹, L¹², L¹³ und L¹⁴ sowie n (n ist 1, 2, 3, 4, 5, 6 oder 7) in den Tabellen 1 und 2 unten angegeben ist:

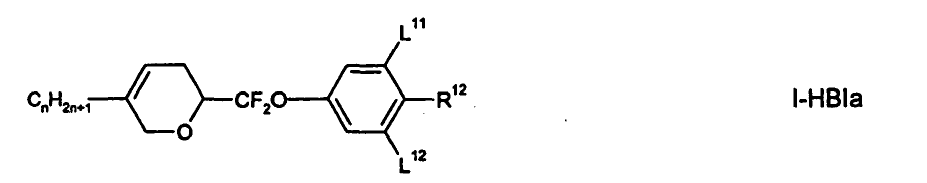

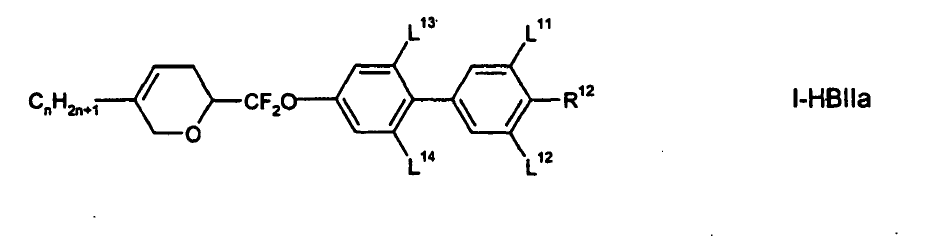

Ganz besonders bevorzugte erfindungsgemäße Verbindungen der Formel I-KB sind solche der Formeln I-KBI und I-KBII:

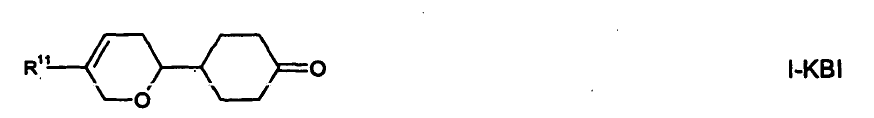

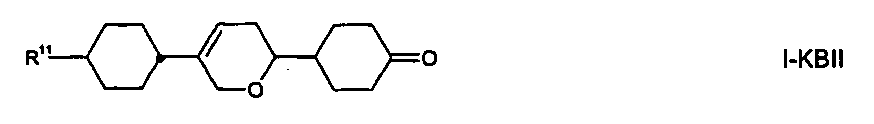

Dabei ist R¹¹ wie für Formel I oben definiert und bevorzugt ein geradkettiger Alkenyl- (CₙH₂ₙ₋₁-) oder insbesondere Alkanylrest (CₙH₂ₙ₊₁-) mit 1 bis 7 Kohlenstoffatomen, im Falle von I-KBII auch H. Beispielhafte Verbindungen der Formeln I-KBI und I-KBII sind solche, in denen R¹¹ für einen geradkettigen Alkanylrest CₙH₂ₙ₊₁- mit n = 1, 2, 3, 4, 5, 6 oder 7 steht.

Ganz besonders bevorzugte erfindungsgemäße Verbindungen der Formel I-LB sind solche der Formeln I-LBI und I-LBII:

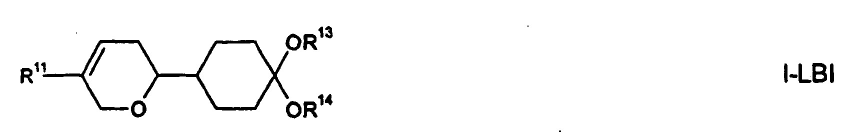

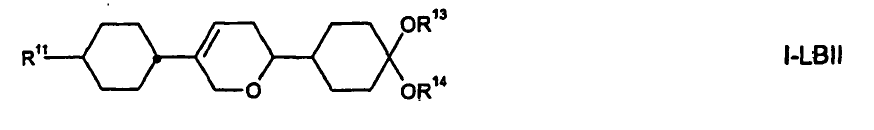

Dabei ist R¹¹ wie für Formel I oben definiert und bevorzugt ein geradkettiger Alkenyl- (CₙH₂ₙ₋₁-) oder insbesondere Alkanylrest (CₙH₂ₙ₊₁-) mit 1 bis 7 Kohlenstoffatomen, im Falle von I-LBII auch H. R¹³ und R¹⁴ sind ebenfalls wie für Formel I oben definiert und bevorzugt beide Methyl oder zusammen -(CH₂)₃-. Beispielhafte Verbindungen der Formeln I-LBI und I-LBII sind solche, in denen R¹¹ für einen geradkettigen Alkanylrest CₙH₂ₙ₊₁- mit n = 1, 2, 3, 4, 5, 6 oder 7 steht.

Ganz besonders bevorzugte erfindungsgemäße Verbindungen der Formel I-MB sind solche der Formeln I-MBA und I-MBB:

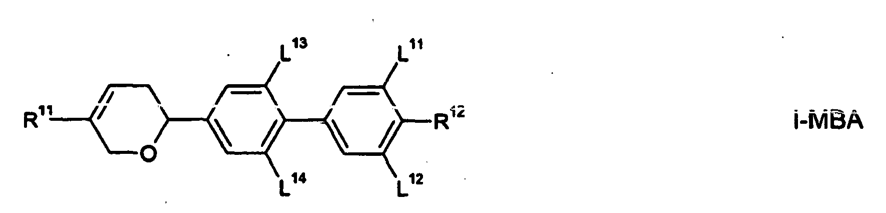

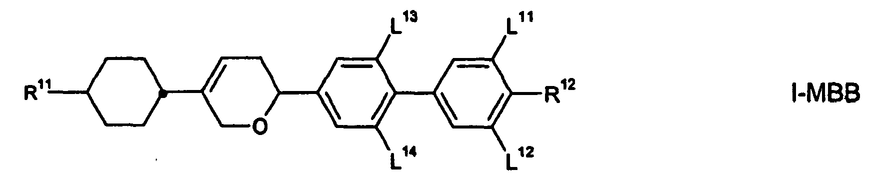

Dabei sind L¹³ und L¹⁴ unabhängig voneinander H oder F und R¹¹, R¹², L¹¹ und L¹² wie für Formel I oben definiert, wobei R¹¹ bevorzugt ein geradkettiger Alkenyl- (CₙH₂ₙ₋₁-) oder insbesondere Alkanylrest (CₙH₂ₙ₊₁-) mit 1 bis 7 Kohlenstoffatomen ist, im Falle von I-MBB auch H, während R¹² bevorzugt Halogen, insbesondere Fluor, oder ein gegebenenfalls mit Halogen, insbesondere Fluor, einfach oder mehrfach substituierter unverzweigter Alkanyl- oder Alkoxyrest mit 1 bis 5 Kohlenstoffatomen ist.

Beispielhafte Verbindungen der Formel I-MBA sind Verbindungen, die auch durch Formel I-CBIIa oben dargestellt werden, und beispielhafte Verbindungen der Formel I-MBB sind Verbindungen, die auch durch Formel I-DBAIIIa oben dargestellt werden.

Ganz besonders bevorzugte erfindungsgemäße Verbindungen der Formel I-NB sind solche der Formeln I-NBA, I-NBB, I-NBC und I-NBD:

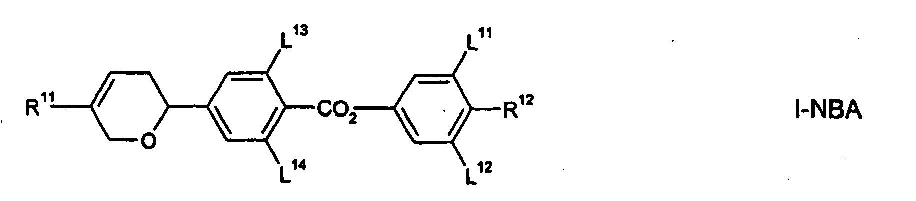

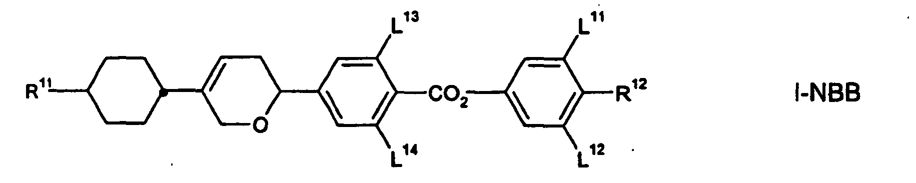

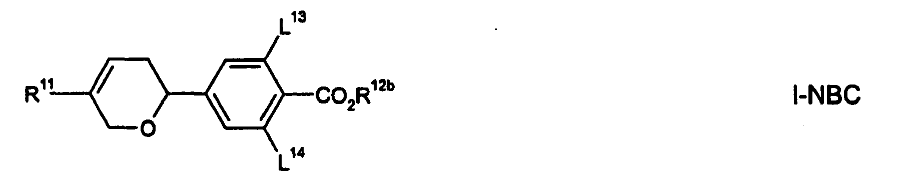

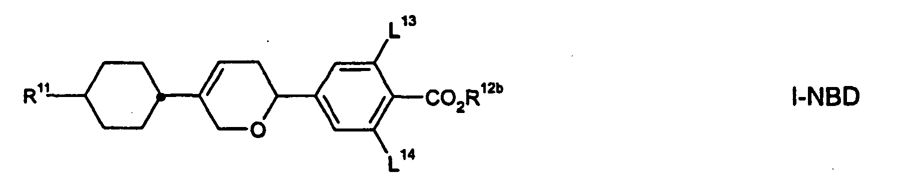

Dabei sind L¹³ und L¹⁴ unabhängig voneinander H oder F und R¹¹, R¹², L¹¹ und L¹² wie für Formel I oben definiert, wobei R¹¹ bevorzugt ein geradkettiger Alkenyl- (CₙH₂ₙ₋₁-) oder insbesondere Alkanylrest (CₙH₂ₙ₊₁-) mit 1 bis 7 Kohlenstoffatomen ist, im Falle von I-NBB und I-NBD auch H, während R¹² bevorzugt Halogen, insbesondere Fluor, oder ein gegebenenfalls mit Halogen, insbesondere Fluor, einfach oder mehrfach substituierter unverzweigter Alkanyl- oder Alkoxyrest mit 1 bis 5 Kohlenstoffatomen ist. R^{12b} hat die gleiche Bedeutung wie für Formel I-CBIIIb beziehungsweise I-GBIa oben.

Beispielhafte Verbindungen der Formel I-NBA sind Verbindungen, die auch durch Formel I-CBIIb oben dargestellt werden, und beispielhafte Verbindungen der Formel I-NBC sind Verbindungen, die auch durch Formel I-CBIIIb oben dargestellt werden. Beispielhafte Verbindungen der Formel I-NBB sind solche, die durch die Formel I-NBBIa wiedergegeben werden, wobei die Bedeutung für R¹², L¹¹, L¹², L¹³ und L¹⁴ sowie n (n ist 0, 1, 2, 3, 4, 5, 6 oder 7) in Tabelle 4 unten angegeben ist:

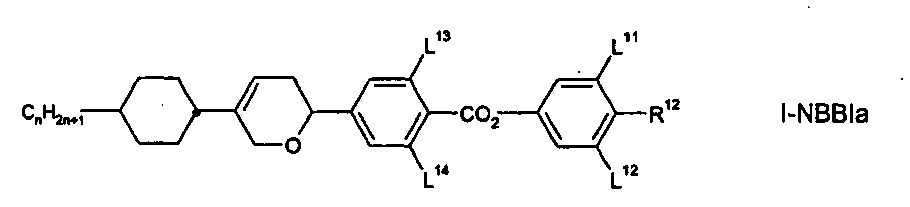

Ganz besonders bevorzugte erfindungsgemäße Verbindungen der Formel I-OB sind solche der Formeln I-OBA und I-OBB:

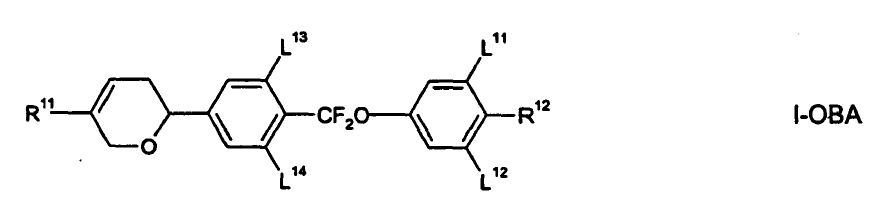

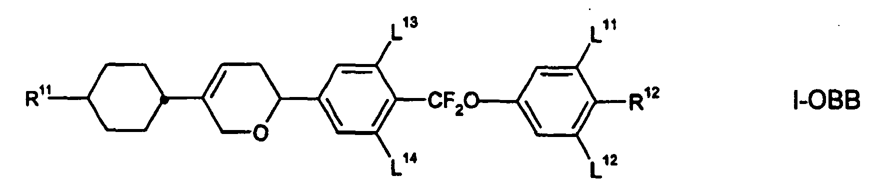

Dabei sind L¹³ und L¹⁴ unabhängig voneinander H oder F und R¹¹, R¹², L¹¹ und L¹² wie für Formel I oben definiert, wobei R¹¹ bevorzugt ein geradkettiger Alkenyl- (CₙH₂ₙ₋₁-) oder insbesondere Alkanylrest (CₙH₂ₙ₊₁-) mit 1 bis 7 Kohlenstoffatomen ist, im Falle von I-OBB auch H, während R¹² bevorzugt Halogen, insbesondere Fluor, oder ein gegebenenfalls mit Halogen, insbesondere Fluor, einfach oder mehrfach substituierter unverzweigter Alkanyl- oder Alkoxyrest mit 1 bis 5 Kohlenstoffatomen ist.

Beispielhafte Verbindungen der Formel I-OBA sind Verbindungen, die auch durch Formel I-CBIIc oben dargestellt werden, und beispielhafte Verbindungen der Formel I-OBB sind Verbindungen, die auch durch Formel I-DBAIIIb oben dargestellt werden.

Ganz besonders bevorzugte erfindungsgemäße Verbindungen der Formel I-PB sind solche der Formeln I-PBA und I-PBB:

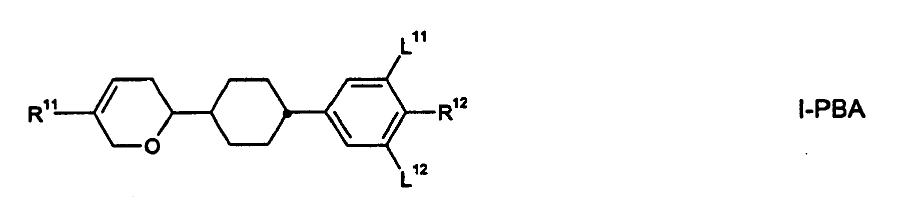

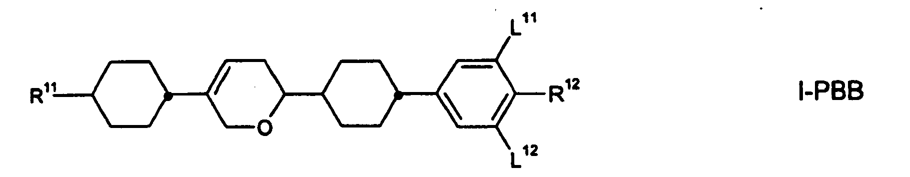

Dabei sind R¹¹, R¹², L¹¹ und L¹² wie für Formel I oben definiert, wobei R¹¹ bevorzugt ein geradkettiger Alkenyl- (CₙH₂ₙ₋₁-) oder insbesondere Alkanylrest (CₙH₂ₙ₊₁-) mit 1 bis 7 Kohlenstoffatomen ist, im Falle von I-PBB auch H, während R¹² bevorzugt Halogen, insbesondere Fluor, oder ein gegebenenfalls mit Halogen, insbesondere Fluor, einfach oder mehrfach substituierter unverzweigter Alkanyl- oder Alkoxyrest mit 1 bis 5 Kohlenstoffatomen ist.

Beispielhafte Verbindungen der Formel I-PBA sind Verbindungen, die auch durch Formel I-CBIa oben dargestellt werden, und beispielhafte Verbindungen der Formel I-PBB sind Verbindungen, die auch durch Formel I-DBAIIa oben dargestellt werden.

Ganz besonders bevorzugte erfindungsgemäße Verbindungen der Formel I-QB sind solche der Formeln I-QBA, I-QBB und I-QBC:

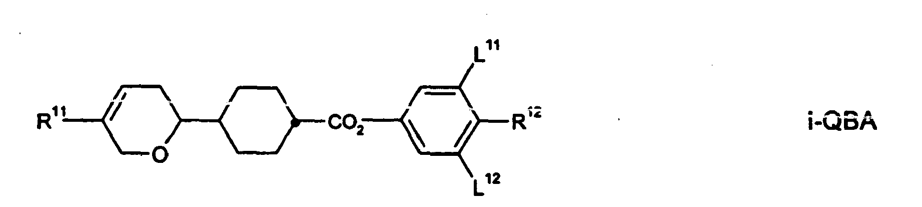

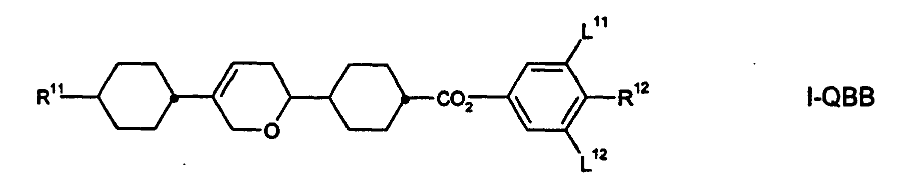

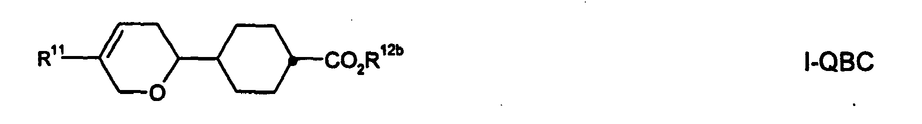

Dabei sind R¹¹, R¹², L¹¹ und L¹² wie für Formel I oben definiert, wobei R¹¹ bevorzugt ein geradkettiger Alkenyl- (CₙH₂ₙ₋₁-) oder insbesondere Alkanylrest (CₙH₂ₙ₊₁-) mit 1 bis 7 Kohlenstoffatomen ist, im Falle von I-QBB auch H, während R¹² bevorzugt Halogen, insbesondere Fluor, oder ein gegebenenfalls mit Halogen, insbesondere Fluor, einfach oder mehrfach substituierter unverzweigter Alkanyl- oder Alkoxyrest mit 1 bis 5 Kohlenstoffatomen ist. R^{12b} hat die gleiche Bedeutung wie für Formel I-CBIIIb oben.

Beispielhafte Verbindungen der Formel I-QBA sind Verbindungen, die auch durch Formel I-CBIb oben dargestellt werden. Beispielhafte Verbindungen der Formel I-QBB sind Verbindungen, die durch Formel I-QBBIa wiedergegeben werden, wobei die Bedeutung für R¹², L¹¹ und L¹² sowie n (n ist 0, 1, 2, 3, 4, 5, 6 oder 7) in Tabelle 3 unten angegeben ist:

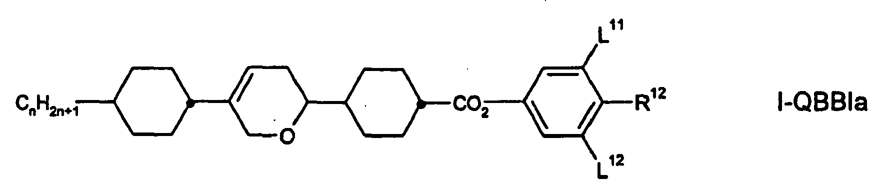

Ganz besonders bevorzugte erfindungsgemäße Verbindungen der Formel I-RB sind solche der Formeln I-RBA und I-RBB:

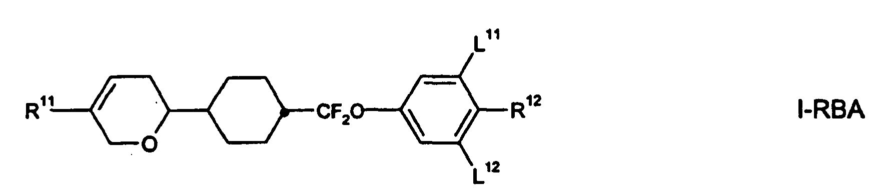

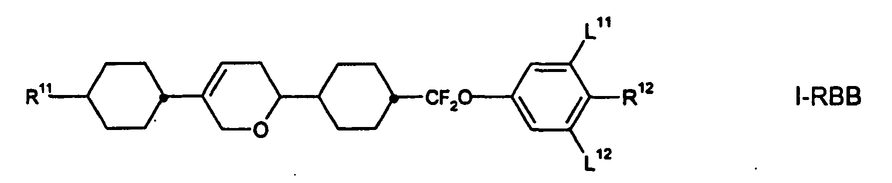

Dabei sind R¹¹, R¹², L¹¹ und L¹² wie für Formel I oben definiert, wobei R¹¹ bevorzugt ein geradkettiger Alkenyl- (CₙH₂ₙ₋₁-) oder insbesondere Alkanylrest (CₙH₂ₙ₊₁-) mit 1 bis 7 Kohlenstoffatomen ist, im Falle von I-RBB auch H, während R¹² bevorzugt Halogen, insbesondere Fluor, oder ein gegebenenfalls mit Halogen, insbesondere Fluor, einfach oder mehrfach substituierter unverzweigter Alkanyl- oder Alkoxyrest mit 1 bis 5 Kohlenstoffatomen ist.

Beispielhafte Verbindungen der Formel 1-RBA sind Verbindungen, die auch durch Formel I-CBIc oben dargestellt werden, und beispielhafte Verbindungen der Formel I-RBB sind Verbindungen, die auch durch Formel I-DBAIIb oben dargestellt werden.

Ganz besonders bevorzugte erfindungsgemäße Verbindungen der Formel I-SB sind solche der Formeln I-SBA und I-SBB: 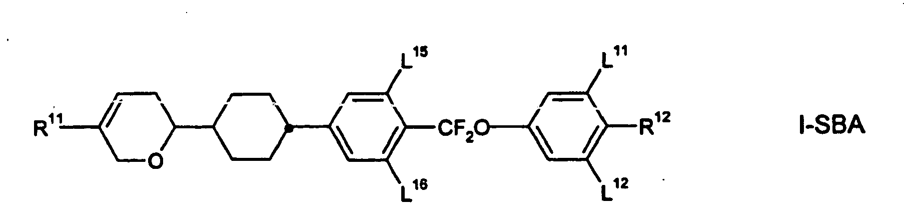 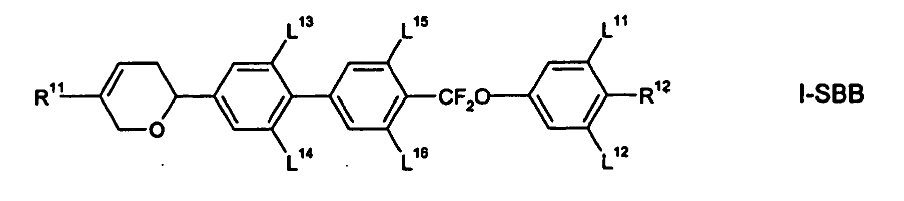 Dabei sind L¹³, L¹⁴, L¹⁵ und L¹⁶ unabhängig voneinander H oder F und R¹¹, R¹², L¹¹ und L¹² wie für Formel I oben definiert, wobei R¹¹ bevorzugt ein geradkettiger Alkenyl- (CₙH₂ₙ₋₁-) oder insbesondere Alkanylrest (CₙH₂ₙ₊₁-) mit 1 bis 7 Kohlenstoffatomen ist, während R¹² bevorzugt Halogen, insbesondere Fluor, oder ein gegebenenfalls mit Halogen, insbesondere Fluor, einfach oder mehrfach substituierter unverzweigter Alkanyl- oder Alkoxyrest mit 1 bis 5 Kohlenstoffatomen ist.

Beispielhafte Verbindungen der Formeln I-SBA und I-SBB sind Verbindungen, die durch Formel I-SBAIa beziehungsweise I-SBBIa wiedergegeben werden, wobei die Bedeutung für R¹², L¹¹, L¹² L¹³, L¹⁴, L¹⁵ und L¹⁶ sowie n (n ist 1, 2, 3, 4, 5, 6 oder 7) in den Tabellen 2 und 6 unten angegeben ist:

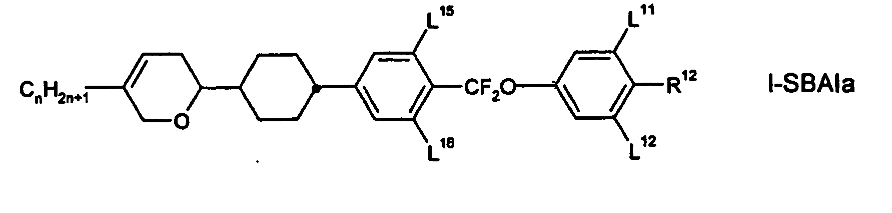

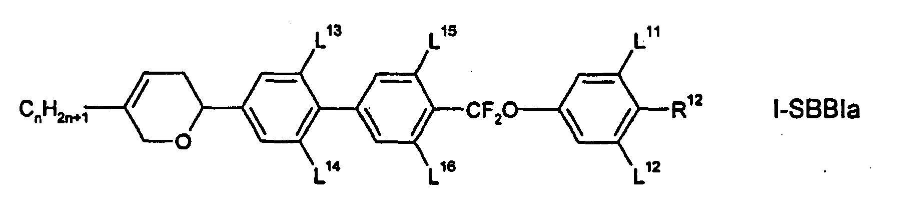

Ganz besonders bevorzugte erfindungsgemäße Verbindungen der Formel I-TB sind solche der Formel I-TBA:

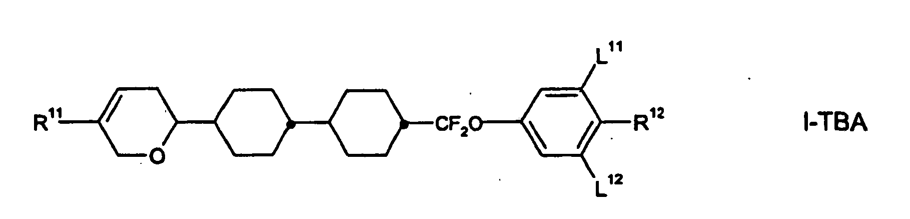

Dabei sind R¹¹, R¹² , L¹¹ und L¹² wie für Formel I oben definiert, wobei R¹¹ bevorzugt ein geradkettiger Alkenyl- (CₙH₂ₙ₋₁-) oder insbesondere Alkanylrest (CₙH₂ₙ₊₁-) mit 1 bis 7 Kohlenstoffatomen ist, während R¹² bevorzugt Halogen, insbesondere Fluor, oder ein gegebenenfalls mit Halogen, insbesondere Fluor, einfach oder mehrfach substituierter unverzweigter Alkanyl- oder Alkoxyrest mit 1 bis 5 Kohlenstoffatomen ist.

Beispielhafte Verbindungen der Formel I-TBA sind Verbindungen, die durch Formel 1-TBAIa wiedergegeben werden, wobei die Bedeutung für R¹², L¹¹ und L¹² sowie n (n ist 1, 2, 3, 4, 5, 6 oder 7) in Tabelle 1 unten angegeben ist:

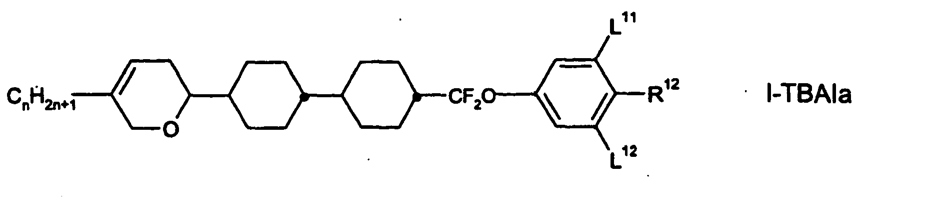

**Tabelle 1**

| **Verbindung** | **n*** | **R**^{**12**} | **L**^{**11**} | **L**^{**12**} |
|---|---|---|---|---|
| **I-CBIa-/I-CBIb-/I-CBIc-/I-GBIIa-/I-HBIa-/I-TBAIa-Nr.** | | | | |
| -1 | 1 | F | H | H |
| -2 | 1 | F | F | H |
| -3 | 1 | F | F | F |
| -4 | 1 | CF₃ | H | H |
| -5 | 1 | CF₃ | F | H |
| -6 | 1 | CF₃ | F | F |
| -7 | 1 | OCF₃ | H | H |
| -8 | 1 | OCF₃ | F | H |
| -9 | 1 | OCF₃ | F | F |
| -10 | 1 | OCHF₂ | H | H |
| -11 | 1 | OCHF₂ | F | H |
| -12 | 1 | OCHF₂ | F | F |
| -13 | 1 | -CN | H | H |
| -14 | 1 | -CN | F | H |
| -15 | 1 | -CN | F | F |
| -16 | 2 | F | H | H |
| -17 | 2 | F | F | H |
| -18 | 2 | F | F | F |
| -19 | 2 | CF₃ | H | H |
| -20 | 2 | CF₃ | F | H |
| -21 | 2 | CF₃ | F | F |
| -22 | 2 | OCF₃ | H | H |
| -23 | 2 | OCF₃ | F | H |
| -24 | 2 | OCF₃ | F | F |
| -25 | 2 | OCHF₂ | H | H |
| -26 | 2 | OCHF₂ | F | H |
| -27 | 2 | OCHF₂ | F | F |
| -28 | 2 | -CN | H | H |
| -29 | 2 | -CN | F | H |
| -30 | 2 | -CN | F | F |
| -31 | 3 | F | H | H |
| -32 | 3 | F | F | H |
| -33 | 3 | F | F | F |
| -34 | 3 | CF₃ | H | H |
| -35 | 3 | CF₃ | F | H |
| -36 | 3 | CF₃ | F | F |
| -37 | 3 | OCF₃ | H | H |
| -38 | 3 | OCF₃ | F | H |
| -39 | 3 | OCF₃ | F | F |
| -40 | 3 | OCHF₂ | H | H |
| -41 | 3 | OCHF₂ | F | H |
| -42 | 3 | OCHF₂ | F | F |
| -43 | 3 | -CN | H | H |
| -44 | 3 | -CN | F | H |
| -45 | 3 | -CN | F | F |
| -46 | 4 | F | H | H |
| -47 | 4 | F | F | H |
| -48 | 4 | F | F | F |
| -49 | 4 | CF₃ | H | H |
| -50 | 4 | CF₃ | F | H |
| -51 | 4 | CF₃ | F | F |
| -52 | 4 | OCF₃ | H | H |
| -53 | 4 | OCF₃ | F | H |
| -54 | 4 | OCF₃ | F | F |
| -55 | 4 | OCHF₂ | H | H |
| -56 | 4 | OCHF₂ | F | H |
| -57 | 4 | OCHF₂ | F | F |
| -58 | 4 | -CN | H | H |
| -59 | 4 | -CN | F | H |
| -60 | 4 | -CN | F | F |
| -61 | 5 | F | H | H |
| -62 | 5 | F | F | H |
| -63 | 5 | F | F | F |
| -64 | 5 | CF₃ | H | H |
| -65 | 5 | CF₃ | F | H |
| -66 | 5 | CF₃ | F | F |
| -67 | 5 | OCF₃ | H | H |
| -68 | 5 | OCF₃ | F | H |
| -69 | 5 | OCF₃ | F | F |
| -70 | 5 | OCHF₂ | H | H |
| -71 | 5 | OCHF₂ | F | H |
| -72 | 5 | OCHF₂ | F | F |
| -73 | 5 | -CN | H | H |
| -74 | 5 | -CN | F | H |
| -75 | 5 | -CN | F | F |

| | | | | |
|---|---|---|---|---|
| *: Der Rest CₙH₂ₙ₊₁ ist unverzweigt. | | | | |

**Tabelle 2**

| **Verbindung** | **n*** | **R**^{**12**} | **L**^{**11**} | **L**^{**12**} | **L**^{**13****} | **L**^{**14*****} |
|---|---|---|---|---|---|---|
| **I-CBIIa-/I-CBIIb-/I-CBIIc-/** | | | | | | |
| **I-GBIIIa-/I-HBIIa-/I-SBAIa-Nr.** | | | | | | |
| -1 | 1 | F | H | H H | | H |
| -2 | 1 | F | F | H | H | H |
| -3 | 1 | F | F | F | H | H |
| -4 | 1 | F | F | F | F | H |
| -5 | 1 | F | F | F | F | F |
| -6 | 1 | F | F | H | F | H |
| -7 | 1 | F | F | H | F | F |
| -8 | 1 | CF₃ | H | H | H | H |
| -9 | 1 | CF₃ | F | H | H | H |
| -10 | 1 | CF₃ | F | F | H | H |
| -11 | 1 | CF₃ | F | F | F | H |
| -12 | 1 | CF₃ | F | F | F | F |
| -13 | 1 | CF₃ | F | H | F | H |
| -14 | 1 | CF₃ | F | H | F | F |
| -15 | 1 | OCF₃ | H | H | H | H |
| -16 | 1 | OCF₃ | F | H | H | H |
| -17 | 1 | OCF₃ | F | F | H | H |
| -18 | 1 | OCF₃ | F | F | F | H |
| -19 | 1 | OCF₃ | F | F | F | F |
| -20 | 1 | OCF₃ | F | H | F | H |
| -21 | 1 | OCF₃ | F | H | F | F |
| -22 | 1 | OCHF₂ | H | H | H | H |
| -23 | 1 | OCHF₂ | F | H | H | H |
| -24 | 1 | OCHF₂ | F | F | H | H |
| -25 | 1 | OCHF₂ | F | F | F | H |
| -26 | 1 | OCHF₂ | F | F | F | F |
| -27 | 1 | OCHF₂ | F | H | F | H |
| -28 | 1 | OCHF₂ | F | H | F | F |
| -29 | 1 | -CN | H | H | H | H |
| -30 | 1 | -CN | F | H | H | H |
| -31 | 1 | -CN | F | F | H | H |
| -32 | 1 | -CN | F | F | F | H |
| -33 | 1 | -CN | F | F | F | F |
| -34 | 1 | -CN | F | H | F | H |
| -35 | 1 | -CN | F | H | F | F |
| -36 | 2 | F | H | H | H | H |
| -37 | 2 | F | F | H | H | H |
| -38 | 2 | F | F | F | H | H |
| -39 | 2 | F | F | F | F | H |
| -40 | 2 | F | F | F | F | F |
| -41 | 2 | F | F | H | F | H |
| -42 | 2 | F | F | H | F | F |
| -43 | 2 | CF₃ | H | H | H | H |
| -44 | 2 | CF₃ | F | H | H | H |
| -45 | 2 | CF₃ | F | F | H | H |
| -46 | 2 | CF₃ | F | F | F | H |
| -47 | 2 | CF₃ | F | F | F | F |
| -48 | 2 | CF₃ | F | H | F | H |
| -49 | 2 | CF₃ | F | H | F | F |
| -50 | 2 | OCF₃ | H | H | H | H |
| -51 | 2 | OCF₃ | F | H | H | H |
| -52 | 2 | OCF₃ | F | F | H | H |
| -53 | 2 | OCF₃ | F | F | F | H |
| -54 | 2 | OCF₃ | F | F | F | F |
| -55 | 2 | OCF₃ | F | H | F | H |
| -56 | 2 | OCF₃ | F | H | F | F |
| -57 | 2 | OCHF₂ | H | H | H | H |
| -58 | 2 | OCHF₂ | F | H | H | H |
| -59 | 2 | OCHF₂ | F | F | H | H |
| -60 | 2 | OCHF₂ | F | F | F | H |
| -61 | 2 | OCHF₂ | F | F | F | F |
| -62 | 2 | OCHF₂ | F | H | F | H |
| -63 | 2 | OCHF₂ | F | H | F | F |
| -64 | 2 | -CN | H | H | H | H |
| -65 | 2 | -CN | F | H | H | H |
| -66 | 2 | -CN | F | F | H | H |
| -67 | 2 | -CN | F | F | F | H |
| -68 | 2 | -CN | F | F | F | F |
| -69 | 2 | -CN | F | H | F | H |
| -70 | 2 | -CN | F | H | F | F |
| -71 | 3 | F | H | H | H | H |
| -72 | 3 | F | F | H | H | H |
| -73 | 3 | F | F | F | H | H |
| -74 | 3 | F | F | F | F | H |
| -75 | 3 | F | F | F | F | F |
| -76 | 3 | F | F | H | F | H |
| -77 | 3 | F | F | H | F | F |
| -78 | 3 | CF₃ | H | H | H | H |
| -79 | 3 | CF₃ | F | H | H | H |
| -80 | 3 | CF₃ | F | F | H | H |
| -81 | 3 | CF₃ | F | F | F | H |
| -82 | 3 | CF₃ | F | F | F | F |
| -83 | 3 | CF₃ | F | H | F | H |
| -84 | 3 | CF₃ | F | H | F | F |
| -85 | 3 | OCF₃ | H | H | H | H |
| -86 | 3 | OCF₃ | F | H | H | H |
| -87 | 3 | OCF₃ | F | F | H | H |
| -88 | 3 | OCF₃ | F | F | F | H |
| -89 | 3 | OCF₃ | F | F | F | F |
| -90 | 3 | OCF₃ | F | H | F | H |
| -91 | 3 | OCF₃ | F | H | F | F |
| -92 | 3 | OCHF₂ | H | H | H | H |
| -93 | 3 | OCHF₂ | F | H | H | H |
| -94 | 3 | OCHF₂ | F | F | H | H |
| -95 | 3 | OCHF₂ | F | F | F | H |
| -96 | 3 | OCHF₂ | F | F | F | F |
| -97 | 3 | OCHF₂ | F | H | F | H |
| -98 | 3 | OCHF₂ | F | H | F | F |
| -99 | 3 | -CN | H | H | H | H |
| -100 | 3 | -CN | F | H | H | H |
| -101 | 3 | -CN | F | F | H | H |
| -102 | 3 | -CN | F | F | F | H |
| -103 | 3 | -CN | F | F | F | F |
| -104 | 3 | -CN | F | H | F | H |
| -105 | 3 | -CN | F | H | F | F |
| -106 | 4 | F | H | H | H | H |
| -107 | 4 | F | F | H | H | H |
| -108 | 4 | F | F | F | H | H |
| -109 | 4 | F | F | F | F | H |
| -110 | 4 | F | F | F | F | F |
| -111 | 4 | F | F | H | F | H |
| -112 | 4 | F | F | H | F | F |
| -113 | 4 | CF₃ | H | H | H | H |
| -114 | 4 | CF₃ | F | H | H | H |
| -115 | 4 | CF₃ | F | F | H | H |
| -116 | 4 | CF₃ | F | F | F | H |
| -117 | 4 | CF₃ | F | F | F | F |
| -118 | 4 | CF₃ | F | H | F | H |
| -119 | 4 | CF₃ | F | H | F | F |
| -120 | 4 | OCF₃ | H | H | H | H |
| -121 | 4 | OCF₃ | F | H | H | H |
| -122 | 4 | OCF₃ | F | F | H | H |
| -123 | 4 | OCF₃ | F | F | F | H |
| -124 | 4 | OCF₃ | F | F | F | F |
| -125 | 4 | OCF₃ | F | H | F | H |
| -126 | 4 | OCF₃ | F | H | F | F |
| -127 | 4 | OCHF₂ | H | H | H | H |
| -128 | 4 | OCHF₂ | F | H | H | H |
| -129 | 4 | OCHF₂ | F | F | H | H |
| -130 | 4 | OCHF₂ | F | F | F | H |
| -131 | 4 | OCHF₂ | F | F | F | F |
| -132 | 4 | OCHF₂ | F | H | F | H |
| -133 | 4 | OCHF₂ | F | H | F | F |
| -134 | 4 | -CN | H | H | H | H |
| -135 | 4 | -CN | F | H | H | H |
| -136 | 4 | -CN | F | F | H | H |
| -137 | 4 | -CN | F | F | F | H |
| -138 | 4 | -CN | F | F | F | F |
| -139 | 4 | -CN | F | H | F | H |
| -140 | 4 | -CN | F | H | F | F |
| -141 | 5 | F | H | H | H | H |
| -142 | 5 | F | F | H | H | H |
| -143 | 5 | F | F | F | H | H |
| -144 | 5 | F | F | F | F | H |
| -145 | 5 | F | F | F | F | F |
| -146 | 5 | F | F | H | F | H |
| -147 | 5 | F | F | H | F | F |
| -148 | 5 | CF₃ | H | H | H | H |
| -149 | 5 | CF₃ | F | H | H | H |
| -150 | 5 | CF₃ | F | F | H | H |
| -151 | 5 | CF₃ | F | F | F | H |
| -152 | 5 | CF₃ | F | F | F | F |
| -153 | 5 | CF₃ | F | H | F | H |
| -154 | 5 | CF₃ | F | H | F | F |
| -155 | 5 | OCF₃ | H | H | H | H |
| -156 | 5 | OCF₃ | F | H | H | H |
| -157 | 5 | OCF₃ | F | F | H | H |
| -158 | 5 | OCF₃ | F | F | F | H |
| -159 | 5 | OCF₃ | F | F | F | F |
| -160 | 5 | OCF₃ | F | H | F | H |
| -161 | 5 | OCF₃ | F | H | F | F |
| -162 | 5 | OCHF₂ | H | H | H | H |
| -163 | 5 | OCHF₂ | F | H | H | H |
| -164 | 5 | OCHF₂ | F | F | H | H |
| -165 | 5 | OCHF₂ | F | F | F | H |
| -166 | 5 | OCHF₂ | F | F | F | F |
| -167 | 5 | OCHF₂ | F | H | F | H |
| -168 | 5 | OCHF₂ | F | H | F | F |
| -169 | 5 | -CN | H | H | H | H |
| -170 | 5 | -CN | F | H | H | H |
| -171 | 5 | -CN | F | F | H | H |
| -172 | 5 | -CN | F | F | F | H |
| -173 | 5 | -CN | F | F | F | F |
| -174 | 5 | -CN | F | H | F | H |
| -175 | 5 | -CN | F | H | F | F |

| | | | | | | |
|---|---|---|---|---|---|---|
| *: Der Rest CₙH₂ₙ₊₁ ist unverzweigt. **: L¹⁵ für die Verbindungen der Formel I-SBAIa ***: L¹⁶ für die Verbindungen der Formel I-SBAIa | | | | | | |

**Tabelle 3**

| **Verbindung** | **n*** | **R**^{**12**} | **L**^{**11**} | **L**^{**12**} |
|---|---|---|---|---|
| **I-DBAIa-/I-DBAIb-/I-DBAIIa-/I-DBAIIb-/I-EBCIa-** | | | | |
| **/I-QBBIa-Nr.** | | | | |
| -1 | 0 | F | H | H |
| -2 | 0 | F | F | H |
| -3 | 0 | F | F | F |
| -4 | 0 | CF₃ | H | H |
| -5 | 0 | CF₃ | F | H |
| -6 | 0 | CF₃ | F | F |
| -7 | 0 | OCF₃ | H | H |
| -8 | 0 | OCF₃ | F | H |
| -9 | 0 | OCF₃ | F | F |
| -10 | 0 | OCHF₂ | H | H |
| -11 | 0 | OCHF₂ | F | H |
| -12 | 0 | OCHF₂ | F | F |
| -13 | 0 | -CN | H | H |
| -14 | 0 | -CN | F | H |
| -15 | 0 | -CN | F | F |
| -16 | 1 | F | H | H |
| -17 | 1 | F | F | H |
| -18 | 1 | F | F | F |
| -19 | 1 | CF₃ | H | H |
| -20 | 1 | CF₃ | F | H |
| -21 | 1 | CF₃ | F | F |
| -22 | 1 | OCF₃ | H | H |
| -23 | 1 | OCF₃ | F | H |
| -24 | 1 | OCF₃ | F | F |
| -25 | 1 | OCHF₂ | H | H |
| -26 | 1 | OCHF₂ | F | H |
| -27 | 1 | OCHF₂ | F | F |
| -28 | 1 | -CN | H | H |
| -29 | 1 | -CN | F | H |
| -30 | 1 | -CN | F | F |
| -31 | 2 | F | H | H |
| -32 | 2 | F | F | H |
| -33 | 2 | F | F | F |
| -34 | 2 | CF₃ | H | H |
| -35 | 2 | CF₃ | F | H |
| -36 | 2 | CF₃ | F | F |
| -37 | 2 | OCF₃ | H | H |
| -38 | 2 | OCF₃ | F | H |
| -39 | 2 | OCF₃ | F | F |
| -40 | 2 | OCHF₂ | H | H |
| -41 | 2 | OCHF₂ | F | H |
| -42 | 2 | OCHF₂ | F | F |
| -43 | 2 | -CN | H | H |
| -44 | 2 | -CN | F | H |
| -45 | 2 | -CN | F | F |
| -46 | 3 | F | H | H |
| -47 | 3 | F | F | H |
| -48 | 3 | F | F | F |
| -49 | 3 | CF₃ | H | H |
| -50 | 3 | CF₃ | F | H |
| -51 | 3 | CF₃ | F | F |
| -52 | 3 | OCF₃ | H | H |
| -53 | 3 | OCF₃ | F | H |
| -54 | 3 | OCF₃ | F | F |
| -55 | 3 | OCHF₂ | H | H |
| -56 | 3 | OCHF₂ | F | H |
| -57 | 3 | OCHF₂ | F | F |
| -58 | 3 | -CN | H | H |
| -59 | 3 | -CN | F | H |
| -60 | 3 | -CN | F | F |
| -61 | 4 | F | H | H |
| -62 | 4 | F | F | H |
| -63 | 4 | F | F | F |
| -64 | 4 | CF₃ | H | H |
| -65 | 4 | CF₃ | F | H |
| -66 | 4 | CF₃ | F | F |
| -67 | 4 | OCF₃ | H | H |
| -68 | 4 | OCF₃ | F | H |
| -69 | 4 | OCF₃ | F | F |
| -70 | 4 | OCHF₂ | H | H |
| -71 | 4 | OCHF₂ | F | H |
| -72 | 4 | OCHF₂ | F | F |
| -73 | 4 | -CN | H | H |
| -74 | 4 | -CN | F | H |
| -75 | 4 | -CN | F | F |
| -76 | 5 | F | H | H |
| -77 | 5 | F | F | H |
| -78 | 5 | F | F | F |
| -79 | 5 | CF₃ | H | H |
| -80 | 5 | CF₃ | F | H |
| -81 | 5 | CF₃ | F | F |
| -82 | 5 | OCF₃ | H | H |
| -83 | 5 | OCF₃ | F | H |
| -84 | 5 | OCF₃ | F | F |
| -85 | 5 | OCHF₂ | H | H |
| -86 | 5 | OCHF₂ | F | H |
| -87 | 5 | OCHF₂ | F | F |
| -88 | 5 | -CN | H | H |
| -89 | 5 | -CN | F | H |
| -90 | 5 | -CN | F | F |

| | | | | |
|---|---|---|---|---|
| *: Der Rest CₙH₂ₙ₊₁ ist unverzweigt. | | | | |

**Tabelle 4**

| **Verbindung** | **n*** | **R**^{**12**} | **L**^{**11**} | **L**^{**12**} | **L**^{**13**} | **L**^{**14**} |
|---|---|---|---|---|---|---|
| **I-DBAIIIa-/I-DBAIIIb-/I-NBBIa-Nr.** | | | | | | |
| -1 | 0 | F | H | H | H | H |
| -2 | 0 | F | F | H | H | H |
| -3 | 0 | F | F | F | H | H |
| -4 | 0 | F | F | F | F | H |
| -5 | 0 | F | F | F | F | F |
| -6 | 0 | F | F | H | F | H |
| -7 | 0 | F | F | H | F | F |
| -8 | 0 | CF₃ | H | H | H | H |
| -9 | 0 | CF₃ | F | H | H | H |
| -10 | 0 | CF₃ | F | F | H | H |
| -11 | 0 | CF₃ | F | F | F | H |
| -12 | 0 | CF₃ | F | F | F | F |
| -13 | 0 | CF₃ | F | H | F | H |
| -14 | 0 | CF₃ | F | H | F | F |
| -15 | 0 | OCF₃ | H | H | H | H |
| -16 | 0 | OCF₃ | F | H | H | H |
| -17 | 0 | OCF₃ | F | F | H | H |
| -18 | 0 | OCF₃ | F | F | F | H |
| -19 | 0 | OCF₃ | F | F | F | F |
| -20 | 0 | OCF₃ | F | H | F | H |
| -21 | 0 | OCF₃ | F | H | F | F |
| -22 | 0 | OCHF₂ | H | H | H | H |
| -23 | 0 | OCHF₂ | F | H | H | H |
| -24 | 0 | OCHF₂ | F | F | H | H |
| -25 | 0 | OCHF₂ | F | F | F | H |
| -26 | 0 | OCHF₂ | F | F | F | F |
| -27 | 0 | OCHF₂ | F | H | F | H |
| -28 | 0 | OCHF₂ | F | H | F | F |
| -29 | 0 | -CN | H | H | H | H |
| -30 | 0 | -CN | F | H | H | H |
| -31 | 0 | -CN | F | F | H | H |
| -32 | 0 | -CN | F | F | F | H |
| -33 | 0 | -CN | F | F | F | F |
| -34 | 0 | -CN | F | H | F | H |
| -35 | 0 | -CN | F | H | F | F |
| -36 | 1 | F | H | H | H | H |
| -37 | 1 | F | F | H | H | H |
| -38 | 1 | F | F | F | H | H |
| -39 | 1 | F | F | F | F | H |
| -40 | 1 | F | F | F | F | F |
| -41 | 1 | F | F | H | F | H |
| -42 | 1 | F | F | H | F | F |
| -43 | 1 | CF₃ | H | H | H | H |
| -44 | 1 | CF₃ | F | H | H | H |
| -45 | 1 | CF₃ | F | F | H | H |
| -46 | 1 | CF₃ | F | F | F | H |
| -47 | 1 | CF₃ | F | F | F | F |
| -48 | 1 | CF₃ | F | H | F | H |
| -49 | 1 | CF₃ | F | H | F | F |
| -50 | 1 | OCF₃ | H | H | H | H |
| -51 | 1 | OCF₃ | F | H | H | H |
| -52 | 1 | OCF₃ | F | F | H | H |
| -53 | 1 | OCF₃ | F | F | F | H |
| -54 | 1 | OCF₃ | F | F | F | F |
| -55 | 1 | OCF₃ | F | H | F | H |
| -56 | 1 | OCF₃ | F | H | F | F |
| -57 | 1 | OCHF₂ | H | H | H | H |
| -58 | 1 | OCHF₂ | F | H | H | H |
| -59 | 1 | OCHF₂ | F | F | H | H |
| -60 | 1 | OCHF₂ | F | F | F | H |
| -61 | 1 | OCHF₂ | F | F | F | F |
| -62 | 1 | OCHF₂ | F | H | F | H |
| -63 | 1 | OCHF₂ | F | H | F | F |
| -64 | 1 | -CN | H | H | H | H |
| -65 | 1 | -CN | F | H | H | H |
| -66 | 1 | -CN | F | F | H | H |
| -67 | 1 | -CN | F | F | F | H |
| -68 | 1 | -CN | F | F | F | F |
| -69 | 1 | -CN | F | H | F | H |
| -70 | 1 | -CN | F | H | F | F |
| -71 | 2 | F | H | H | H | H |
| -72 | 2 | F | F | H | H | H |
| -73 | 2 | F | F | F | H | H |
| -74 | 2 | F | F | F | F | H |
| -75 | 2 | F | F | F | F | F |
| -76 | 2 | F | F | H | F | H |
| -77 | 2 | F | F | H | F | F |
| -78 | 2 | CF₃ | H | H | H | H |
| -79 | 2 | CF₃ | F | H | H | H |
| -80 | 2 | CF₃ | F | F | H | H |
| -81 | 2 | CF₃ | F | F | F | H |
| -82 | 2 | CF₃ | F | F | F | F |
| -83 | 2 | CF₃ | F | H | F | H |
| -84 | 2 | CF₃ | F | H | F | F |
| -85 | 2 | OCF₃ | H | H | H | H |
| -86 | 2 | OCF₃ | F | H | H | H |
| -87 | 2 | OCF₃ | F | F | H | H |
| -88 | 2 | OCF₃ | F | F | F | H |
| -89 | 2 | OCF₃ | F | F | F | F |
| -90 | 2 | OCF₃ | F | H | F | H |
| -91 | 2 | OCF₃ | F | H | F | F |
| -92 | 2 | OCHF₂ | H | H | H | H |
| -93 | 2 | OCHF₂ | F | H | H | H |
| -94 | 2 | OCHF₂ | F | F | H | H |
| -95 | 2 | OCHF₂ | F | F | F | H |
| -96 | 2 | OCHF₂ | F | F | F | F |
| -97 | 2 | OCHF₂ | F | H | F | H |
| -98 | 2 | OCHF₂ | F | H | F | F |
| -99 | 2 | -CN | H | H | H | H |
| -100 | 2 | -CN | F | H | H | H |
| -101 | 2 | -CN | F | F | H | H |
| -102 | 2 | -CN | F | F | F | H |
| -103 | 2 | -CN | F | F | F | F |
| -104 | 2 | -CN | F | H | F | H |
| -105 | 2 | -CN | F | H | F | F |
| -106 | 3 | F | H | H | H | H |
| -107 | 3 | F | F | H | H | H |
| -108 | 3 | F | F | F | H | H |
| -109 | 3 | F | F | F | F | H |
| -110 | 3 | F | F | F | F | F |
| -111 | 3 | F | F | H | F | H |
| -112 | 3 | F | F | H | F | F |
| -113 | 3 | CF₃ | H | H | H | H |
| -114 | 3 | CF₃ | F | H | H | H |
| -115 | 3 | CF₃ | F | F | H | H |
| -116 | 3 | CF₃ | F | F | F | H |
| -117 | 3 | CF₃ | F | F | F | F |
| -118 | 3 | CF₃ | F | H | F | H |
| -119 | 3 | CF₃ | F | H | F | F |
| -120 | 3 | OCF₃ | H | H | H | H |
| -121 | 3 | OCF₃ | F | H | H | W |
| -122 | 3 | OCF₃ | F | F | H | H |
| -123 | 3 | OCF₃ | F | F | F | H |
| -124 | 3 | OCF₃ | F | F | F | F |
| -125 | 3 | OCF₃ | F | H | F | H |
| -126 | 3 | OCF₃ | F | H | F | F |
| -127 | 3 | OCHF₂ | H | H | H | H |
| -128 | 3 | OCHF₂ | F | H | H | H |
| -129 | 3 | OCHF₂ | F | F | H | H |
| -130 | 3 | OCHF₂ | F | F | F | H |
| -131 | 3 | OCHF₂ | F | F | F | F |
| -132 | 3 | OCHF₂ | F | H | F | H |
| -133 | 3 | OCHF₂ | F F | H | F | F |
| -134 | 3 | -CN | H | H | H | H |
| -135 | 3 | -CN | F | H | H | H |
| -136 | 3 | -CN | F | F | H | H |
| -137 | 3 | -CN | F | F | F | H |
| -138 | 3 | -CN | F | F | F | F |
| -139 | 3 | -CN | F | H | F | H |
| -140 | 3 | -CN | F | H | F | F |
| -141 | 4 | F | H | H | H | H |
| -142 | 4 | F | F | H | H | H |
| -143 | 4 | F | F | F | H | H |
| -144 | 4 | F | F | F | F | H |
| -145 | 4 | F | F | F | F | F |
| -146 | 4 | F | F | H | F | H |
| -147 | 4 | F | F | H | F | F |
| -148 | 4 | CF₃ | H | H | H | H |
| -149 | 4 | CF₃ | F | H | H | H |
| -150 | 4 | CF₃ | F | F | H | H |
| -151 | 4 | CF₃ | F | F | F | H |
| -152 | 4 | CF₃ | F | F | F | F |
| -153 | 4 | CF₃ | F | H | F | H |
| -154 | 4 | CF₃ | F | H | F | F |
| -155 | 4 | OCF₃ | H | H | H | H |
| -156 | 4 | OCF₃ | F | H | H | H |
| -157 | 4 | OCF₃ | F | F | H | H |
| -158 | 4 | OCF₃ | F | F | F | H |
| -159 | 4 | OCF₃ | F | F | F | F |
| -160 | 4 | OCF₃ | F | H | F | H |
| -161 | 4 | OCF₃ | F | H | F | F |
| -162 | 4 | OCHF₂ | H | H | H | H |
| -163 | 4 | OCHF₂ | F | H | H | H |
| -164 | 4 | OCHF₂ | F | F | H | H |
| -165 | 4 | OCHF₂ | F | F | F | H |
| -166 | 4 | OCHF₂ | F | F | F | F |
| -167 | 4 | OCHF₂ | F | H | F | H |
| -168 | 4 | OCHF₂ | F | H | F | F |
| -169 | 4 | -CN | H | H | H | H |
| -170 | 4 | -CN | F | H | H | H |
| -171 | 4 | -CN | F | F | H | H |
| -172 | 4 | -CN | F | F | F | H |
| -173 | 4 | -CN | F F | F | F | F |
| -174 | 4 | -CN | F | H | F | H |
| -175 | 4 | -CN | F | H | F | F |
| -176 | 5 | F | H | H | H | H |
| -177 | 5 | F | F | H | H | H |
| -178 | 5 | F | F | F | H | H |
| -179 | 5 | F | F | F | F | H |
| -180 | 5 | F | F | F | F | F |
| -181 | 5 | F | F | H | F | H |
| -182 | 5 | F | F | H | F | F |
| -183 | 5 | CF₃ | H | H | H | H |
| -184 | 5 | CF₃ | F | H | H | H |
| -185 | 5 | CF₃ | F | F | H | H |
| -186 | 5 | CF₃ | F | F | F | H |
| -187 | 5 | CF₃ | F | F | F | F |
| -188 | 5 | CF₃ | F | H | F | H |
| -189 | 5 | CF₃ | F | H | F | F |
| -190 | 5 | OCF₃ | H | H | H | H |
| -191 | 5 | OCF₃ | F | H | H | H |
| -192 | 5 | OCF₃ | F | F | H | H |
| -193 | 5 | OCF₃ | F | F | F | H |
| -194 | 5 | OCF₃ | F | F | F | F |
| -195 | 5 | OCF₃ | F | H | F | H |
| -196 | 5 | OCF₃ | F | H | F | F |
| -197 | 5 | OCHF₂ | H | H | H | H |
| -198 | 5 | OCHF₂ | F | H | H | H |
| -199 | 5 | OCHF₂ | F | F | H | H |
| -200 | 5 | OCHF₂ | F | F | F | H |
| -201 | 5 | OCHF₂ | F | F | F | F |
| -202 | 5 | OCHF₂ | F | H | F | H |
| -203 | 5 | OCHF₂ | F | H | F | F |
| -204 | 5 | -CN | H | H | H | H |
| -205 | 5 | -CN | F | H | H | H |
| -206 | 5 | -CN | F | F | H | H |
| -207 | 5 | -CN | F | F | F | H |
| -208 | 5 | -CN | F | F | F | F |
| -209 | 5 | -CN | F | H | F | H |
| -210 | 5 | -CN | F | H | F | F |

**Tabelle 5**

| **Verbindung** | **n*** | **R**^{**12b**} | **Verbindung** | **n*** | **R**^{**12b**} |
|---|---|---|---|---|---|
| **I-GBIa** | | | **I-GBIa** | | |
| -1 | 0 | H | -2 | 0 | CH₃ |
| -3 | 0 | C₂H₅ | -4 | 0 | n-C₃H₇ |
| -5 | 0 | t-C₄H₉ | -6 | 0 | CH₂Phenyl |
| -7 | 1 | H | -8 | 1 | CH₃ |
| -9 | 1 | C₂H₅ | -10 | 1 | n-C₃H₇ |
| -11 | 1 | t-C₄H₉ | -12 | 1 | CH₂Phenyl |
| -13 | 3 | H | -14 | 3 | CH₃ |
| -15 | 3 | C₂H₅ | -16 | 3 | n-C₃H₇ |
| -17 | 3 | t-C₄H₉ | -18 | 3 | CH₂Phenyl |
| -19 | 5 | H | -20 | 5 | CH₃ |
| -21 | 5 | C₂H₅ | -22 | 5 | n-C₃H₇ |
| -23 | 5 | t-C₄H₉ | -24 | 5 | CH₂Phenyl |

| | | | | | |
|---|---|---|---|---|---|
| *: Der Rest CₙH₂ₙ₊₁ ist unverzweigt. | | | | | |

**Tabelle 6**

| **Verbindung I-SBBIa-Nr.** | **n*** | **R**^{**12**} | **L**^{**11**} | **L**^{**12**} | **L**^{**15**} | **L**^{**16**} | **L**^{**13**} | **L**^{**14**} |
|---|---|---|---|---|---|---|---|---|
| -1 | 1 | F | H | H | H | H | H | H |
| -2 | 1 | F | F | H | H | H | H | H |
| -3 | 1 | F | F | F | H | H | H | H |
| -4 | 1 | F | F | F | F | H | H | H |
| -5 | 1 | F | F | F | F | F | H | H |
| -6 | 1 | F | F | H | F | H | H | H |
| -7 | 1 | F | F | H | F | F | H | H |
| -8 | 1 | F | H | H | H | H | F | H |
| -9 | 1 | F | F | H | H | H | F | H |
| -10 | 1 | F | F | F | H | H | F | H |
| -11 | 1 | F | F | F | F | H | F | H |
| -12 | 1 | F | F | F | F | F | F | H |
| -13 | 1 | F | F | H | F | H | F | H |
| -14 | 1 | F | F | H | F | F | F | H |
| -15 | 1 | CF₃ | H | H | H | H | H | H |
| -16 | 1 | CF₃ | F | H | H | H | H | H |
| -17 | 1 | CF₃ | F | F | H | H | H | H |
| -18 | 1 | CF₃ | F | F | F | H | H | H |
| -19 | 1 | CF₃ | F | F | F | F | H | H |
| -20 | 1 | CF₃ | F | H | F | H | H | H |
| -21 | 1 | CF₃ | F | H | F | F | H | H |
| -22 | 1 | CF₃ | H | H | H | H | F | H |
| -23 | 1 | CF₃ | F | H | H | H | F | H |
| -24 | 1 | CF₃ | F | F | H | H | F | H |
| -25 | 1 | CF₃ | F | F | F | H | F | H |
| -26 | 1 | CF₃ | F | F | F | F | F | H |
| -27 | 1 | CF₃ | F | H | F | H | F | H |
| -28 | 1 | CF₃ | F | H | F | F | F | H |
| -29 | 1 | OCF₃ | H | H | H | H | H | H |
| -30 | 1 | OCF₃ | F | H | H | H | H | H |
| -31 | 1 | OCF₃ | F | F | H | H | H | H |
| -32 | 1 | OCF₃ | F | F | F | H | H | H |
| -33 | 1 | OCF₃ | F | F | F | F | H | H |
| -34 | 1 | OCF₃ | F | H | F | H | H | H |
| -35 | 1 | OCF₃ | F | H | F | F | H | H |
| -36 | 1 | OCF₃ | H | H | H | H | F | H |
| -37 | 1 | OCF₃ | F | H | H | H | F | H |
| -38 | 1 | OCF₃ | F | F | H | H | F | H |
| -39 | 1 | OCF₃ | F | F | F | H | F | H |
| -40 | 1 | OCF₃ | F | F | F | F | F | H |
| -41 | 1 | OCF₃ | F | H | F | H | F | H |
| -42 | 1 | OCF₃ | F | H | F | F | F | H |
| -43 | 1 | OCHF₂ | H | H | H | H | H | H |
| -44 | 1 | OCHF₂ | F | H | H | H | H | H |
| -45 | 1 | OCHF₂ | F | F | H | H | H | H |
| -46 | 1 | OCHF₂ | F | F | F | H | H | H |
| -47 | 1 | OCHF₂ | F | F | F | F | H | H |
| -48 | 1 | OCHF₂ | F | H | F | H | H | H |
| -49 | 1 | OCHF₂ | F | H | F | F | H | H |
| -50 | 1 | OCHF₂ | H | H | H | H | F | H |
| -51 | 1 | OCHF₂ | F | H | H | H | F | H |
| -52 | 1 | OCHF₂ | F | F | H | H | F | H |
| -53 | 1 | OCHF₂ | F | F | F | H | F | H |
| -54 | 1 | OCHF₂ | F | F | F | F | F | H |
| -55 | 1 | OCHF₂ | F | H | F | H | F | H |
| -56 | 1 | OCHF₂ | F | H | F | F | F | H |
| -57 | 1 | -CN | H | H | H | H | H | H |
| -58 | 1 | -CN | F | H | H | H | H | H |
| -59 | 1 | -CN | F | F | H | H | H | H |
| -60 | 1 | -CN | F | F | F | H | H | H |
| -61 | 1 | -CN | F | F | F | F | H | H |
| -62 | 1 | -CN | F | H | F | H | H | H |
| -63 | 1 | -CN | F | H | F | F | H | H |
| -64 | 1 | -CN | H | H | H | H | F | H |
| -65 | 1 | -CN | F | H | H | H | F | H |
| -66 | 1 | -CN | F | F | H | H | F | H |
| -67 | 1 | -CN | F | F | F | H | F | H |
| -68 | 1 | -CN | F | F | F | F | F | H |
| -69 | 1 | -CN | F | H | F | H | F | H |
| -70 | 1 | -CN | F | H | F | F | F | H |
| -71 | 2 | F | H | H | H | H | H | H |
| -72 | 2 | F | F | H | H | H | H | H |
| -73 | 2 | F | F | F | H | H | H | H |
| -74 | 2 | F | F | F | F | H | H | H |
| -75 | 2 | F | F | F | F | F | H | H |
| -76 | 2 | F | F | H | F | H | H | H |
| -77 | 2 | F | F | H | F | F | H | H |
| -78 | 2 | F | H | H | H | H | F | H |
| -79 | 2 | F | F | H | H | H F | | H |
| -80 | 2 | F | F | F | H | H | F | H |
| -81 | 2 | F | F | F | F | H | F | H |
| -82 | 2 | F | F | F | F | F | F | H |
| -83 | 2 | F | F | H | F | H | F | H |
| -84 | 2 | F | F | H | F | F | F | H |
| -85 | 2 | CF₃ | H | H | H | H | H | H |
| -86 | 2 | CF₃ | F | H | H | H | H | H |
| -87 | 2 | CF₃ | F | F | H | H | H | H |
| -88 | 2 | CF₃ | F | F | F | H | H | H |
| -89 | 2 | CF₃ | F | F | F | F | H | H |
| -90 | 2 | CF₃ | F | H | F | H | H | H |
| -91 | 2 | CF₃ | F | H | F | F | H | H |
| -92 | 2 | CF₃ | H | H | H | H | F | H |
| -93 | 2 | CF₃ | F | H | H | H | F | H |
| -94 | 2 | CF₃ | F | F | H | H | F | H |
| -95 | 2 | CF₃ | F | F | F | H | F | H |
| -96 | 2 | CF₃ | F | F | F | F | F | H |
| -97 | 2 | CF₃ | F | H | F | H | F | H |
| -98 | 2 | CF₃ | F | H | F | F | F | H |
| -99 | 2 | OCF₃ | H | H | H | H | H | H |
| -100 | 2 | OCF₃ | F | H | H | H | H | H |
| -101 | 2 | OCF₃ | F | F | H | H | H | H |
| -102 | 2 | OCF₃ | F | F | F | H | H | H |
| -103 | 2 | OCF₃ | F | F | F | F | H | H |
| -104 | 2 | OCF₃ | F | H | F | H | H | H |
| -105 | 2 | OCF₃ | F | H | F | F | H | H |
| -106 | 2 | OCF₃ | H | H | H | H | F | H |
| -107 | 2 | OCF₃ | F | H | H | H | F | H |
| -108 | 2 | OCF₃ | F | F | H | H | F | H |
| -109 | 2 | OCF₃ | F | F | F | H | F | H |
| -110 | 2 | OCF₃ | F | F | F | F | F | H |
| -111 | 2 | OCF₃ | F | H | F | H | F | H |
| -112 | 2 | OCF₃ | F | H | F | F | F | H |
| -113 | 2 | OCHF₂ | H | H | H | H | H | H |
| -114 | 2 | OCHF₂ | F | H | H | H | H | H |
| -115 | 2 | OCHF₂ | F | F | H | H | H | H |
| -116 | 2 | OCHF₂ | F | F | F | H | H | H |
| -117 | 2 | OCHF₂ | F | F | F | F | H | H |
| -118 | 2 | OCHF₂ | F | H | F | H | H | H |
| -119 | 2 | OCHF₂ | F | H | F | F | H | H |
| -120 | 2 | OCHF₂ | H | H | H | H | F | H |
| -121 | 2 | OCHF₂ | F | H | H | H | F | H |
| -122 | 2 | OCHF₂ | F | F | H | H | F | H |
| -123 | 2 | OCHF₂ | F | F | F | H | F | H |
| -124 | 2 | OCHF₂ | F | F | F | F | F | H |
| -125 | 2 | OCHF₂ | F | H | F | H | F | H |
| -126 | 2 | OCHF₂ | F | H | F | F | F | H |
| -127 | 2 | -CN | H | H | H | H | H | H |
| -128 | 2 | -CN | F | H | H | H | H | H |
| -129 | 2 | -CN | F | F | H | H | H | H |
| -130 | 2 | -CN | F | F | F | H | H | H |
| -131 | 2 | -CN | F | F | F | F | H | H |
| -132 | 2 | -CN | F | H | F | H | H | H |
| -133 | 2 | -CN | F | H | F | F | H | H |
| -134 | 2 | -CN | H | H | H | H | F | H |
| -135 | 2 | -CN | F | H | H | H | F | H |
| -136 | 2 | -CN | F | F | H | H | F | H |
| -137 | 2 | -CN | F | F | F | H | F | H |
| -138 | 2 | -CN | F | F | F | F | F | H |
| -139 | 2 | -CN | F | H | F | H | F | H |
| -140 | 2 | -CN | F | H | F | F | F | H |
| -141 | 3 | F | H | H | H | H | H | H |
| -142 | 3 | F | F | H | H | H | H | H |
| -143 | 3 | F | F | F | H | H | H | H |
| -144 | 3 | F | F | F | F | H | H | H |
| -145 | 3 | F | F | F | F | F | H | H |
| -146 | 3 | F | F | H | F | H | H | H |
| -147 | 3 | F | F | H | F | F | H | H |
| -148 | 3 | F | H | H | H | H | F | H |
| -149 | 3 | F | F | H | H | H | F | H |
| -150 | 3 | F | F | F | H | H | F | H |
| -151 | 3 | F | F | F | F | H | F | H |
| -152 | 3 | F | F | F | F | F | F | H |
| -153 | 3 | F | F | H | F | H | F | H |
| -154 | 3 | F | F | H | F | F | F | H |
| -155 | 3 | CF₃ | H | H | H | H | H | H |
| -156 | 3 | CF₃ | F | H | H | H | H | H |
| -157 | 3 | CF₃ | F | F | H | H | H | H |
| -158 | 3 | CF₃ | F | F | F | H | H | H |
| -159 | 3 | CF₃ | F | F | F | F | H | H |
| -160 | 3 | CF₃ | F | H | F | H | H | H |
| -161 | 3 | CF₃ | F | H | F | F | H | H |
| -162 | 3 | CF₃ | H | H | H | H | F | H |
| -163 | 3 | CF₃ | F | H | H | H | F | H |
| -164 | 3 | CF₃ | F | F | H | H | F | H |
| -165 | 3 | CF₃ | F | F | F | H | F | H |
| -166 | 3 | CF₃ | F | F | F | F | F | H |
| -167 | 3 | CF₃ | F | H | F | H | F | H |
| -168 | 3 | CF₃ | F | H | F | F | F | H |
| -169 | 3 | OCF₃ | H | H | H | H | H | H |
| -170 | 3 | OCF₃ | F | H | H | H | H | H |
| -171 | 3 | OCF₃ | F | F | H | H | H | H |
| -172 | 3 | OCF₃ | F | F | F | H | H | H |
| -173 | 3 | OCF₃ | F | F | F | F | H | H |
| -174 | 3 | OCF₃ | F | H | F | H | H | H |
| -175 | 3 | OCF₃ | F | H | F | F | H | H |
| -176 | 3 | OCF₃ | H | H | H | H | F | H |
| -177 | 3 | OCF₃ | F | H | H | H | F | H |
| -178 | 3 | OCF₃ | F | F | H | H | F | H |
| -179 | 3 | OCF₃ | F | F | F | H | F | H |
| -180 | 3 | OCF₃ | F | F | F | F | F | H |
| -181 | 3 | OCF₃ | F | H | F | H | F | H |
| -182 | 3 | OCF₃ | F | H | F | F | F | H |
| -183 | 3 | OCHF₂ | H | H | H | H | H | H |
| -184 | 3 | OCHF₂ | F | H | H | H | H | H |
| -185 | 3 | OCHF₂ | F | F | H | H | H | H |
| -186 | 3 | OCHF₂ | F | F | F | H | H | H |
| -187 | 3 | OCHF₂ | F | F | F | F | H | H |
| -188 | 3 | OCHF₂ | F | H | F | H | H | H |
| -189 | 3 | OCHF₂ | F | H | F | F | H | H |
| -190 | 3 | OCHF₂ | H | H | H | H | F | H |
| -191 | 3 | OCHF₂ | F | H | H | H | F | H |
| -192 | 3 | OCHF₂ | F | F | H | H | F | H |
| -193 | 3 | OCHF₂ | F | F | F | H | F | H |
| -194 | 3 | OCHF₂ | F | F | F | F | F | H |
| -195 | 3 | OCHF₂ | F | H | F | H | F | H |
| -196 | 3 | OCHF₂ | F | H | F | F | F | H |
| -197 | 3 | -CN | H | H | H | H | H | H |
| -198 | 3 | -CN | F | H | H | H | H | H |
| -199 | 3 | -CN | F | F | H | H | H | H |
| -200 | 3 | -CN | F | F | F | H | H | H |
| -201 | 3 | -CN | F | F | F | F | H | H |
| -202 | 3 | -CN | F | H | F | H | H | H |
| -203 | 3 | -CN | F | H | F | F | H | H |
| -204 | 3 | -CN | H | H | H | H | F | H |
| -205 | 3 | -CN | F | H | H | H | F | H |
| -206 | 3 | -CN | F | F | H | H | F | H |
| -207 | 3 | -CN | F | F | F | H | F | H |
| -208 | 3 | -CN | F | F | F | F | F | H |
| -209 | 3 | -CN | F | H | F | H | F | H |
| -210 | 3 | -CN | F | H | F | F | F | H |
| -211 | 4 | F | H | H | H | H | H | H |
| -212 | 4 | F | F | H | H | H | H | H |
| -213 | 4 | F | F | F | H | H | H | H |
| -214 | 4 | F | F | F | F | H | H | H |
| -215 | 4 | F | F F | F | F | F | H | H |
| -216 | 4 | F | F | H | F | H | H | H |
| -217 | 4 | F | F | H | F | F | H | H |
| -218 | 4 | F | H | H | H | H | F | H |
| -219 | 4 | F | F | H | H | H | F | H |
| -220 | 4 | F | F | F | H | H | F | H |
| -221 | 4 | F | F | F | F | H | F | H |
| -222 | 4 | F | F | F | F | F | F | H |
| -223 | 4 | F | F | H | F | H | F | H |
| -224 | 4 | F | F | H | F | F | F | H |
| -225 | 4 | CF₃ | H | H | H | H | H | H |
| -226 | 4 | CF₃ | F | H | H | H | H | H |
| -227 | 4 | CF₃ | F | F | H | H | H | H |
| -228 | 4 | CF₃ | F | F | F | H | H | H |
| -229 | 4 | CF₃ | F | F | F | F | H | H |
| -230 | 4 | CF₃ | F | H | F | H | H | H |
| -231 | 4 | CF₃ | F | H | F | F | H | H |
| -232 | 4 | CF₃ | H | H | H | H | F | H |
| -233 | 4 | CF₃ | F | H | H | H | F | H |
| -234 | 4 | CF₃ | F | F | H | H | F | H |
| -235 | 4 | CF₃ | F | F | F | H | F | H |
| -236 | 4 | CF₃ | F | F | F | F | F | H |
| -237 | 4 | CF₃ | F | H | F | H | F | H |
| -238 | 4 | CF₃ | F | H | F | F | F | H |
| -239 | 4 | OCF₃ | H | H | H | H | H | H |
| -240 | 4 | OCF₃ | F | H | H | H | H | H |
| -241 | 4 | OCF₃ | F | F | H | H | H | H |
| -242 | 4 | OCF₃ | F | F | F | H | H | H |
| -243 | 4 | OCF₃ | F | F | F | F | H | H |
| -244 | 4 | OCF₃ | F | H | F | H | H | H |
| -245 | 4 | OCF₃ | F | H | F | F | H | H |
| -246 | 4 | OCF₃ | H | H | H | H | F | H |
| -247 | 4 | OCF₃ | F | H | H | H | F | H |
| -248 | 4 | OCF₃ | F | F | H | H | F | H |
| -249 | 4 | OCF₃ | F | F | F | H | F | H |
| -250 | 4 | OCF₃ | F | F | F | F | F | H |
| -251 | 4 | OCF₃ | F | H | F | H | F | H |
| -252 | 4 | OCF₃ | F | H | F | F | F | H |
| -253 | 4 | OCHF₂ | H | H | H | H | H | H |
| -254 | 4 | OCHF₂ | F | H | H | H | H | H |
| -255 | 4 | OCHF₂ | F | F | H | H | H | H |
| -256 | 4 | OCHF₂ | F | F | F | H | H | H |
| -257 | 4 | OCHF₂ | F | F | F | F | H | H |
| -258 | 4 | OCHF₂ | F | H | F | H | H | H |
| -259 | 4 | OCHF₂ | F | H | F | F | H | H |
| -260 | 4 | OCHF₂ | H | H | H | H | F | H |
| -261 | 4 | OCHF₂ | F | H | H | H | F | H |
| -262 | 4 | OCHF₂ | F | F | H | H | F | H |
| -263 | 4 | OCHF₂ | F | F | F | H | F | H |
| -264 | 4 | OCHF₂ | F | F | F | F | F | H |
| -265 | 4 | OCHF₂ | F | H | F | H | F | H |
| -266 | 4 | OCHF₂ | F | H | F | F | F | H |
| -267 | 4 | -CN | H | H | H | H | H | H |
| -268 | 4 | -CN | F | H | H | H | H | H |
| -269 | 4 | -CN | F | F | H | H | H | H |
| -270 | 4 | -CN | F | F | F | H | H | H |
| -271 | 4 | -CN | F | F | F | F | H | H |
| -272 | 4 | -CN | F | H | F | H | H | H |
| -273 | 4 | -CN | F | H | F | F | H | H |
| -274 | 4 | -CN | H | H | H | H | F | H |
| -275 | 4 | -CN | F | H | H | H | F | H |
| -276 | 4 | -CN | F | F | H | H | F | H |
| -277 | 4 | -CN | F | F | F | H | F | H |
| -278 | 4 | -CN | F | F | F | F | F | H |
| -279 | 4 | -CN | F | H | F | H | F | H |
| -280 | 4 | -CN | F | H | F | F | F | H |
| -281 | 5 | F | H | H | H | H | H | H |
| -282 | 5 | F | F | H | H | H | H | H |
| -283 | 5 | F | F | F | H | H | H | H |
| -284 | 5 | F | F | F | F | H | H | H |
| -285 | 5 | F | F | F | F | F | H | H |
| -286 | 5 | F | F | H | F | H | H | H |
| -287 | 5 | F | F | H | F | F | H | H |
| -288 | 5 | F | H | H | H | H | F | H |
| -289 | 5 | F | F | H | H | H | F | H |
| -290 | 5 | F | F | F | H | H | F | H |
| -291 | 5 | F | F | F | F | H | F | H |
| -292 | 5 | F | F | F | F | F | F | H |
| -293 | 5 | F | F | H | F | H | F | H |
| -294 | 5 | F | F | H | F | F | F | H |
| -295 | 5 | CF₃ | H | H | H | H | H | H |
| -296 | 5 | CF₃ | F | H | H | H | H | H |
| -297 | 5 | CF₃ | F | F | H | H | H | H |
| -298 | 5 | CF₃ | F | F | F | H | H | H |
| -299 | 5 | CF₃ | F | F | F | F | H | H |
| -300 | 5 | CF₃ | F | H | F | H | H | H |
| -301 | 5 | CF₃ | F | H | F | F | H | H |
| -302 | 5 | CF₃ | H | H | H | H | F | H |
| -303 | 5 | CF₃ | F | H | H | H | F | H |
| -304 | 5 | CF₃ | F | F | H | H | F | H |
| -305 | 5 | CF₃ | F F | F | F | H | F | H |
| -306 | 5 | CF₃ | F | F | F | F | F | H |
| -307 | 5 | CF₃ | F | H | F | H | F | H |
| -308 | 5 | CF₃ | F | H | F | F | F | H |
| -309 | 5 | OCF₃ | H | H | H | H | H | H |
| -310 | 5 | OCF₃ | F | H | H | H | H | H |
| -311 | 5 | OCF₃ | F | F | H | H | H | H |
| -312 | 5 | OCF₃ | F | F | F | H | H | H |
| -313 | 5 | OCF₃ | F | F | F | F | H | H |
| -314 | 5 | OCF₃ | F | H | F | H | H | H |
| -315 | 5 | OCF₃ | F | H | F | F | H | H |
| -316 | 5 | OCF₃ | H | H | H | H | F | H |
| -317 | 5 | OCF₃ | F | H | H | H | F | H |
| -318 | 5 | OCF₃ | F | F | H | H | F | H |
| -319 | 5 | OCF₃ | F | F | F | H | F | H |
| -320 | 5 | OCF₃ | F | F | F | F | F | H |
| -321 | 5 | OCF₃ | F | H | F | H | F | H |
| -322 | 5 | OCF₃ | F | H | F | F | F | H |
| -323 | 5 | OCHF₂ | H | H | H | H | H | H |
| -324 | 5 | OCHF₂ | F | H | H | H | H | H |
| -325 | 5 | OCHF₂ | F | F | H | H | H | H |
| -326 | 5 | OCHF₂ | F | F | F | H | H | H |
| -327 | 5 | OCHF₂ | F | F | F | F | H | H |
| -328 | 5 | OCHF₂ | F | H | F | H | H | H |
| -329 | 5 | OCHF₂ | F | H | F | F | H | H |
| -330 | 5 | OCHF₂ | H | H | H | H | F | H |
| -331 | 5 | OCHF₂ | F | H | H | H | F | H |
| -332 | 5 | OCHF₂ | F | F | H | H | F | H |
| -333 | 5 | OCHF₂ | F | F | F | H | F | H |
| -334 | 5 | OCHF₂ | F | F | F | F | F | H |
| -335 | 5 | OCHF₂ | F | H | F | H | F | H |
| -336 | 5 | OCHF₂ | F | H | F | F | F | H |
| -337 | 5 | -CN | H | H | H | H | H | H |
| -338 | 5 | -CN | F | H | H | H | H | H |
| -339 | 5 | -CN | F | F | H | H | H | H |
| -340 | 5 | -CN | F | F | F | H | H | H |
| -341 | 5 | -CN | F | F | F | F | H | H |
| -342 | 5 | -CN | F | H | F | H | H | H |
| -343 | 5 | -CN | F | H | F | F | H | H |
| -344 | 5 | -CN | H | H | H | H | F | H |
| -345 | 5 | -CN | F | H | H | H | F | H |
| -346 | 5 | -CN | F | F | H | H | F | H |
| -347 | 5 | -CN | F | F | F | H | F | H |
| -348 | 5 | -CN | F | F | F | F | F | H |
| -349 | 5 | -CN | F | H | F | H | F | H |
| -350 | 5 | -CN | F | H | F | F | F | H |

| | | | | | | | | |
|---|---|---|---|---|---|---|---|---|
| *: Der Rest CₙH₂ₙ₊₁ ist unverzweigt. | | | | | | | | |

Ein weiterer erfindungsgemäßer Gegenstand ist ein Verfahren zur Herstellung von Pyranderivaten, insbesondere der allgemeinen Formel I.

Das erfindungsgemäße Verfahren ist dadurch gekennzeichnet, dass es als einen Verfahrensschritt eine ringschließende Metathese-Reaktion einer Verbindung der allgemeinen Formel II 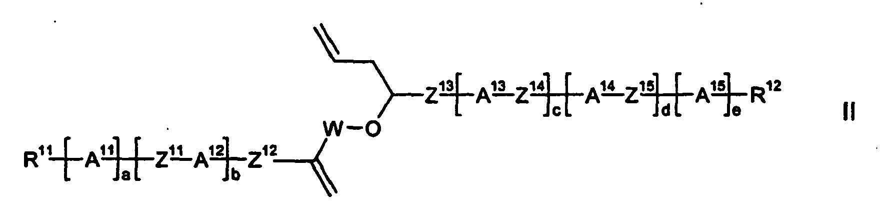 zu einem Pyranderivat der Formel I 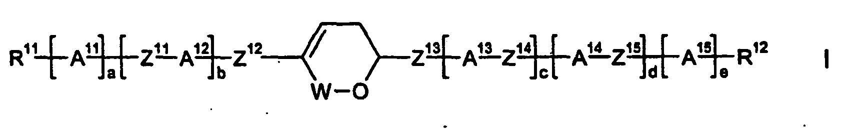 in Gegenwart eines Metathese-Katalysators umfaßt,
wobei a, b, c, d, e, W, R¹¹, R¹², Z¹¹, Z¹², Z¹³, Z¹⁴, Z¹⁵, A¹¹, A¹², A¹³, A¹⁴ und A¹⁵ in den FormeIn I und II die gleiche Bedeutung wie für Formel I oben haben; mit der Maßgabe, dass für den Fall der direkten Verknüpfung von Z¹³ und R¹² zu -Z¹³-R¹² R¹² H, Aralkyl oder Alkanyl bedeutet, wenn Z¹³ -C(=O)- bedeutet, R¹² Aralkyl oder Alkanyl bedeutet, wenn Z¹³ -C(=O)-O-bedeutet, und Z¹³ nicht -CH₂O- oder -CF₂O- bedeutet; dass für den Fall der direkten Verknüpfung von Z¹⁴ und R¹² zu -Z¹⁴-R¹² R¹² H, Aralkyl oder Alkanyl bedeutet, wenn Z¹⁴ -C(=O)- bedeutet, R¹² Aralkyl oder Alkanyl bedeutet, wenn Z¹⁴ -C(=O)-O- bedeutet, und Z¹⁴ nicht -CH₂O- oder -CF₂O-bedeutet; dass für den Fall der direkten Verknüpfung von Z¹⁵ und R¹² zu -Z¹⁵-R¹² R¹² H, Aralkyl oder Alkanyl bedeutet, wenn Z¹⁵ -C(=O)- bedeutet, R¹² Aralkyl oder Alkanyl bedeutet, wenn Z¹⁵ -C(=O)-O- bedeutet, und Z¹⁵ nicht -CH₂O- oder -CF₂O- bedeutet; dass R¹¹ nicht H bedeutet, wenn a und b beide zugleich null sind und Z¹² eine Einfachbindung bedeutet; dass R¹¹ nicht CH₃ bedeutet, wenn zugleich a und b beide null sind, Z¹² und Z¹³ jeweils eine Einfachbindung bedeuten, W -CH₂- bedeutet und -[-A¹³-Z¹⁴-]_{c}-[-A¹⁴-Z¹⁵-]_{d}-[-A¹⁵-]ₑ-R¹² für unsubstituiertes Phenyl steht.

Metathese-Reaktionen (auch häufig als Olefin-Metathese bezeichnet) zur Synthese von Carbocyclen (bzw. von (Poly-)Olefinen aus Carbocyclen) sind im Stand der Technik gut bekannt (siehe u.a. R. H. Grubbs und S. Chang, *Tetrahedron* 54 (1998) 4413; T. M. Trnka und R. H. Grubbs, Acc. Chem. Res. **2001,** 34, 18; S. K. Armstrong, *J. Chem. Soc., Perkin Trans. I,* 1998, 371; A. Fürstner et al., *Chem. Eur. J.* **2001,** 7, 3236, und dort zitierte Literaturstellen). Mit ihrer Hilfe können durch gleichzeitige Spaltung und Knüpfung von ungesättigten Kohlenstoff-Kohlenstoff-Bindungen insbesondere in Gegenwart ausgewählter Metall-Carben-Komplexe neue C-C-Bindungen und somit komplexere Moleküle aufgebaut werden.

Dabei kann man verschiedene ringschließende und ringöffnende Metathese-Reaktionstypen unterscheiden. Neben der hier nicht weiter interessierenden ringöffnenden polymerisierenden Metathese (ROMP; Ring-opening metathesis polymerization) sind insbesondere die (intramolekulare) ringschließende Metathese (RCM; Ring closing metathesis), die Kreuzmetathese (CM; Cross metathesis) und die Enin-Metathese (Enin) von besonderer Bedeutung. Die genannten Metathese-Typen sind in Schema 1 am Beispiel eines Kohlenwasserstoffs veranschaulicht:

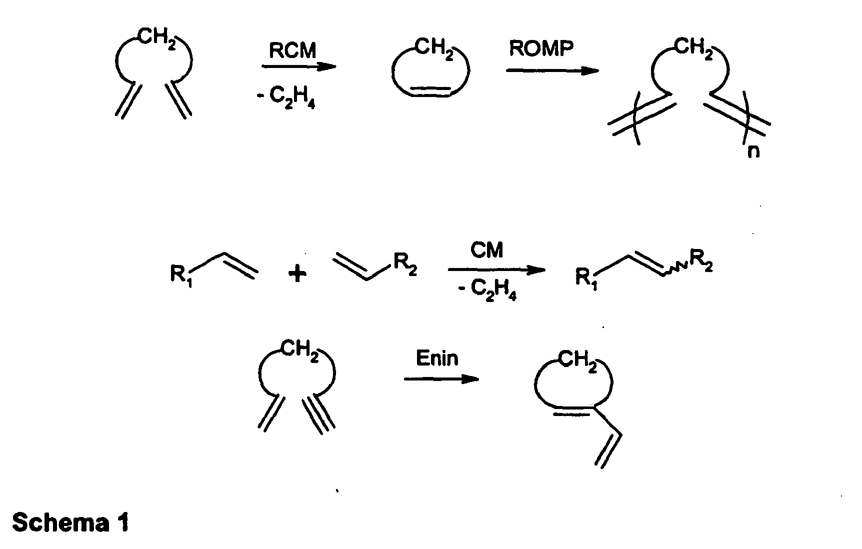

Die Metathese-Reaktionen werden bevorzugt durch so genannte Schrock-(oder auch Grubbs-)Carben-Komplexe (Metall-Alkyliden-Komplexe) von Übergangsmetallen wie Wolfram, Molybdän und insbesondere Ruthenium katalysiert. Diese Komplexe weisen zumeist eine Struktur auf, die durch die folgende Formel COMP-A wiedergegeben werden kann (vgl. u.a. WO 96/04289, WO 97/06185, WO 99/00396 und WO 99/00397):

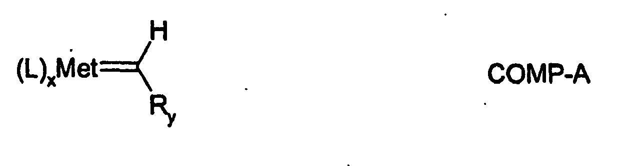

Dabei steht Met für ein Übergangsmetall, (L)ₓ für mehrere gleiche oder verschiedene Liganden und R_{y} für einen organischen Rest, zumeist Aryl, Alkanyl oder Alkenyl.

Die genannten ringschließenden Metathese-Reaktionen sind auch eingesetzt worden, um heterocyclische Ringsysteme zu bilden und entsprechende Verbindungen darzustellen. So sind Stickstoff-Heterocyclen in großer Vielzahl durch Olefin-Metathese zugänglich. Auch Sauerstoff-Heterocyclen sind laut Literatur mit Hilfe dieser synthetischen Methodologie herstellbar, jedoch offenbar in deutlich geringerer struktureller Breite.

So sind von gut zugänglichen Ausgangsverbindungen ausgehende Metathese-Verfahren zur Synthese von Pyranderivaten, die sowohl einen exocyclischen Substituenten an einem Kohlenstoffatom der durch Metathese gebildeten endocyclischen C=C-Doppelbindung und eine 2,5-Disubstitution aufweisen 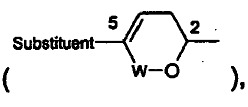 als auch über technisch nützliche Eigenschaften verfügen und zum Beispiel als mesogene Materialien oder als Vorstufen zur Herstellung von anderen einen Pyranring enthaltenden Verbindungen mit mesogenen Eigenschaften Verwendung finden können, bislang im Stand der Technik nicht beschrieben worden. Möglicherweise ist das darauf zurückzuführen, dass die Ringschlussreaktion zu O-Heterocyclen unter Metathesebedingungen bis zur vorliegenden Erfindung gewöhnlich nicht oder nicht reproduzierbar gelungen ist, wenn eines der Kohlenstoffatome einer der reagierenden C=C-Doppelbindungen der Ausgangsverbindung(en) disubstituiert ist (vgl. auch S. K. Armstrong, *J. Chem. Soc., Perkin Trans. I,* 1998, 371, insbesondere S. 376).

Es ist daher besonders überraschend, dass die ringschließende Metathese ausgehend von Verbindungen der Formel II in Gegenwart eines Metathese-Katalysators zuverlässig und effizient zu den erfindungsgemäßen Pyranderivaten der Formel 1 führt.

Als Metathese-Katalysator für die ringschließende Metathese finden bevorzugt die im Stand der Technik beschriebenen Übergangsmetall-Alkyliden-Komplexe der allgemeinen Formel COMP-A Verwendung (siehe u.a. R. H. Grubbs und S. Chang, *Tetrahedron* 54 (1998) 4413; T. M. Tmka und R. H. Grubbs, Acc. Chem. Res. **2001**, *34*, 18; S. K. Armstrong, J. *Chem. Soc., Perkin Trans. I,* 1998, 371; A. Fürstner et al., *Chem. Eur. J.* **2001**, 7, 3236, und dort zitierte Literaturstellen). Bevorzugt handelt es sich dabei um einen Komplex der Formel COMP-A mit Met = Wolfram, Molybdän oder Ruthenium. Ferner finden Oxo-Wolfram-Komplexe des Typs trans-W(=O)Cl₂(Aryl)₂ (mit Aryl vorzugsweise gleich 2,6-Dibromphenyl), die unter anderem von W. A Nugent et al., J. Am. Chem. Soc., 1995, 117, 8992, beschrieben worden sind, Verwendung als Metathese-Katalysatoren in den erfindungsgemäßen Verfahren.

Bevorzugte Molybdän-Metathese-Katalysatoren sind solche der Formeln COMP-Mo1, COMP-Mo2, COMP-Mo3 und COMP-Mo4, während Formel COMP-W1 einen bevorzugten Wolfram-Metathese-Katalysator zeigt:

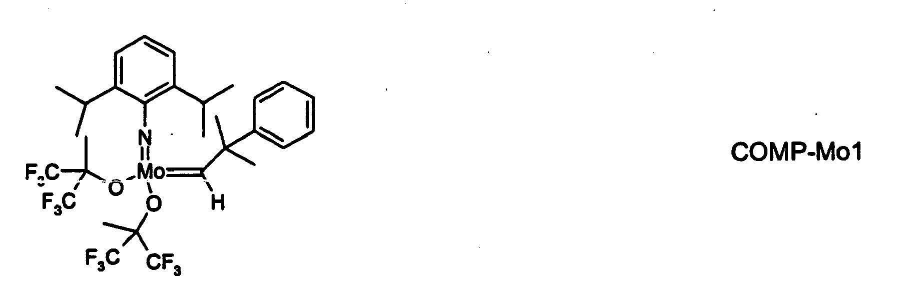

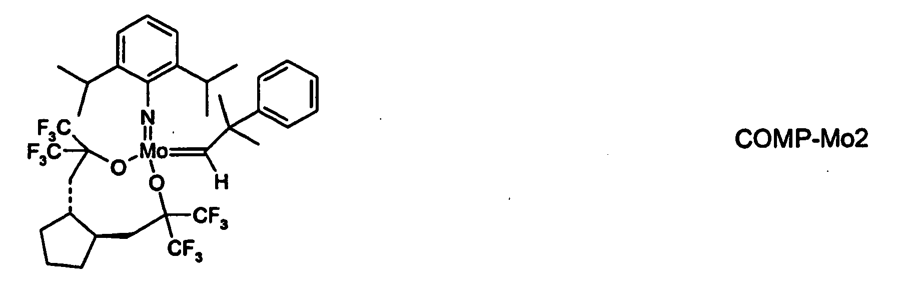

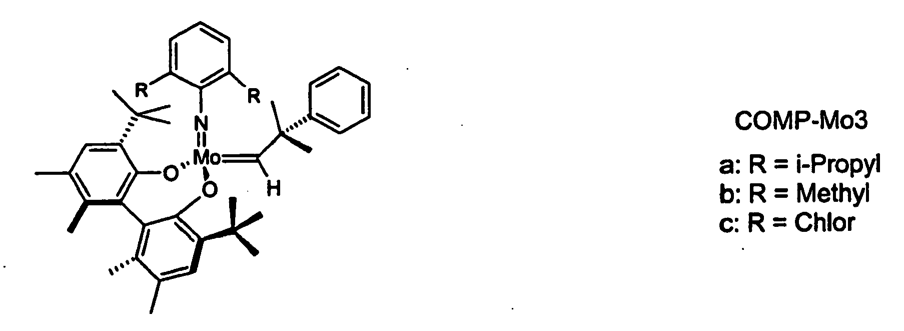

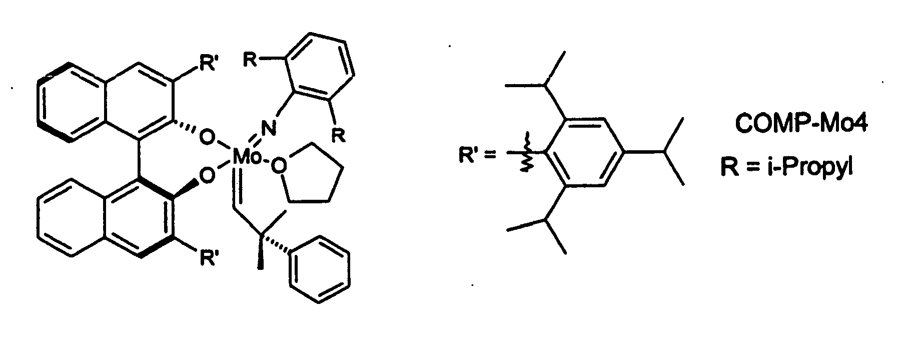

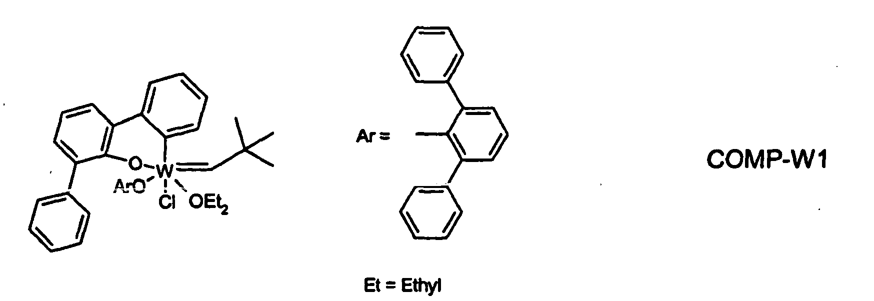

Besonders bevorzugte Komplexe zur Verwendung als Metathese-Katalysator in dem erfindungsgemäßen Verfahren sind Ruthenium-Alkyliden-Komplexe des Typs COMP-RuA, die an sich literaturbekannt sind (siehe u.a. WO 96/04289, WO 97/06185, WO 99/00396, WO 99/00397, WO 99/29701, WO 99/51344, WO 00/15339, EP 1 022 282 A2, WO 00/58322, WO 00/71554, WO 02/14336, WO 02/14376, WO 02/083742):

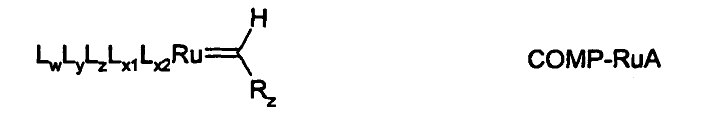

Vorzugsweise sind dabei Lₓ₁ und/oder Lₓ₂ anionische Liganden, bevorzugt Brom, Iod oder insbesondere Chlor, gegebenenfalls auch O-Aralkyl, während L_{y} und L_{z} vorzugsweise andere, zumeist neutrale Liganden wie zum Beispiel PPh₃ (Ph = Phenyl), P(i-Pr)₃ (i-Pr = iso-Propyl), PCy₃ (Cy = Cyclohexyl), P(Cp)₃ (Cp = Cyclopentadienyl), unsubstituiertes oder substituiertes Pyridyl, Im¹, Im² oder über das Sauerstoffatom koordinierendes Alkoxy darstellen. L_{w} steht für einen optional vorhandenen Liganden, d.h. w ist 0 oder 1, wobei dieser zumeist die gleiche Bedeutung hat wie Lₓ₁, Lₓ₂, L_{y} und/oder L_{z}. R_{z} steht für einen organischen Rest, insbesondere Aryl, Alkanyl oder Alkenyl.

| | |
|---|---|
| 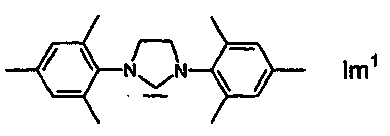 | 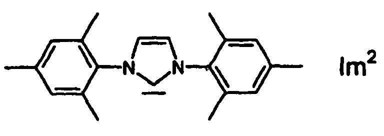 |

Diese Ruthenium-Komplexe sind im allgemeinen wenig empfindlich für Luftsauerstoff und tolerieren sowohl Feuchtigkeitsspuren als auch geringe Verunreinigungen. Unter den Ruthenium-Alkyliden-Komplexen COMP-RuA sind beispielhaft solche der folgenden Formeln COMP-Ru1 bis COMP-Ru13 zu nennen:

| | |
|---|---|
| 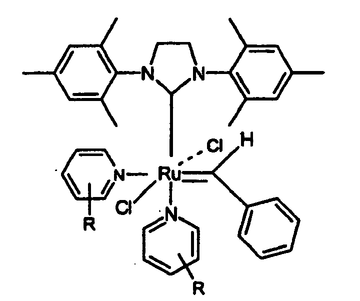 | COMP-Ru1 |
| | a:R=H |
| | b: R = 3-Br |
| | c: R = 4-Phenyl |
| 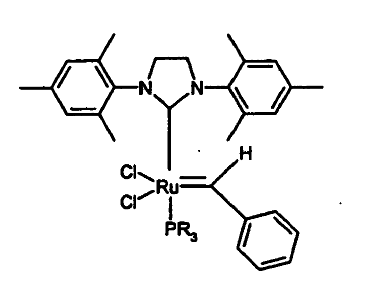 | COMP-Ru2 |
| | a: R=Cy |
| | b: R = Phenyl |
| | c: R = |
| | **p-CF**_{**3**}**-Phenyl** |
| 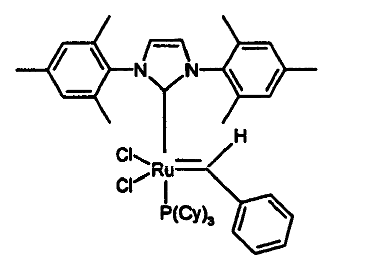 | COMP-Ru3 |
| 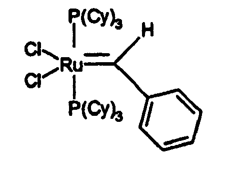 | COMP-Ru4 |
| 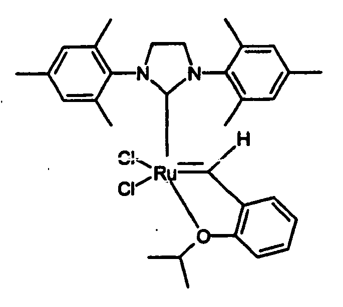 | COMP-Ru5 |
| | a: R=H |
| | b: R = Br |
| | **c: R = NO**_{**2**} |
| 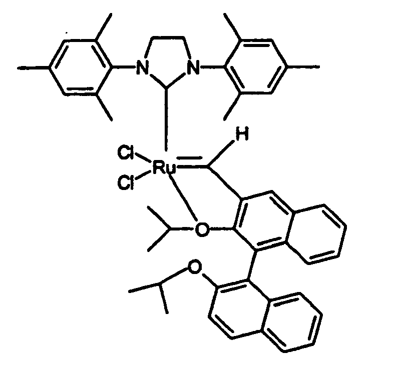 | COMP-Ru6 |
| 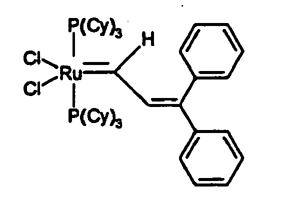 | COMP-Ru7 |
| 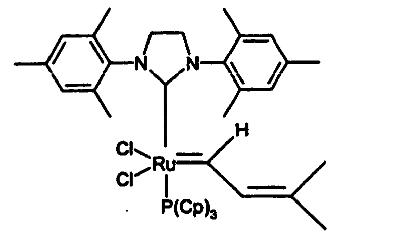 | COMP-Ru8 |
| 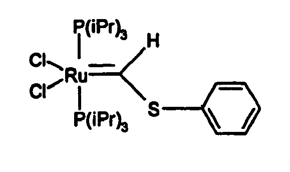 | COMP-Ru9 |
| 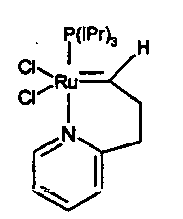 | COMP-Ru10 |
| 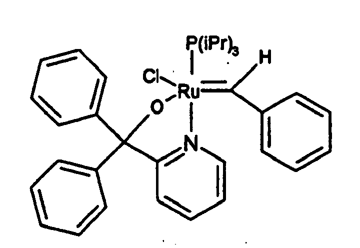 | COMP-Ru11 |
| 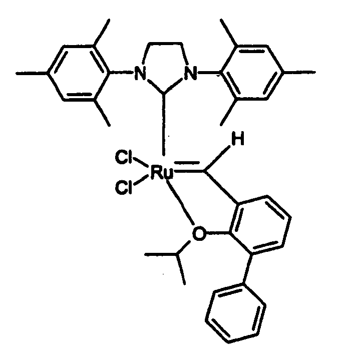 | COMP-Ru12 |
| 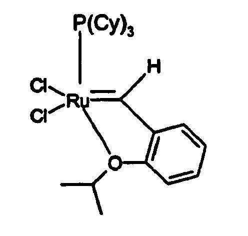 | COMP-Ru13 |

Die als Metathese-Katalysatoren eingesetzten Alkyliden-Komplexe werden entweder nach literaturbekannten Verfahren hergestellt (siehe neben WO 96/04289, WO 97/06185, WO 99/00396, WO 99/00397, WO 99/29701, WO 99/51344, WO 00/15339, EP 1 022 282 A2, WO-00/58322, WO 00/71554, WO 02/14336, WO 02/14376, WO 02/083742 u.a. auch u.a. R. H. Grubbs und S. Chang, *Tetrahedron* 54 (1998) 4413; T. M. Tmka und R. H. Grubbs, Acc. Chem. Res. 2001, 34, 18; S. K. Armstrong, *J*. *Chem. Soc., Perkin Trans. I*, 1998, 371; A. Fürstner et al., *Chem. Eur. J*. **2001**, 7, 3236; J. A. Love et al., Angew. Chem. 2002, 114, 4207; K. Grela et al., Angew. Chem. 2002, 114, 4210; G. S. Weatherhead et al., Tetrahedron Lett. 41 (2000) 9553; J. S. Kingsbury et al., J. Am. Chem. Soc. 1999, 121, 791, und dort angegebene Literaturstellen) oder sind kommerziell erhältlich, zum Beispiel von Sigma-Aldrich, Inc. (USA), oder Strem Chemicals Inc. (Kehl, Deutschland).

Einige dieser Katalysatoren können auch an immobilisierenden Trägem, zum Beispiel aus Polystyrol (z.B. COMP-Ru3: M. Ahmed et al., Synlett 2000, 1007; oder COMP-Ru5a und davon abgeleitete Komplexe: St. Randl et al., Synlett 2001, 1547) oder Glas (z.B. COMP-Ru5 und davon abgeleitete Komplexe: J. S. Kingsbury et al., Angew. Chem. 2001, *113*, 4381), angebracht sein.

Ganz besonders bevorzugt wird als Katalysator für den Schritt der ringschließenden Metathese des erfindungsgemäßen Verfahrens außer den oben genannten Molybdän- und Wolfram-Komplexen ein MetallKomplex, ausgewählt aus der Gruppe bestehend aus Komplexen der Formeln COMP-Ru1, -Ru2, -Ru3, -Ru4, -Ru5, -Ru6, -Ru7, -Ru8, -Ru9, -Ru10, -Ru11, -Ru12 und -Ru13, insbesondere der Formeln COMP-Ru2a, -Ru3, -Ru5a, -Ru6, -Ru11 und -Ru13, verwendet. Das Mol-Verhältnis von Verbindung der Formel II zu Katalysator beträgt im allgemeinen von 0,001 bis 20 mol-% (bezogen auf die Verbindung der Formel II), bevorzugt 0,01 bis 10 mol-%, ganz besonders bevorzugt 0,01 bis 2 mol-% und insbesondere 0,01 mol-% bis 0,1 mol-%.

Die ringschließende Metathese des erfindungsgemäßen Verfahrens wird unter für solche Metallkomplex-katalysierten Reaktionen üblichen Bedingungen ausgeführt. Die Reaktion erfolgt lösungsmittelfrei oder in einem geeigneten Solvens. Als Lösungsmittel wird ein inertes Lösungsmittel, bevorzugt ein aromatisches und/oder halogeniertes organisches Lösungsmittel und insbesondere Toluol, Benzol, Chlorbenzol oder Dichlormethan verwendet. Die Reaktion erfolgt in der Regel bei Temperaturen von Raumtemperatur bis Siedetemperatur des Lösungsmittels (z.B. ca. 20 °C bis 40 °C (Dichlormethan) beziehungsweise bis 80 °C oder 110 °C (Toluol)). Bevorzugt beträgt die Reaktionstemperatur 40 °C bis 60 °C. Die Reaktionszeit ist an sich nicht kritisch, da die Metathese-Reaktion im allgemeinen unter vollständiger Umsetzung des Edukts der Formel II abläuft und das gewünschte Pyranderivat der Formel I in guten Ausbeuten ergibt. Üblicherweise beträgt die Reaktionsdauer von 10 min bis 2 Tagen, bevorzugt von 1 h bis 24 h, insbesondere von 2 h bis 8 h.

Es ist ferner vorteilhaft, den Katalysator dem Reaktionsgemisch portionsweise zuzusetzen, was eine bessere Steuerung der Umsetzung erlaubt und zu einer Einsparung an Katalysator-Gesamtmenge führt.

In einer bevorzugten Ausführungsform des erfindungsgemäßen Verfahrens werden Verbindungen der Formel I-A (d.h. Verbindungen der Formel I mit W = -C(=O)-), in denen Z¹³, Z¹⁴ und Z¹⁵ nicht -C(O)- bedeuten, nach dem erfindungsgemäßen Metathese-Reaktionsschritt in einem weiteren Reaktionsschritt einer Reduktionsreaktion zur Überführung in ein Pyranderivat der Formel I, worin W eine Methylen-Gruppe ist (d.h. ein Pyranderivat der Formel 1-B) unterworfen. Diese Reduktionsreaktion kann mit geeigneten Reduktionsmitteln, zum Beispiel Düsobutylaluminiumhydrid (DIBAH) oder Bortrifluorid-Etherat + Lithiumaluminumhydrid oder Lithiumborhydrid oder Natriumborhydrid (siehe J. March: Advanced Organic Chemistry; John Wiley & Sons, New York u.a., 3. Aufl., 1985, S. 1100, 9-41), ausgeführt werden. Dabei werden auch andere im Molekül vorhandene Carboxy-Funktionen (C(=O)O) zu Ether-Funktionen (CH₂O) reduziert.

Eine weitere bevorzugte Ausführungsform des erfindungsgemäßen Verfahrens umfaßt als einen weiteren, vor dem ringschließenden Metathese-Schritt erfolgenden Reaktionsschritt die Umsetzung einer Acrylverbindung beziehungsweise Allylverbindung der Formel IV mit einer Homoallylverbindung der Formel V unter Bildung der Verbindung der Formel II 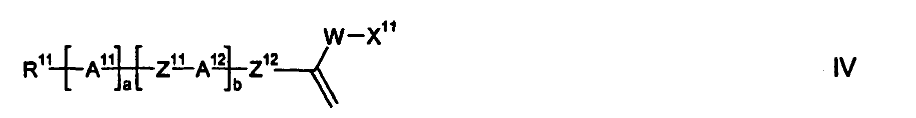 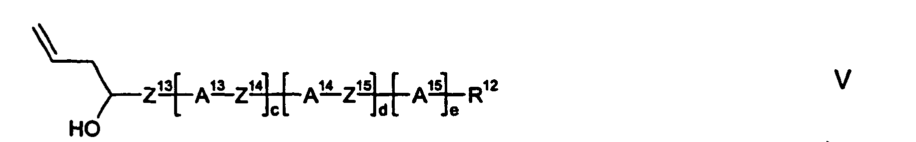 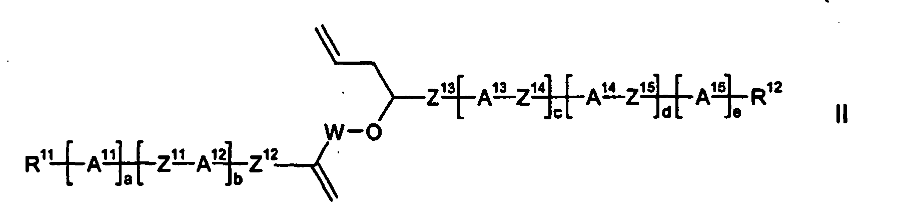 wobei X¹¹ eine Abgangsgruppe bedeutet und a, b, c, d, e, W, R¹¹, R¹², Z¹¹, Z¹², Z¹³, Z¹⁴, Z¹⁵, A¹¹, A¹², A¹³, A¹⁴ und A¹⁵ in den Formeln II, IV und V die gleiche Bedeutung wie für Formel I oben haben.

Wenn W für -C(=O)- steht (d.h. die Verbindung der Formel IV ist ein Acrylderivat (Formel IV-A)), ist X¹¹ bevorzugt Methoxy, Ethoxy, Propoxy, t-Butoxy, Chlor, Brom oder ein Anhydrid-Rest, insbesondere Acetoxy. Der Verfahrensschritt verläuft dann unter für derartige Esterbildungsreaktionen üblichen Bedingungen (vgl: u.a. J. March: Advanced Organic Chemistry; John Wiley & Sons, New York u.a., 3. Aufl., 1985, S. 346-351, 0-22, 0-23 und 0-24).

Vorzugsweise steht W für -CH₂- (d.h. die Verbindung der Formel IV ist ein Allylderivat (Formel IV-B)). In diesem Fall ist X¹¹ bevorzugt Chlor, Brom, iod oder ein Sulfonsäurerest, z.B. CF₃-SO₃-, CH₃-SO₃-, C₄F₉-SO₃ oder p-CH₃₋C₆H₄-SO₃-. Der erfindungsgemäße Etherbildungs-Reaktionsschritt erfolgt im allgemeinen unter Bedingungen der Williamson-Ether-Synthese (s. z.B. J. March: Advanced Organic Chemistry; John Wiley & Sons, New York u.a., 3. Aufl., 1985, S. 342, 0-14), d.h. unter basischen Reaktionsbedingungen und bei Temperaturen von Raumtemperatur bis ca. 60 °C in geeigneten Lösungsmitteln, z.B. Ethern wie Tetrahydrofuran (THF), Methyl-tert.-butylether (MTBE), Dioxan oder Dimethoxyethan. Eine besonders bevorzugte Variante dieser Reaktion arbeitet mit granuliertem NaOH in THF in Gegenwart eines Phasentransferkatalysators und von wenig Wasser, wobei die Allylverbindung der Formel IV-B mit X¹¹ = Brom oder Chlor und der Homoallylalkohol der Formel V für 4 bis 48 h auf 40 °C bis 60 °C erwärmt werden. Diese Variante eignet sich besonders für diejenigen Homoallylalkohole der Formel V, die keine Carboxylfunktion aufweisen (also Homoallylalkohole der Formel V, in denen Z¹³, Z¹⁴ und Z¹⁵ nicht CO₂ bedeuten). Alternativ kann als Base Natriumhydrid, bevorzugt in einem organischen Lösunsmittel, eingesetzt werden.

Die in dem erfindungsgemäßen Verfahren eingesetzten Acrylverbindungen beziehungsweise Allylverbindungen der Formel IV und Homoallylverbindungen der Formel V sind - soweit sie literaturbekannt sind - entweder käuflich erhältlich oder werden nach an sich bekannten Methoden dargestellt, wie sie in der Literatur (zum Beispiel den Standardwerken wie Houben-Weyl, Methoden der Organischen Chemie, Georg-Thieme-Verlag, Stuttgart) beschrieben sind, und zwar unter Reaktionsbedingungen, die für die dort genannten Umsetzungen bekannt und geeignet sind. Dabei kann man auch von an sich bekannten, hier nicht näher erwähnten Varianten Gebrauch machen.

Die Allylverbindungen der Formel IV-Ba mit a = b = 0 sind unter anderem über die in Anlehnung an ein Verfahren von Weigand und Brückner (S. Weigand, R. Brückner, Synthesis 1996,475) gemäß Schema 2 zugänglichen Allylalkohole der Formel IV-Ba1 erhältlich. Die Umwandlung von IV-Ba1 in die Allylverbindungen der Formel IV-Ba2 erfolgt nach bekannten Verfahren, z.B. für X¹¹ = Br mit Brom/Triphenylphosphan und für X¹¹ = CF₃SO₃ mittels Trifluormethansulfonsäurechlorid oder Trifluormethansulfonsäureanhydrid.

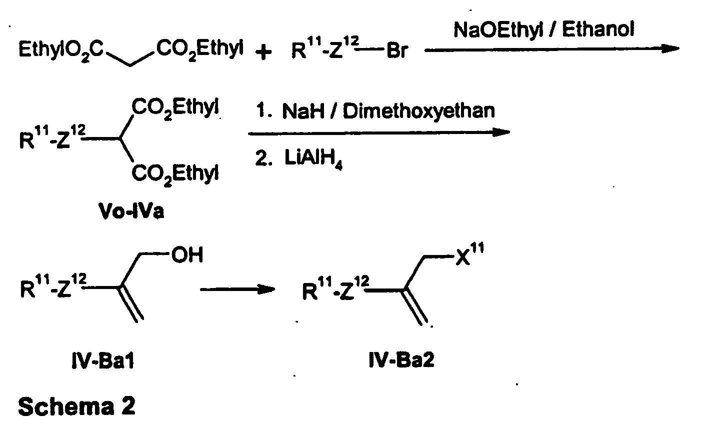

Die Synthese der bislang unbekannten Allylderivate der Formel IV-Bb mit a und/oder b gleich 1 erfolgt analog und ist in Schema 3 dargestellt:

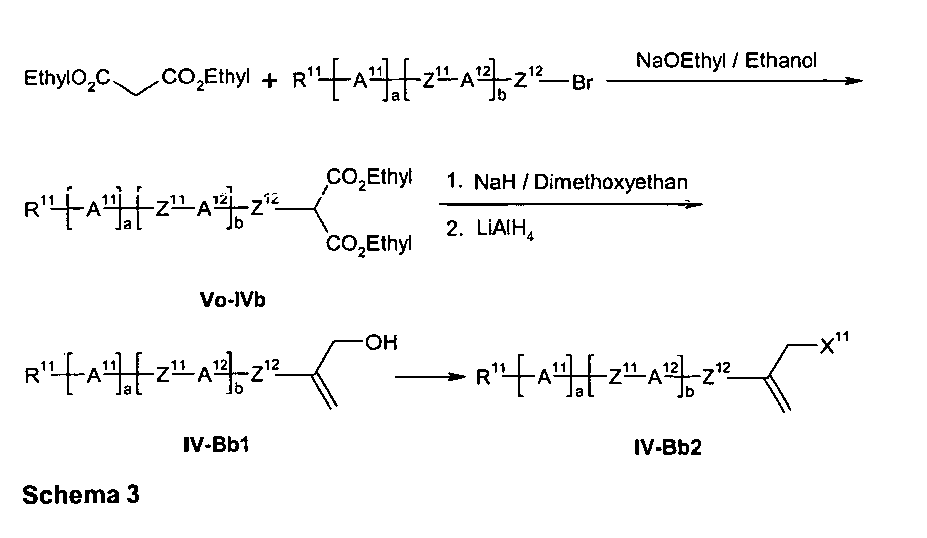

Anstelle der substituierten Malonsäurediester Vo-IVa beziehungsweise Vo-IVb können zur Herstellung der Allylderivate der Formel IV-B auch die entsprechenden literaturbekannten oder in Analogie zu bekannten Verfahren zugänglichen Diole der Formel Vo-IVc (siehe unter anderem P. Kirsch und E. Poetsch, Adv. Mater. **1998**, *10*, 602, und dort angegebene Literaturstellen; C. Tschierske et al., Mol. Cryst. Liq. Cryst. 1990, 191, 295; EP 400 861 A1) verwendet werden. Die Vorstufe Vo-IVc ergibt zum Beispiel nach Dihalogenierung und Behandlung mit 1 Äquivalent Kalium-tert.-butylat die Allylverbindung IV-B mit X¹¹ = Halogen.

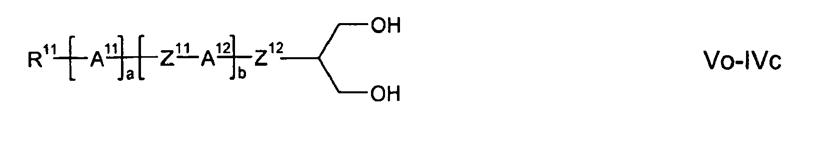

Die Allylalkohole der FormeIn IV-Ba1 und IV-Ba2 sind auch in Analogie zu einem Verfahren von H. Mitzutani et al. (Tetrahedron 58 (2002) 8929) aus den entsprechenden Acroleinderivaten mit NaBH₄/CeCl₃ zugänglich; die Acroleinderivate ihrerseits werden aus dem entsprechenden Aldehyd R¹¹-Z¹²-CH₂-CHO beziehungsweise R¹¹-[-A¹¹-]ₐ-[-Z¹¹-A¹²-]_{b}-Z¹²-CH₂-CHO mit Eschenmosers Reagenz erhalten.
Die neuen Allylderivate der Formel IV-Bb 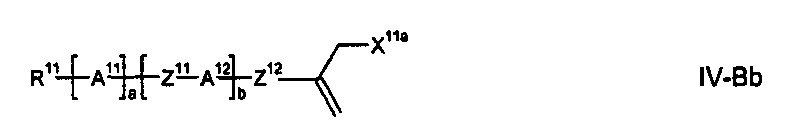 wobei a null und b 1 oder a 1 und b null oder a und b beide 1 sind;
- X^{11a}: Chlor, Brom, Iod, einen Sulfonsäurerest, -OH, Alkoxy oder O-Aralkyl bedeutet oder -X^{11a} für =O steht;
- R¹¹, Z¹¹ und Z¹²: wie für Formel I oben definiert sind; und
- A¹¹ und A¹²: wie für Formel I oben definiert sind und bevorzugt 1,4-Cyclohexylen bedeuten;
sind als nützliche Zwischenverbindungen zur Herstellung der erfindungsgemäßen Verbindungen der Formel I ebenfalls Gegenstand der vorliegenden Erfindung. Ihre Herstellung erfolgt wie oben und in den angefügten Beispielen dargelegt; die Verbindungen der Formel IV-Bb mit X^{11a} = Alkoxy oder O-Aralkyl sind unter anderem durch Umsetzung der entsprechenden Verbindungen der Formel IV-Bb mit X^{11a} = Cl, Br, I oder Sulfonsäurerest mit einem entsprechenden Alkohol (Alkyl-O-H beziehungsweise Aralkyl-O-H) zugänglich. Die erfindungsgemäßen aldehyde der Formel IV-Bb (mit -X^{11a} gleich =O) sind auch durch milde Oxidation mit beispielsweise Braunstein aus den Allylalkoholen der Formel IV-Bb (mit X^{11a} gleich -OH) zugänglich.

Die korrespondierenden Acrylderivate der Formel IV-A sind unter anderem durch Oxidation der entsprechenden Allylalkohole der Formel IV-Ba1 beziehungsweise IV-Bb1 mit einem starken Oxidationsmittel wie einem Chrom(VI)-Oxidationsmittel zugänglich (J. March: Advanced Organic Chemistry; John Wiley & Sons, New York u.a., 3. Aufl., 1985, S. 1084, 9-22). Alternativ können die Allylalkohole auch zunächst mit einem milderen Oxidationsmittel wie Braunstein (MnO₂) in die entsprechenden Aldehyde (Acroleinderivate) überführt werden, die dann beispielsweise mit einer alkalischen Ag₂O- oder Silbernitrat-Lösung oder mittels Luft-Sauerstoff in die Acrylderivate der Formel IV-A überführt werden.

Die Homoallylverbindungen der Formel V sind gemäß Schema 4 durch Umsetzung von Aldehyden der Formel Vo-Va mit Allylbromid zugänglich (wobei anstelle des Bromids auch andere Allylhalogenide, insbesondere das Allylchlorid, verwendet werden können):

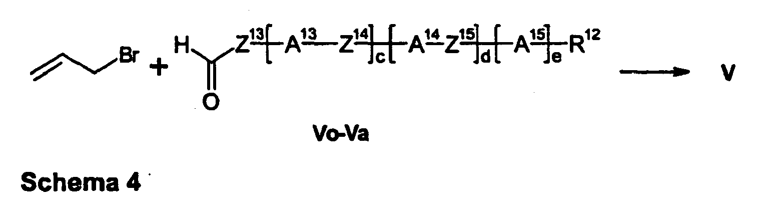

Dabei kann die erforderliche Aktivierung des Allylhalogenids auf unterschiedliche Weise erfolgen: Beispielsweise wird durch Zugabe von Allylbromid zu Indium-Pulver in einem geeigneten Reaictionsmedium, zum Beispiel Wasser oder Wasser/Tetrahydrofuran, zunächst eine entsprechende intermediäre Indium-Allylverbindung (nach T.-P. Loh et al., Tetrahedron Letters 42 (2001) 8701 und 8705) gebildet, die dann mit dem Aldehyd Vo-Va unter Bildung des korrespondierenden Homoallylalkohols V reagiert. Allylbromid kann auch mit Lithium oder einer Lithium-organischen Base oder mit Magnesium, zumeist im Überschuß, oder einer reaktiven Grignard-Base unter Halogen-Metall-Austausch in die entsprechende intermediäre Lithium- beziehungsweise Magnesiumbromid-Allylverbindung überführt werden, die dann mit dem Aldehyd Vo-Va zu dem Homoallylalkohol V reagiert. Enthält der Aldehyd Vo-Va zusätzlich eine Ester- oder Nitrilfunktion, so ist es vorteilhaft, die intermediäre Lithiumbeziehungsweise Magnesiumbromid-Allylverbindung in bekannter Weise mit Zink- oder Titansalzen umzumetallieren, da die entsprechenden Zinkbeziehungsweise Titan-Allylverbindungen chemoselektiv nur mit der aldehydischen Carbonyl-Funktion und nicht mit der Ester-Carboxybeziehungsweise Nitril-Funktion reagieren.

Ferner können auch Allylderivate anderer Metalle und Halbmetalle, zum Beispiel Allylderivate von Chrom, Zinn, Zink, Samarium, Bor und Silicium, zur Herstellung der Homoallylverbindungen V eingesetzt werden. Ausgehend von Allylmesylat (Brom im Allylbromid ist durch einen Mesylatrest (-OSO₂CH₃) ersetzt), sind mittels Transmetallierung mit z.B. LiSn(butyl)₃ die entsprechenden Allyl-Stannane zugänglich, die auch durch Umsetzung von Allylbromid mit Zinn(II)chlorid und Kaliumiodid in Wasser (vgl. V.V. Samoshin et al., Tetrahedron Lett. 43 (2002) 6329) oder mit Zinnmetall unter Einwirken von Ultraschall und Wasser (vgl. P. C. Andrews, Tetrahedron Lett. 43 (2002) 7541) hergestellt und mit dem Aldehyd Vo-V zum Homoallylalkohol V umgesetzt werden können.

Entsprechend ist unter anderem mit Zinkstaub in Tetrahydrofuran die Zinkallylverbindung (vgl. B. C. Ranu et al., Tetrahedron Lett. 36 (1995), 4885), mit Sml₂ in Tetrahydrofuran die Samariumallylverbindung (vgl. B. Hamann-Gaudinet et al., Tetrahedron Lett. 38 (1997) 6585) beziehungsweise mit Cr(II)Cl₂/Mn die Chromallylverbindung erhältlich, die mit einem Aldehyd Vo-V zu Homoallylalkoholen V umgesetzt werden können.

Da die Aldehyde der Formel Vo-V am Carbonyl-Kohlenstoffatom ein prochirales Zentrum aufweisen, wird bei der Umsetzung mit dem aus Allylbromid gebildeten aktivierten Allylderivat zu dem Homoallylalkohol V ein Chiralitätszentrum am die Hydroxyfunktion tragenden Kohlenstoffatom gebildet. Im allgemeinen entsteht dabei ein Racemat der optischen Antipoden des Homoallylalkohols V. Es ist jedoch auch möglich, eines der optischen Isomeren des Homoallylalkohols V stereoselektiv herzustellen oder alternativ aus der racemischen Mischung zu isolieren.

Die stereoselektive Synthese erfolgt bevorzugt durch katalytische asymmetrische Allylierung des Aldehyds der Formel Vo-V mit einer Allylzinnverbindung, zumeist dem Allyltributylstannan, in Gegenwart eines chiralen Katalysators. Als chirale Katalysatoren eignen sich insbesondere Komplexe chiraler Binaphtol- (BINOL-)Verbindungen mit Zirkonium {z.B. (*R,R*)- oder (*S,S*)-BINOL-Zr(O-tert.-Butyl)₄: M. Kurosu et al., Tetrahedron Lett. 43 (2002) 1765) beziehungsweise Titan (z.B. Bis(((S)(napthoxy)-(isopropxy)titanoxid: H. Hanawa et al., J. Am. Chem. Soc. **2003**, 125, 1708) oder entsprechende Boronate (vgl. z.B. S. Thormeier et al., J. Organomet. Chem. 657 (2002) 136). Grundsätzlich ist so - je nach Wahl des chiralen Katalysators - das R- oder das S-Isomere selektiv zugänglich.

Die Isolierung der Enantiomeren aus der racemischen Mischung erfolgt nach üblichen Verfahren, zum Beispiel mittels Kristallisation mit einer chiralen Base oder Chromatographie an einem chiralen Säulenmaterial.

Durch Verwendung eines so zugänglichen enantiomerenreinen Homoallylalkohols der Formel V wird über die Zwischenstufe II nach der erfindungsgemäßen ringschließenden Metathese-Reaktion das entsprechende Pyranderivat der Formel I, das in 2-Position ein asymmetrisches Zentrum aufweist, in enantiomerenreiner Form erhalten. Eine weitere Möglichkeit, einen der optischen Antipoden des Pyranderivats der Formel I stereoselektiv zu erhalten, besteht in der Verwendung eines chiralen Metathese-Katalysators, z.B. COMP-Mo3 oder COMP-Mo4 (G. S. Weatherhead et al., Tetrahedron Lett. 41 (2000) 9553).

Aldehyde der Formel Vo-Va sind als solche literaturbekannt (siehe u.a. EP 122 389 A2) beziehungsweise in Analogie dazu herstellbar. Die Synthese von Aldehyden der Formel Vo-Va mit Z¹³ = CF₂O erfolgt dabei zum Beispiel ausgehend von dem Säurechlorid

EthylO-C(=O)-C(=O)-Cl Vo-Vb

durch Umsetzung mit zunächst NaS-(CH₂)₃-SH. Der dabei entstehende Thiolthioester wird mit Trifluormethansulfonsäure (in Analogie zu den Verfahren gemäß P. Kirsch et al., Angew. Chem. **2001**, *113*, 1528, und WO 01/64667) zu dem entsprechenden Bis(alkylthio)carbenium-Salz 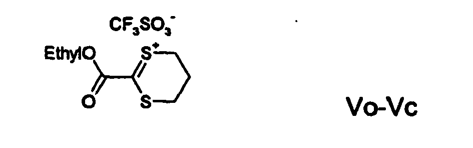 umgesetzt, das dann einer oxidativen Fluordesulfurierung (gemäß P. Kirsch et al., Angew. Chem. **2001**, *113,* 1528, und WO 01/64667) unterworfen wird, indem das Bis(alkylthio)carbenium-Salz der Formel Vo-Vc zunächst bei tiefen Temperaturen mit NEt₃·3 HF (Et = Ethyl) und einem Alkohol der Formel

HO-[A¹³-Z¹⁴]_{c}-[A¹⁴-Z¹⁵]_{d}-[A¹⁵]ₑ-R¹² Vo-Vd,

dann mit 1,3-Dibrom-5,5-dimethylhydanthoin (DBH) oder N-Bromsuccinimid (NBS) oder Brom und schließlich mit wäßriger Lauge zum Ester

Ethyl-O-C(=O)-CF₂O-[A¹³-Z¹⁴]_{c}-[A¹⁴-Z]_{d}-[A¹⁵]ₑ-R¹² Vo-Ve

umgesetzt wird. Die abschließende Einführung der Aldehyd-Funktion unter Ergeben des Aldehyds der Formel Vo-Va erfolgt entweder durch direkte Reduktion des Esters mit einem geeigneten Reduktionsmittel wie Diisobutylaluminiumhydrid in einem inerten Solvens, zum Beispiel Methylenchlorid, oder über die Reduktion des Esters zum entsprechenden Alkohol und anschließende Oxidation zum Aldehyd mit einem geeigneten Oxidationsmittel, zum Beispiel mit Dess-Martin-Reagenz.

Eine alternative Syntheseroute, die sich vor allem für die Darstellung von Aldehyden der Formel Vo-Va eignet, in denen die Difluoroxymethylen-Brücke mit einem aromatischen Rest verknüpft ist, geht von dem Ester Ethyl-O-C(=O)-CF₂Br aus, der mit einem geeigneten Phenolat in Gegenwart von beispielsweise Hexamethylenphosphortribromid oder unter Pd⁰-Komplex-Katalyse unter Bildung der CF₂O-Brücke und nach abschließender Reduktion der Esterfunktion in den gewünschten Aldehyd Vo-Va überführt wird.

Sofern es sich bei dem Aldehyd der Formel V-IV um ein Phenanthrenderivat der Formel 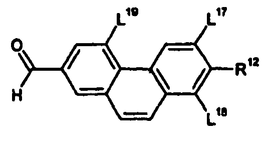 handelt, so ist dieses nach folgendem Schema 5 zugänglich:

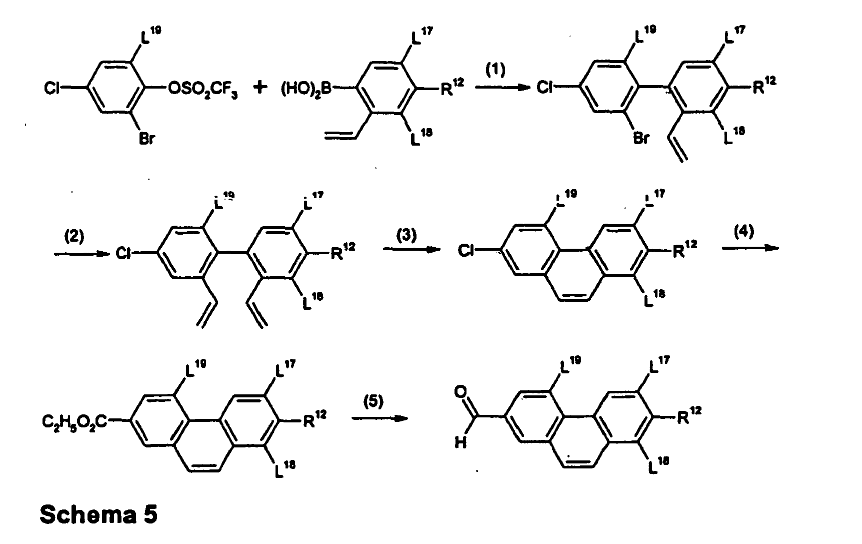

In Schritt (1) erfolgt eine C-C-Kupplung unter Pd°-Katalyse zum Biphenyl, das in Schritt (2) mit Vinylmagnesiumbromid oder Vinylzinkbromid in Gegenwart eines Palladium-Komplexes (PdCl₂·dppf) in das Divinylderivat überführt wird. In Schritt (3) erfolgt eine intramolekulare Kreuzmetathese zum Phenanthrenderivat in Gegenwart eines Ruthenium-Alkyliden-Komplexes COMP-RuA, bevorzugt COMP-Ru2a-c oder COMP-Ru4. Das Chlorphenanthren wird anschließend in Schritt (4) mit CO/Ethanol bei 70 °C und 5 bar in Gegenwart von PdCl₂·[2P(Cyclohexyl)₃] als Katalysator in den Ethylester überführt, der nach abschließender Reduktion mit DIBAH in Schritt (5) den gewünschten Phenanthrenaldehyd ergibt.

Zur Herstellung des Phenanthrenaldehyds der Formel 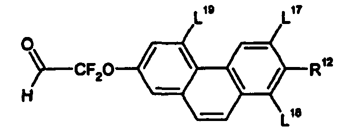 setzt man in Schritt (1) von Schema 5 anstelle der Chlor-substituierten Verbindung die korrespondierende Benzyloxyverbindung 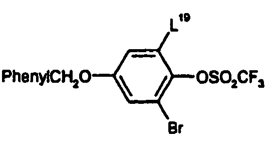 ein, die entsprechend in den Schritten (1) bis (4) in die korrespondierende Benzyloxy-substituierte Phenanthrenverbindung überführt wird. Nach reduktiver Abspaltung der Benzyloxyschutzgruppe (mit Wasserstoff und Pd-C) wird das resultierende Hydroxyphenanthren wie oben dargelegt mit Ethyl-O-C(=O)-CF₂Br und nach abschließender Reduktion der Esterfunktion in den gewünschten Aldehyd überführt. Die Ausgangsverbindungen für diese Phenanthrensynthesen sind entweder kommerziell oder nach bekannten Synthesemethoden leicht zugänglich.

Die für die Herstellung von erfindungsgemäßen Verbindungen der Formel I-G nach dem erfindungsgemäßen Verfahren mittels ringschließender Metathese-Reaktion erforderlichen Ausgangsverbindungen sind auch nach einem weiteren Verfahren zugänglich, das in Schema 6 illustriert ist:

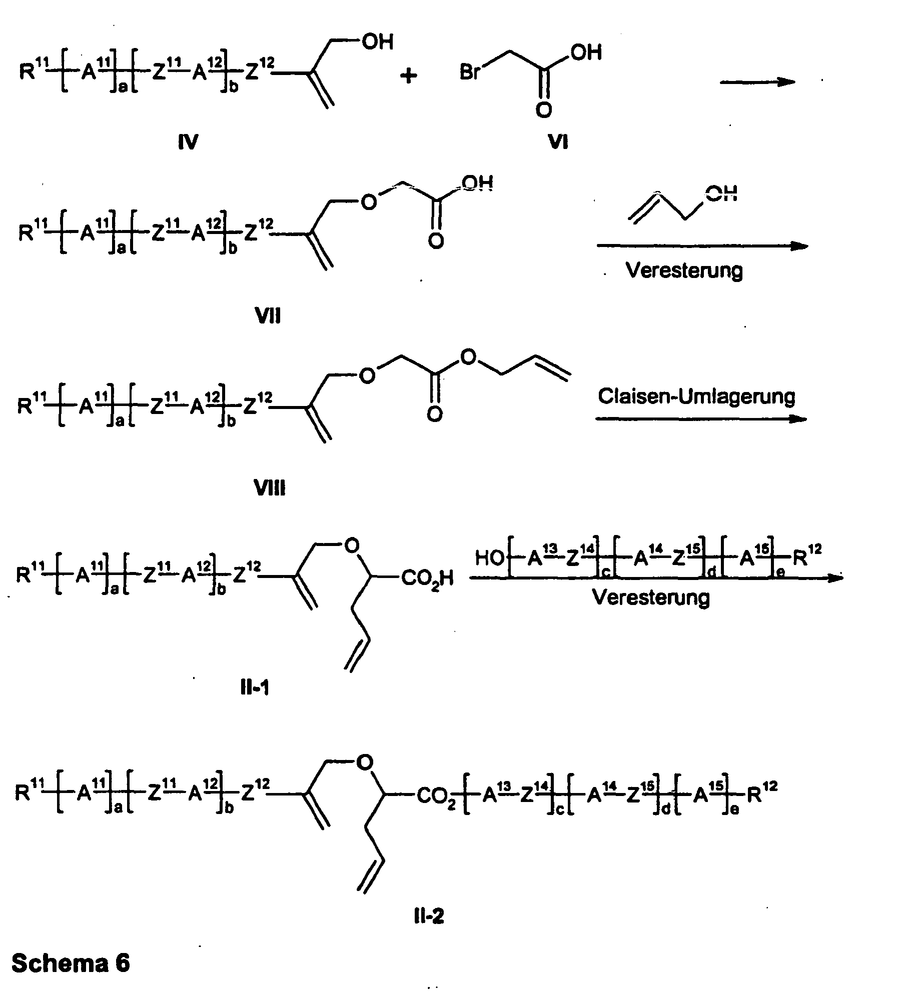

Dabei wird zunächst z.B. aus dem Allylalkohol IV und Bromessigsäure VI die Säure VII hergestellt (gemäß z.B. J. March: Advanced Organic Chemistry; John Wiley & Sons, New York u.a., 3. Aufl., 1985, S. 342, 0-14), die anschließend mit Allylalkohol unter üblichen Bedingungen (vgl. u.a. J. March: Advanced Organic Chemistry; John Wiley & Sons, New York u.a., 3. Aufl., 1985, S. 346-351, 0-22, 0-23 und 0-24) in die Verbindung VIII überführt wird.

Diese wird anschließend einer Claisen-Umlagerung (siehe z.B. J. March: Advanced Organic Chemistry; John Wiley & Sons, New York u.a., 3. Aufl., 1985, S. 1028, 8-37) unterworfen, z.B. indem eine Lösung von VIII in THF bei sehr tiefen Temperaturen (ca. -100 °C) zunächst mit Lithiumdiisopropylamid (LDA) und dann mit einer 1:1-Mischung von Trimethylsilylchlorid und Triethylamin versetzt wird; nach langsamem Auftauen wird mit wäßriger Base oder Säure versetzt und nach üblicher Aufarbeitung die Säure II-1 erhalten. Für die erfindungsgemäße ringschließende Metathese-Reaktion ist es bevorzugt, anstelle der freien Carbonsäure II-1 einen Ester II-2 einzusetzen, der nach üblichen Veresterungs-Methoden hergestellt wird und nach der Metathese-Reaktion die erfindungsgemäßen Verbindungen der Formel I-G ergibt.

Es ist selbstverständlich, dass zum einen Vorstufen und Ausgangsverbindungen der Formeln II, IV und V für erfindungsgemäße Verbindungen der Formel I aus anderen geeigneten Vorstufen und Ausgangsverbindungen der Formeln II, IV und V hergestellt werden können, und zum anderen auch erfindungsgemäße Verbindungen der Formel I nach erfolgtem Ringschluss in andere erfindungsgemäße Pyranderivate der Formel I umgewandelt werden können. Als besonders nützlich haben sich dabei Carbonsäure-, Benzyloxy- und Halogenderivate erwiesen.

So kann beispielsweise der Carbonsäureester der Formel II-2-(mit c = d = e = 0 und R¹² Aralkyl, Alkanyl oder Alkenyl) zuerst einer ringschließenden Metathese-Reaktion unter Bildung des entsprechenden Pyranderivats der Formel I-G (mit c = d = e = 0 und R¹² Aralkyl, Alkanyl oder Alkenyl) unterworfen werden. Durch Umesterung dieses Esters mit Synthesebausteinen des Typs HO-[A¹³-Z¹⁴]_{c}-[A¹⁴-Z¹⁵]_{d}-[A¹⁵]ₑ-R¹² können dann andere erfindungsgemäße Verbindungen der Formel I-G erhalten werden. Eine entsprechende Veresterung kann aber auch, falls gewünscht, vor der ringschließenden Metathese-Reaktion mit der Vorstufe II-1 ausgeführt werden.

Die erfindungsgemäßen Carbonsäuren der Formel I-G (mit R¹² = H und c = d = e = 0), die aus den entsprechenden Estern durch basische oder saure Verseifung dargestellt werden, können eingesetzt werden, um über das korrespondierende Bis(alkythio)carbeniumsalz und anschließende oxidative Fluordesulfurierung (vgl. WO 01/64667) das entsprechende CF₂O-verbrückte Pyranderivat der Formel I-H zu erhalten. Die oxidative Fluordesulfurierung ist natürlich auch bei solchen erfindungsgemäßen Verbindungen der Formel I anwendbar, bei denen die Carboxyl-Funktion nicht direkt mit dem zentralen Pyranring verknüpft ist, d.h. wenn Z¹⁴ oder Z¹⁵ = -CO₂H bedeutet; auf diese Weise werden dann -CF₂O-[A¹⁴-Z¹⁵]_{d}-[A¹⁵]ₑ-R¹²- beziehungsweise -CF₂O-A¹⁵-R¹²-Reste eingeführt.

Aus den erfindungsgemäßen Carbonsäureestern der Formel I-G mit R¹²≠ H und c = d = e = 0 sind entweder durch direkte Reduktion beispielsweise mit einem geeigneten Metallhydrid oder zweistufig durch Reduktion zum primären Alkohol und anschließende schonende Oxidation mit beispielsweise Dess-Martin-Reagenz erfindungsgemäße Aldehyde der Formel I mit Z¹³-R¹² = -C(=O)-H zugänglich. Die Reduktion zu erfindungsgemäßen Pyranderivaten mit Aldehyd-Funktion ist selbstverständlich auch mit Verbindungen der Formel I durchführbar, bei denen die Carbonsäureester-Funktion nicht direkt mit dem zentralen Pyranring verknüpft ist, d.h. wenn Z¹⁴ oder Z¹⁵ = -CO₂R¹² bedeutet. Auf diese Weise wird die -C(=O)-H-Funktion als Rest Z¹⁴-R¹² beziehungsweise Z¹⁵-R¹² eingeführt.

Aus den so zugänglichen Aldehyden der Formel I sind beispielsweise mittels Wittig- oder Wittig-Horner-Reaktion mit Molekülen des Typs R^{x}-[A¹³-Z¹⁴]_{c}-[A¹⁴-Z¹⁵]_{d}-[A¹⁵]ₑ-R¹² (wobei R^{x} beispielsweise für (Phenyl)₃P=CH- oder (EthylO)₂P(=O)-CH₂- steht) die entsprechenden erfindungsgemäßen Verbindungen der Formel I mit Z¹³, Z¹⁴ beziehungsweise Z¹⁵ = -CH=CH- zugänglich.

Die Herstellung von erfindungsgemäßen Ketonen der Formel I mit Z¹³-R¹², Z¹⁴-R¹² beziehungsweise Z¹⁵-R¹² = -C(O)-R¹² und R¹² = Alkanyl, Alkenyl oder Aralkyl erfolgt z.B. durch Umsetzung des korrespondierenden Carbonsäureesters der Formel I-G mit einem geeigneten metallorganischen Reagenz, zum Beispiel mit einer Verbindung R¹²-Mg-Br nach Grignard (siehe z.B. J. March: Advanced Organic Chemistry, John Wiley & Sons, New York u.a., 3. Aufl., 1985, S. 434, 0-107). Analog sind auch weitere Ketone der Formel I mit beispielsweise Z¹³-[A¹³-Z¹⁴]_{c}-[A¹⁴⁻Z¹⁵]_{d}-[A¹⁵]ₑ-R¹² gleich -C(O)-[A¹³-Z¹⁴]_{c}-[A¹⁴-Z¹⁵]_{d}-[A¹⁵]ₑ-R¹² durch Umsetzung mit einem geeigneten metallorganischen Reagenz Met*-[A¹³-Z¹⁴]_{c}-[A¹⁴⁻Z¹⁵]_{d}-[A¹⁵]ₑ-R¹² (wobei "Met*" zum Beispiel Br-Mg oder Li bedeutet) zugänglich. Sofern Z¹⁴ und Z¹⁵ nicht CO₂ bedeuten und R¹² keine Carboxylfunktion enthält, sind die Ketone der Formel I durch Reduktion der entsprechenden Ester der Formel I-G erhältlich. Analog können Keto-Funktionen auch als Z¹⁴ oder Z¹⁵ eingeführt werden.

Auch erfindungsgemäße Verbindungen der Formel I-K sind gut für weitere Derivatisierungen geeignet. Durch Umsetzung am Cyclohexanonring mit beispielsweise Trimethylsilyl-1,3-dithian in THF in Gegenwart von n-Butyllithium (in Anlehnung an das Verfahren von J. Mlynarski und A. Banaszek, Tetrahedron **55** (1999) 2785) oder analog zu den Verfahren von P. Kirsch et al., Angew. Chem. **2001**, *113,* 1528, sind die entsprechenden Ketendithioketale (mit dem Strukturbestandteil 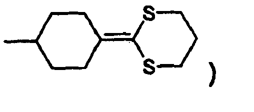 zugänglich. Oxidative Fluordesulfurierung (nach P. Kirsch et al., Angew. Chem. **2001,** *113,* 1528) ergibt dann erfindungsgemäße Verbindungen der Fomel I mit 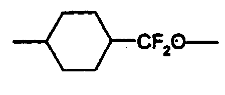 als Strukturbestandteil.

Die erfindungsgemäßen Cyclohexanone der Formel I-K können auch mit einem geeigneten Grignard-Reagenz, zum Beispiel des Typs Br-Mg-Z¹⁴-A¹⁴-R¹² mit Z¹⁴ beispielsweise -CH₂- oder -CH₂CH₂-, zu den entsprechenden tertiären Alkoholen, in diesem Beispiel mit dem Strukturelement 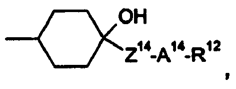 umgesetzt werden. Anschließende Reduktion des Alkohols mit Triethylsilan und Bortrifluorid-Etherat ergibt das korrespondierende 1,4-disubstiuerte Cyclohexan-Derivat (im angegebenen Beispiel mit dem Strukturelement

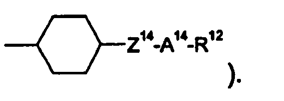

Erfindungsgemäße Verbindungen der Formel I, die einen endständigen Phenylring mit R¹² = Halogen, insbesondere Brom oder Iod, aufweisen, zum Beispiel entsprechende Verbindungen der Formeln I-CBI, I-CBII, I-CBIIIc, I-GII, I-GIII, I-HI, I-HII oder I-J, können ebenfalls als Ausgangsverbindungen zur Herstellung weiterer erfindungsgemäßer Verbindungen der Formel I eingesetzt werden. So kann - nach Metallierung unter Metall-Halogen-Austausch beispielsweise mit einer metallorganischen Base wie n-Butyllithium - die intermediäre metallierte Verbindung mit verschiedenen Reagenzien weiter umgesetzt werden, so z.B. mit CO₂ unter Bildung der korrespondierenden Carbonsäure, mit Borsäureestem oder verwandten Borverbindungen unter Bildung der korrespondierenden Aryl-Bor-Verbindungen, oder in Gegenwart geeigneter Katalysatoren mit Reaktionspartnern, die C-C-Kreuzkupplungsreaktionen beispielsweise vom Typ der Heck- oder der Suzuki-Reaktion eingehen.

Auch die genannten Aryl-Bor-Verbindungen oder die halogenierten Verbindungen selbst können in derartigen literaturbekannten Kreuzkupplungsreaktionen (siehe z.B. N. Miyaura, A. Suzuki, Chem. Rev. **1995**, 95, 2457) umgesetzt werden. Ferner sei angemerkt, dass auch bestimmte Vorstufen und Ausgangsverbindungen der erfindungsgemäßen Verfahren, zum Beispiel solche, in denen ein aromatischer Ring A¹³ direkt mit einem aromatischen Ring A¹⁴ (beziehungsweise A¹⁵) über Z¹⁴ (beziehungsweise Z¹⁵) = Einfachbindung verknüpft ist, mit Hilfe dieser Kreuzkupplungsreaktionen hergestellt werden können.

Ferner ist es bevorzugt, in dem erfindungsgemäßen Verfahren nach dem ringschließenden Metathese-Reaktionsschritt als einen weiteren Reaktionsschritt eine katalytische Hydrierung zu einem Pyranderivat der allgemeinen Formel III durchzuführen: 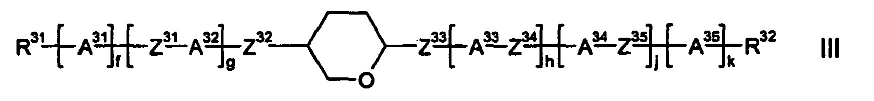 worin
- f, g, h, j und k: jeweils unabhängig voneinander 0 oder 1 sind;
- R³¹: H, einen unsubstituierten oder einfach oder mehrfach gleich oder verschieden mit Halogen oder -CN substituierten Alkylrest mit 1 bis 15 Kohlenstoffatomen bedeutet, wobei in diesem Rest auch eine oder mehrere CH₂-Gruppen durch -O-, -S-, -C(O)-O- und/oder -O-C(O)- so ersetzt sein können, dass Heteroatome (O, S) nicht direkt miteinander verknüpft sind;
- R³²: H, Halogen, -CN, -NCS, Aralkyl oder einen unsubstituierten oder einfach oder mehrfach gleich oder verschieden mit Halogen oder -CN substituierten Alkylrest mit 1 bis 15 Kohlenstoffatomen bedeutet, wobei in diesem Rest auch eine oder mehrere CH₂-Gruppen durch -O-, -S-, -C(O)-O- und/oder -O-C(O)- so ersetzt sein können, dass Heteroatome (O, S) nicht direkt miteinander verknüpft sind;
- Z³¹ und Z³²: unabhängig voneinander eine Einfachbindung, -CH₂-, -CH₂CH₂-, -CF₂CH₂-, -CH₂CF₂- oder -CF₂CF₂- bedeuten;
- Z³³, Z³⁴ und Z³⁵: jeweils unabhängig voneinander eine Einfachbindung, -CH₂CH₂-, -CF₂CH₂-, -CH₂CF₂-, -CF₂CF₂-, -CH₂O-, -CF₂O-, -C(O)- oder -C(O)-O- bedeuten;
- A³¹ und A³²: unabhängig voneinander für 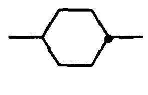 oder 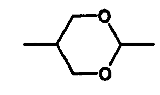 stehen;
- A³³ und A³⁴: unabhängig voneinander für 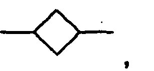 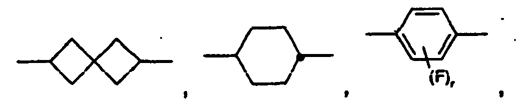 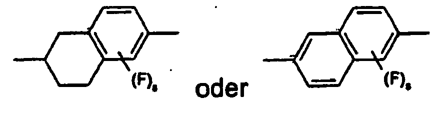 stehen;
- A³⁵: unabhängig voneinander für 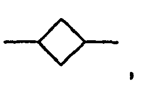 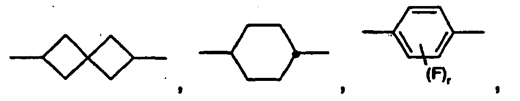 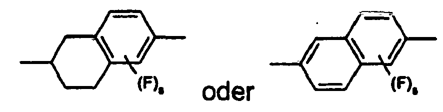 steht oder
- A³⁵ -R³²: zusammen für 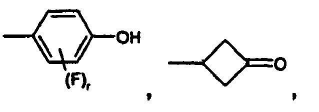 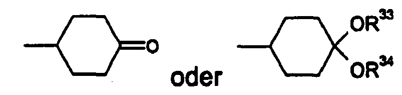 steht; oder
- Z³³-[-A³³-Z³⁴-]ₕ-[-A³⁴-Z³⁵-]ⱼ-[-A³⁵-]ₖ-R³²: für 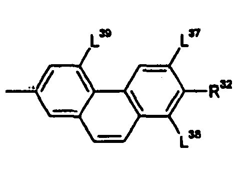 oder 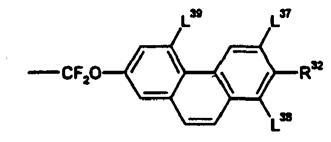 steht, wobei R³² wie oben definiert ist und L³⁷, L³⁸ und L³⁹ unabhängig voneinander H oder F bedeuten;
- r: 0, 1, 2, 3 oder 4 ist;
- s: 0, 1, 2 oder 3 ist;
- R³³ und R³⁴: unabhängig voneinander einen Alkanylrest mit 1 bis 7 Kohlenstoffatomen bedeutet oder zusammen für eine Alkylenbrücke mit 2 bis 7 Kohlenstoffatomen stehen;
mit der Maßgabe, dass für den Fall der direkten Verknüpfung von Z³³ und R³² zu -Z³³-R³²R³² H, Aralkyl oder Alkanyl bedeutet, wenn Z³³ -C(=O)-bedeutet, R³² Aralkyl oder Alkanyl bedeutet, wenn Z³³ -C(=O)-O- bedeutet, und Z³³ nicht -CH₂O- oder -CF₂O- bedeutet; dass für den Fall der direkten Verknüpfung von Z³⁴ und R³² zu -Z³⁴ -R³² R³² H, Aralkyl oder Alkanyl bedeutet, wenn Z³⁴ -C(=O)- bedeutet, R³² Aralkyl oder Alkanyl bedeutet, wenn Z³⁴ -C(=O)-O- bedeutet, und Z³⁴ nicht -CH₂O- oder -CF₂O- bedeutet; dass für den Fall der direkten Verknüpfung von Z³⁵ und R³² zu -Z³⁵-R³² R³² H, Aralkyl oder Alkanyl bedeutet, wenn Z³⁵ -C(=O)- bedeutet, R³² Aralkyl oder Alkanyl bedeutet, wenn Z³⁵ -C(=O)-O- bedeutet, und Z³⁵ nicht -CH₂O- oder -CF₂O- bedeutet; dass R³¹ nicht H bedeutet, wenn zugleich f und g beide null sind und Z³² eine Einfachbindung bedeutet.

Als Ausgangsverbindungen zur Herstellung eines Pyranderivats der allgemeinen Formel III können grundsätzlich alle erfindungsgemäßen Verbindungen der Formel I mit W = -CH₂- (d.h. Verbindungen der Formel I-B) eingesetzt werden. Bei der Hydrierung werden neben der (endocyclischen) C=C-Doppelbindung in dem zentralen Pyranring auch weitere gegebenenfalls vorhandene aliphatische C=C-Doppelbindungen der Verbindung der Formel I-B zu einer C-C-Einfachbindung hydriert. So wird beispielsweise ausgehend von einer Verbindung der Formel I-CB durch die katalytische Hydrierung die entsprechende Verbindung der Formel III-CB erhalten (siehe Schema 7), wobei dann die Bedeutung von f, g, h, j, k, R³¹, R³², Z³¹, Z³³, Z³⁴, Z³⁵, A³¹, A³², A³³, A³⁴ und A³⁵ in Formel III-CB der Bedeutung von a, b, c, d, e, R¹¹, R¹², Z¹¹, Z¹³, Z¹⁴, Z¹⁵, A¹¹, A¹², A¹³, A¹⁴ beziehungsweise A¹⁵ in Formel I-CB entspricht und Z¹² = Z³² = Einfachbindung bedeutet unter der Maßgabe, dass, wenn Z¹¹, Z¹³, Z¹⁴ und/oder Z¹⁵ -CH=CH- bedeuten beziehungsweise R¹¹ und/oder R¹² eine -CH=CH-Gruppe enthalten, diese aliphatischen -CH=CH-Gruppen in CH₂₋CH₂-Gruppen überführt werden.

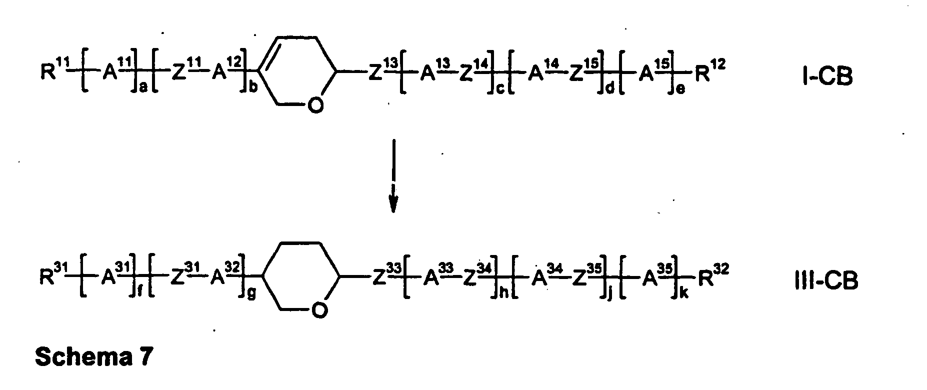

Analog werden aus den erfindungsgemäßen Verbindungen der Formel I-DB die entsprechenden hydrierten Verbindungen III-DB, aus I-EB III-EB, aus I-FB III-FB, aus I-GB III-GB, aus I-JB III-JB, aus I-KB III-KB, aus I-LB III-LB, aus I-MB III-MB, aus I-NB III-NB, aus I-OB III-OB, aus I-PB III-PB, I-QB III-QB, aus I-RB III-RB, aus I-SB III-SB und aus I-TB III-TB erhalten. Beispielhafte Verbindungen der Formel III sind solche, die sich von den in den Tabellen 1 bis 6 dargestellten Pyranderivaten der Formel I ableiten und von diesen dadurch unterscheiden, dass sie im zentralen Pyranring keine C=C-Doppelbindung aufweisen. (Der Einfachheit und der besseren Kongruenz wegen werden Verbindungen der Formel III auch als Verbindungen der Formel III-B bezeichnet.)

Die katalytische Hydrierung erfolgt üblicherweise bei einem Wasserstoff-Partialdruck von 1 bar bis 10 bar. Als Hydrierungskatalysatoren werden üblicherweise Übergangsmetall-Katalysatoren aus Nickel, Platin oder Palladium, wie zum Beispiel Raney-Nickel, 5% oder 10% Platin auf Kohlenstoff und 5% Palladium auf Aktivkohle, in einem geeigneten, Lösungsmittel, wie zum Beispiel n-Heptan, Ethylacetat, Toluol, Ethanol, Methanol oder THF, eingesetzt. Die Reaktionszeit ist an sich nicht kritisch; üblicherweise wird die Hydrierung bis zur vollständigen Umsetzung der jeweiligen Ausgangsverbindung ausgeführt. Die Reaktionstemperatur liegt im allgemeinen im Bereich zwischen Raumtemperatur und 100 °C.

Bei der Hydrierung des Pyranderivats I zu dem Pyranderivat III wird zusätzlich zu dem Asymmetriezentrum in 2-Position des Pyranrings ein weiteres chirales Zentrum in 5-Position gebildet. Sofern als Ausgangsverbindung für die katalytische Hydrierung zur Bildung des Tetrahydropyrans III ein solches Pyranderivat der Formel I eingesetzt wird, das nach entsprechender, oben beschriebener stereoselektiver Synthese oder Aufreiniung in enantiomerenreiner (oder enantiomerenangereicherter) Form vorliegt, werden üblicherweise nur 2 der 4 theoretisch denkbaren Diastereomeren von III erhalten (in Abhängigkeit von der absoluten Konfiguration am C-2-Atom mit *2R,5R-* und *2R,5S-* oder mit *2S,5R-* und 2S,5S- Konfiguration). Diese beiden Isomeren, die sich zueinander als Diastereomeren verhalten, können mittels herkömmlicher Verfahren wie fraktionierter Kristallisation oder Chromatographie voneinander getrennt werden, so dass das Tetrahydropyran der Formel III auch in enantiomerenreiner Form erhalten werden kann. Die isomerenreinen Verbindungen der Formel III finden ebenso wie die isomerenreinen Verbindungen der Formel I unter anderem Verwendung als chirale Dotierstoffe für nematische flüssigkristalline Medien, die die für verschiedene elektrooptische Anwendungen erforderliche verdrillte Anordnung der in den flüssigkristallinen Medien enthaltenen Verbindungen bewirken.

Selbstverständlich können Verbindungen der Formel III ihrerseits in andere Verbindungen der Formel III überführt werden, und zwar mittels der gleichen synthetischen Verfahren, die oben für die erfindungsgemäßen Verbindungen der Formel I ausführlich erläutert worden sind. Wie auch im Falle der erfindungsgemäßen Verbindungen der Formel I, die eine Carboxylfunktion aufweisen, eignen sich hierfür besonders Carbonsäureester der Formel III-GB, insbesondere solche der Formel III-GBI. Sie können beispielsweise zur entsprechenden freien Carbonsäure verseift, durch Hydrierung debenzyliert oder Palladium-katalysiert desallyliert und dann weiter derivatisiert werden, um zum Beispiel die Einführung einer Difluoroxymethylenbrücke zu ermöglichen. Unter vielen anderen Derivatisierungsmöglichkeiten soll hier auch beispielhaft die Möglichkeit erwähnt werden, den Ester der Formel III-GBI zum entsprechenden Aldehyd zu reduzieren, welcher seinerseits in einer Wittig- oder Wittig-Horner-Reaktion unter Einführung einer aliphatischen C=C-Doppelbindung umgesetzt werden kann.

Ferner sind Verbindungen der Formel III-KB nicht nur direkt durch Hydrierung von entsprechenden Verbindungen der Formel I-KB zugänglich. Sie können zum Beispiel auch in der Weise hergestellt werden, dass ein Pyranderivat der Formel I-B mit einem endständigem Phenylring mit R¹² = O-Aralkyl, wie z.B. Verbindungen der Formel I-CBIIIa mit R^{12a} = Aralkyl, zunächst unter gleichzeitiger Debenzylierung zu der korrespondierenden Verbindung der Formel III-B mit endständigem Phenolring hydriert wird, um sie anschließend mit geeigneten Reduktionsmitteln (siehe z.B. J. March: Advanced Organic Chemistry; John Wiley & Sons, New York u.a., 3. Aufl., 1985, S. 700, 5-11) in die entsprechende Verbindung der Formel III-KB mit endständigem Cyclohexanonring zu überführen. Für die beispielhaften Verbindungen wird dieses Vorgehen durch Schema 8 wiedergegeben. Die Verbindungen der Formel III-KB sind insbesondere für die Einführung von CF₂O-Brücken geeignet, deren Einzelheiten oben für die Umsetzung der ebenfalls erfindungsgemäßen Cyclohexanonderivate der Formel I-KB beschrieben sind.

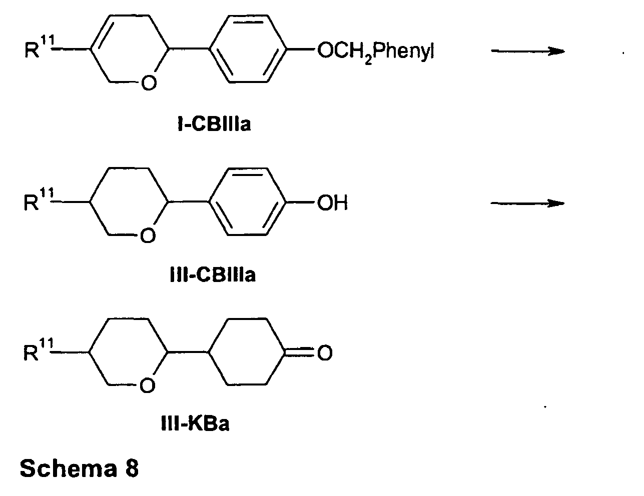

Aufgrund der 2,5-Disubstitution des zentralen Pyranrings kann die Verbindung der Formel III sowohl als cis- als auch als trans-Isomer vorliegen. Mitunter wird das im allgemeinen für viele Verwendungen bevorzugte trans-Isomer als einziges Produkt der Hydrierung erhalten. Fällt das Pyran der Formel III vorwiegend als cis-Isomer oder als Gemisch beider Isomeren an, so wird das bevorzugte trans-Isomer durch Behandlung mit einer starken Base, zum Beispiel Kalium-tert.-butylat in N-Methylpyrrolidon, oder mit einer starken Säure, zum Beispiel Schwefelsäure in Dioxan, aus dem cis-Isomeren erhalten.

Die Verbindungen der Formel III sind mesogen, vorzugsweise flüssigkristallin, und finden Verwendung in flüssigkristallinen Medien.

Die vorliegende Erfindung betrifft ferner die Verwendung eines Pyranderivats der Formel I oben als Bestandteil eines flüssigkristallinen Mediums, das insbesondere in elektrooptischen Anzeigevorrichtungen wie TN-, STN- und Aktiv-Matrix-Displays eingesetzt wird. Bei dieser elektrooptischen Anzeigevorrichtung handelt es sich beispielsweise um Displays von Mobilfunkgeräten, Bildschirme von portablen Computern (Notebooks) und TFT-Flachbildschirme.

Vor- und nachstehend bedeuten Prozentangaben Gewichtsprozent. Alle Temperaturen sind in Grad Celsius angegeben. Fp. bedeutet Schmelzpunkt, Klp. Klärpunkt. Ferner bedeuten K beziehungsweise C = kristalliner Zustand, N = nematische Phase, S = smektische Phase und I = isotrope Phase. S_{c} bedeutet eine smektische C Phase, S_{B} eine smektische B Phase, S_{A} eine smektische A Phase. An bezeichnet die optische Anisotropie (Δn = nₑ - nₒ, wobei nₑ der Brechungsindex des außerordentlichen und nₒ der Brechungsindex des ordentlichen Strahls ist) (589 nm, 20 °C). Δε bezeichnet die dielektrische Anisotropie (Δε = ε_{∥} - ε_{⊥}, wobei ε_{∥} die Dielektrizitätskonstante parallel zu den Moleküllängsachsen und ε_{⊥} die Dielektrizitätskonstante senkrecht dazu bedeutet) (1 kHz, 20 °C). Die optischen Daten wurden bei 20 °C gemessen, sofern nicht ausdrücklich etwas anderes angegeben wird. Die Rotationsviskosität γ₁ [mPa·s] wurde ebenfalls bei 20 °C bestimmt. Zur experimentellen Bestimmung der physikalischen Parameter wurde gemäß "Licristal, Physical Properties Of Liquid Crystals, Description of the measurement methods", Hrsg. W. Becker, Merck KGaA, Darmstadt, überarbeitete Ausgabe, 1998, verfahren, wobei die Eigenschaften von Einzelverbindungen zum Teil nach Messung einer definierten Menge der Verbindung (zumeist 5 oder 10 Gew.-%) in einer definierten Host-Mischung mit bekannten Eigenschaften und anschließende Extrapolation ermittelt wurden.

Die beigefügten Beispiele veranschaulichen die vorliegende Erfindung weiter, ohne sie in irgendeiner Weise zu beschränken.

### Beispiele

Die in den beispielhaften Synthesen eingesetzten Ausgangsverbindungen, Reagenzien und Lösungsmittel wurden entweder käuflich erworben oder nach literaturbekannten Verfahren hergestellt. Die beispielhaften Synthesen wurden üblicherweise in trockenen Apparaturen unter Feuchtigkeitsausschluss und - sofern die betreffende Umsetzung dies erforderte - auch unter Schutzgasatmosphäre zum Luftausschluss ausgeführt.

Der Verlauf von Reaktionen wurde im allgemeinen mittels Dünnschichtchromatographie oder Gaschromatographie überwacht. Die Reaktionsprodukte wurden nach üblichen Verfahren aufgearbeitet und gereinigt, beispielsweise mittels Säulenchromatographie beziehungsweise Kristallisation. Ihre strukturelle Identität wurde mittels Massenspektrometrie und ¹H-NMR-Spektroskopie gesichert. Die Ausbeuten sind nicht optimiert.

### Beispiel 1 - Ringschließende Metathese

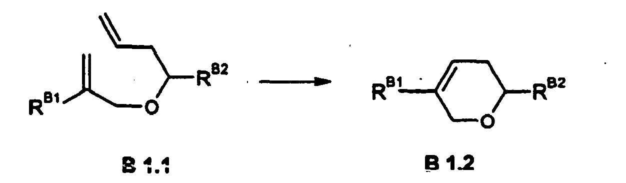

### Allgemeine Arbeitsvorschrift B1 (AAV B1)

Verbindung B1.1 (100 mmol) wird in Substanz oder gelöst in Toluol unter Stickstoffatmosphäre auf 40 bis 60 °C erwärmt. Dann wird Tricyclohexylphospin(1,3-bis(2,4,6-trimethylphenyl)-4,5-dihydroimidazol-2-yliden-benzylidenrutheniumdichlorid (COMP-Ru2a) (0,1 bis 0,5 mmol; Strem Chemicals Inc., Kehl, Deutschland) auf einmal hinzugegeben, wobei sofort sehr starke Gasentwicklung einsetzt. Es wird 15 Minuten bei 60 °C gerührt. Nach dem Abklingen der Gasentwicklung aufgrund des durch die Reaktion gebildeten Ethens wird nochmals etwas Katalysator (0,05-0,1 mmol) hinzugegeben, um sich durch das ausbleibende Aufschäumen vom Ende der Reaktion zu überzeugen. Die Reaktionsmischung wird an Kieselgel gereinigt und ergibt das Produkt B1.2 nach Kristallisation oder Vakuumdestillation. Ausbeuten: 45-75%.

Einige der gemäß AAV B1 hergestellten Verbindungen sind in Tabelle B1 wiedergegeben.

(Bei Durchführung der Reaktion in größerem Maßstab erfolgt die Zugabe des Katalysators portionsweise (zumeist 4 bis 8 Portionen zu je ca. 0,0025 mol-%). Das Ende der Reaktion wird dadurch indiziert, dass nach erneuter Zugabe von Katalysator keine Gasentwicklung mehr zu beobachten ist.)

**Tabelle B1**

| **Verbindung B 1.2-Nr.** | **R**^{**B1**} | **R**^{**B2**} |
|---|---|---|
| -1 | 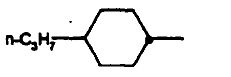 | 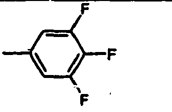 |
| -2 | 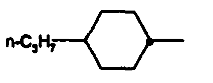 | 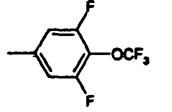 |
| -3 | 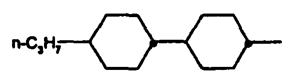 | 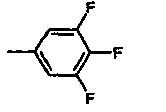 |
| -4 | n-C₃H₇ | 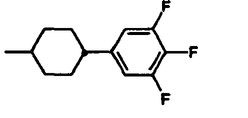 |
| -5 | n-C₃H₇ | 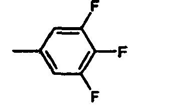 |
| -6 | 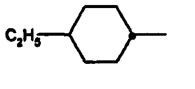 | 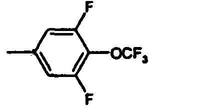 |
| -7 | 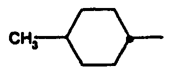 | 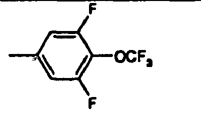 |
| -8 | CH₃ | 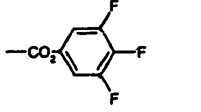 |
| -9 | CH₃ | 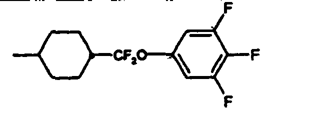 |
| -10 | CH₃ | 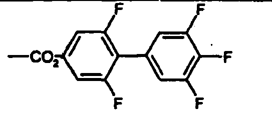 |
| -11 | 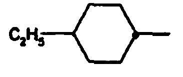 | 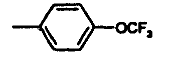 |
| -12 | 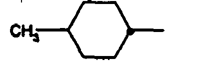 | 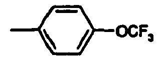 |
| -13 | C₂H₅ | 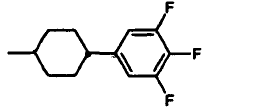 |
| -14 | n-C₃H₇ | 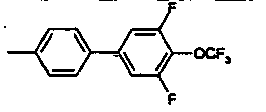 |
| -15 | n-C₃H₇ | 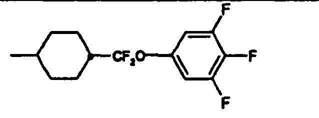 |
| -16 | C₂H₅ | 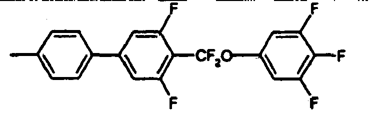 |
| -17 | C₂H₅ | 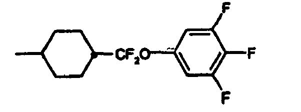 |
| -18 | n-C₃H₇ | 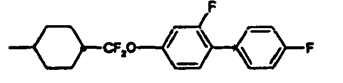 |
| -19 | n-C₃H₇ | 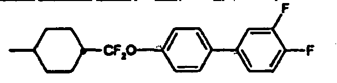 |
| -20 | n-C₃H₇ | 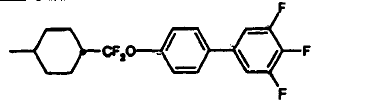 |
| -21 | n-C₃H₇ | 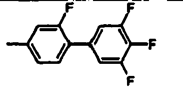 |
| -22 | C₂H₅ | 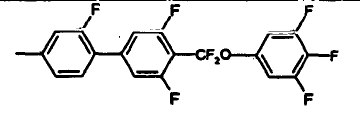 |
| -23 | n-C₃H₇ | 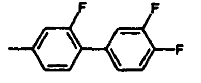 |
| -24 | n-C₅H₁₁ | 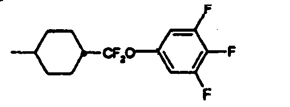 |
| -25 | n-C₃H₇ | 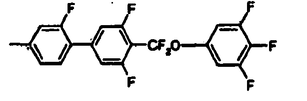 |
| -26 | n-C₃H₇ | 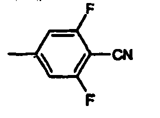 |
| -27 | n-C₃H₇ | 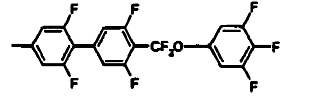 |
| -28 | n-C₄H₉ | 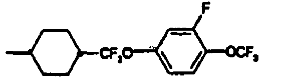 |
| -29 | n-C₄H₉ | 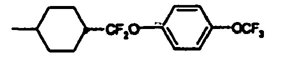 |
| -30 | n-C₃H₇ | 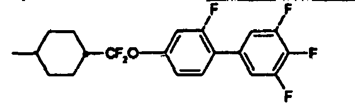 |
| -31 | n-C₃H₇ | 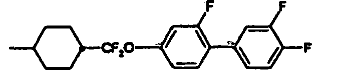 |
| -32 | C₂H₆ | 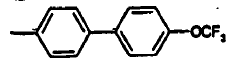 |
| -33 | n-C₃H₇ | 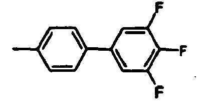 |
| -34 | n-C₃H₇ | 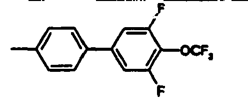 |
| -35 | n-C₃H₇ | 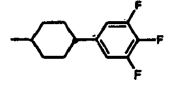 |
| -36 | n-C₃H₇ | 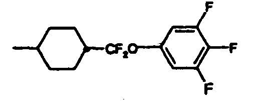 |
| -37 | C₂H₅ | 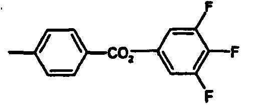 |
| -38 | C₂H₅ | 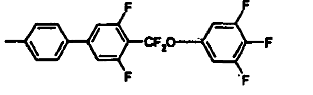 |
| -39 | C₂H₅ | 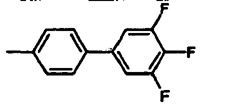 |
| -40 | n-C₃H₇ | 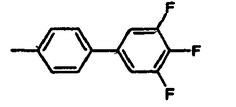 |
| -41 | C₂H₅ | 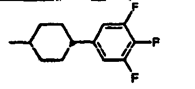 |
| -42 | C₂H₅ | 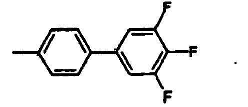 |
| -43 | n-C₃H₇ | 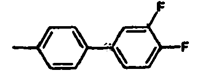 |
| -44 | n-C₃H₇ | 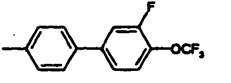 |
| -45 | n-C₃H₇ | 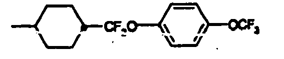 |
| -46 | n-C₃H₇ | 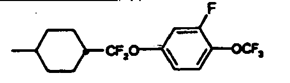 |
| -47 | n-C₃H₇ | 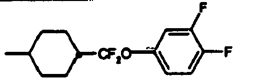 |
| -48 | n-C₃H₇ | 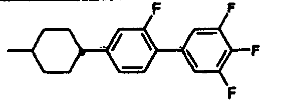 |
| -49 | C₂H₅ | 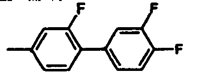 |
| -50 | C₂H₅ | 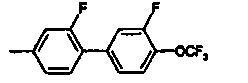 |
| -51 | C₂H₅ | 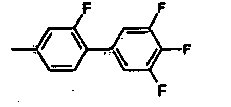 |
| -52 | n-C₃H₇ | 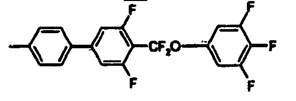 |
| -53 | n-C₃H₇ | 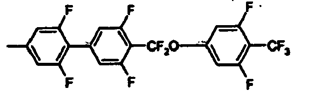 |
| -54 | n-C₃H₇ | 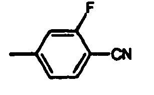 |
| -55 | n-C₃H₇ | 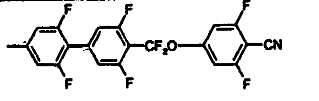 |
| -56 | n-C₃H₇ | 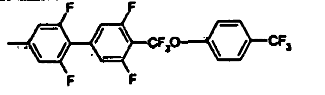 |
| -57 | 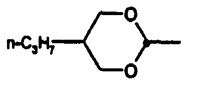 | 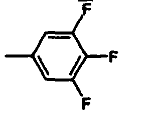 |
| -58 | n-C₃H₇ | 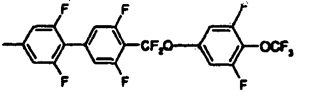 |
| -59 | n-C₃H₇ | 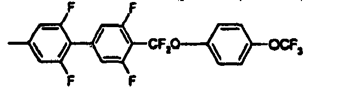 |
| -60 | n-C₃H₇ | 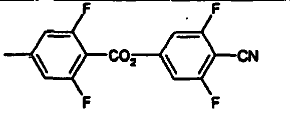 |
| -61 | n-C₃H₇ | 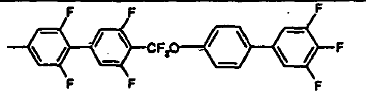 |
| -62 | CH₃ | 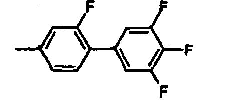 |
| -63 | 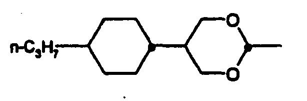 | 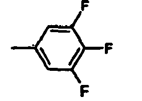 |
| -64 | n-C₃H₇ | 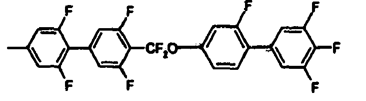 |
| -65 | n-C₃H₇ | 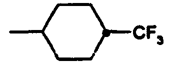 |
| -66 | 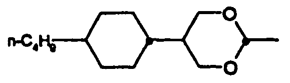 | 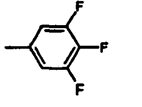 |
| -67 | n-C₃H₇ | 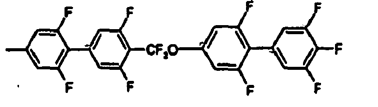 |
| -68 | n-C₃H₇ | 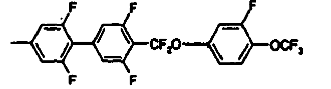 |
| -69 | n-C₅H₁₁ | 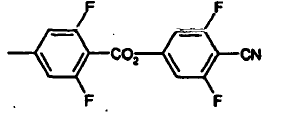 |
| -70 | C₂H₅ | 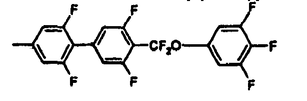 |
| -71 | C₂H₅ | 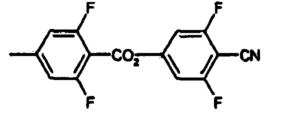 |
| -72 | n-C₄H₉ | 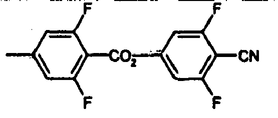 |
| -73 | n-C₃H₇ | 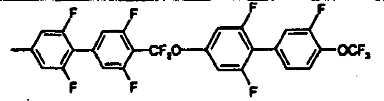 |
| -74 | n-C₃H₇ | 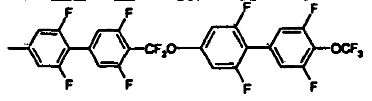 |
| -75 | C₂H₅ | 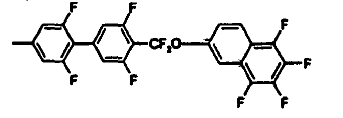 |
| -76 | C₂H₅ | 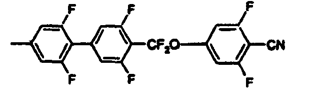 |
| -77 | n-C₅H₁₁ | 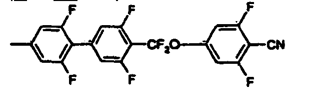 |
| -78 | n-C₄H₉ | 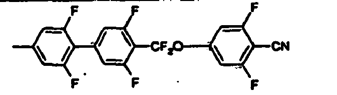 |
| -79 | n-C₇H₁₅ | 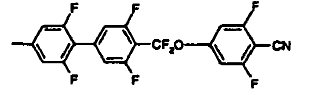 |
| -80 | n-C₄H₉ | 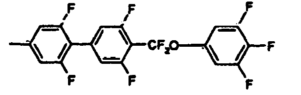 |
| -81 | n-C₅H₁₁ | 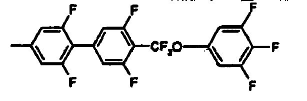 |
| -82 | n-C₇H₁₃ | 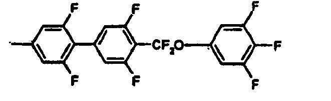 |
| -83 | CH₃ | 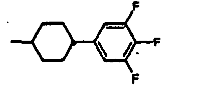 |
| -84 | n-C₄H₉ | 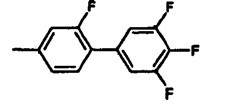 |
| -85 | n-C₃H₇ | 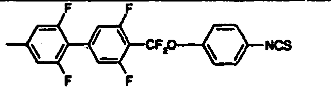 |
| -86 | n-C₃H₇ | 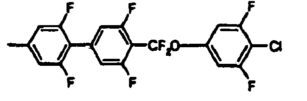 |
| -87 | n-C₆H₁₃ | 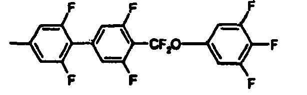 |
| -88 | n-C₃H₇ | 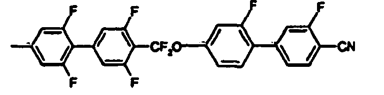 |

### Beispiel 2 - Hydrierung

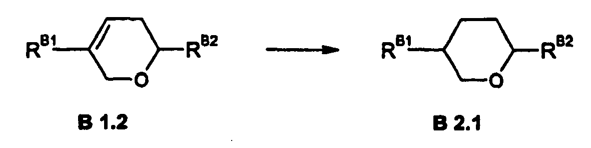

### Allgemeine Arbeitsvorschrift B2 (AAV B2)

Verbindung B1.2 (100 mmol) wird in Heptan gelöst, mit Hydrierkatalysator (10% Platin auf Aktivkohle oder 5%-Pd-C) versetzt und bei einer Temperatur zwischen Raumtemperatur und 50°C unter einem Wasserstoff-Partialdruck von 5 bar hydriert. Die Reaktionslösung wird eingeengt, filtriert, und das Rohprodukt B2.1 wird gegebenenfalls isomerisiert und chromatographiert sowie anschließend im Vakuum destilliert und/oder kristallisiert. Die cis/trans-Isomerisierung wird entweder durch zweistündiges Rühren mit 15 mol-% Kalium-tert.-butylat in N-Methylpyrrolidon oder unter Verwendung einer Lewissäure wie MgBr₂ in Toluol zwischen -10 °C und Raumtemperatur oder mit einer Brönsted-Säure wie Trifluormethansulfonsäure bei Raumtemperatur für 24 h durchgeführt.

Einige der gemäß AAV B2 hergestellten Verbindungen sind in Tabelle B2 wiedergegeben.

**Tabelle B2**

| **Verbindung B 2.1-Nr.** | **R**^{**81**} | **R**^{**82**} |
|---|---|---|
| -1 | 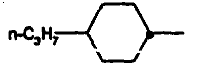 | 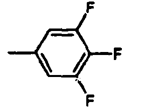 |
| -2 | 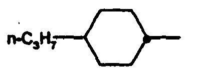 | 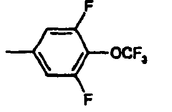 |
| -3 | 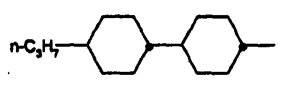 | 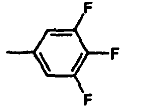 |
| -4 | n-C₃H₇ | 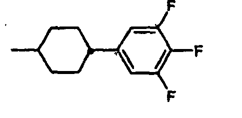 |
| -5 | n-C₃H₇ | 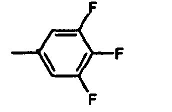 |
| -6 | 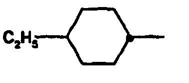 | 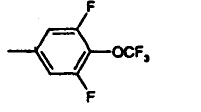 |
| -7 | 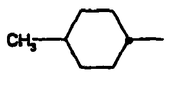 | 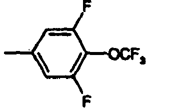 |
| -8 | CH₃ | 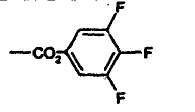 |
| -9 | CH₃ | 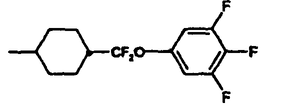 |
| -10 | CH₃ | 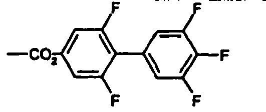 |
| -11 | 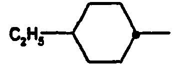 | 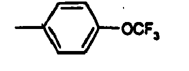 |
| -12 | 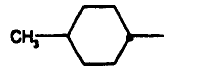 | 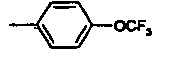 |
| -13 | C₂H₅ | 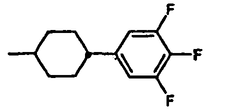 |
| -14 | n-C₃H₇ | 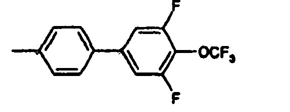 |
| -15 | n-C₃H₇ | 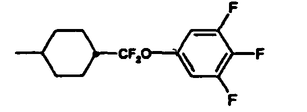 |
| -16 | C₂H₅ | 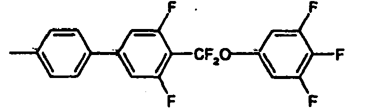 |
| -17 | C₂H₅ | 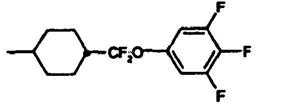 |
| -18 | n-C₃H₇ | 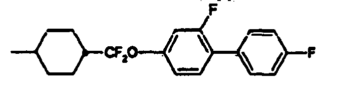 |
| -19 | n-C₃H₇ | 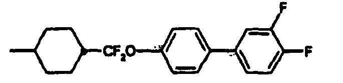 |
| -20 | n-C₃H₇ | 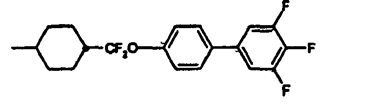 |
| -21 | n-C₃H₇ | 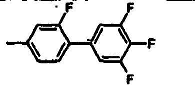 |
| -22 | C₂H₅ | 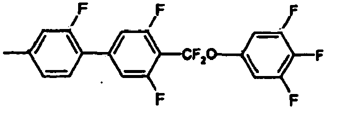 |
| -23 | n-C₃H₇ | 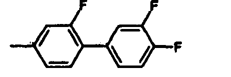 |
| -24 | n-C₅H₁₁ | 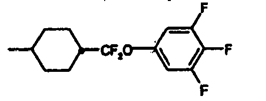 |
| -25 | n-C₃H₇ | 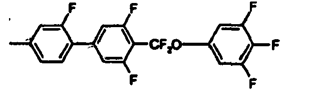 |
| -26 | n-C₃H₇ | 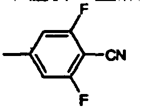 |
| -27 | n-C₃H₇ | 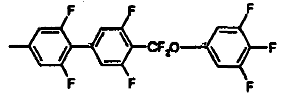 |
| -28 | n-C₄H₉ | 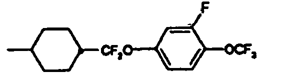 |
| -29 | n-C₄H₉ | 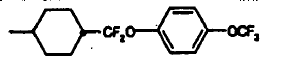 |
| -30 | n-C₃H₇ | 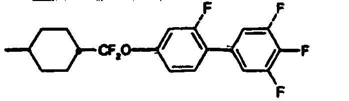 |
| -31 | n-C₃H₇ | 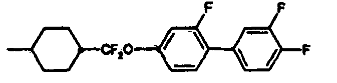 |
| -32 | C₂H₅ | 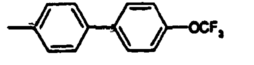 |
| -33 | n-C₃H₇ | 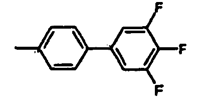 |
| -34 | n-C₃H₇ | 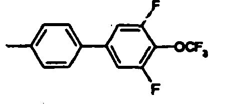 |
| -35 | n-C₃H₇ | 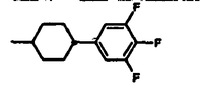 |
| -36 | n-C₃H₇ | 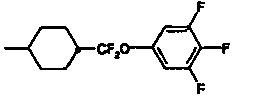 |
| -37 | C₂H₅ | 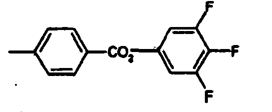 |
| -38 | C₂H₅ | 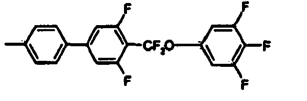 |
| -39 | C₂H₅ | 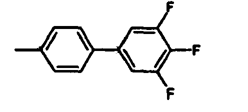 |
| -40 | n-C₃H₇ | 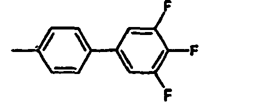 |
| -41 | C₂H₅ | 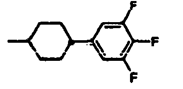 |
| -42 | C₂H₅ | 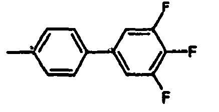 |
| -43 | n-C₃H₇ | 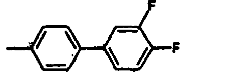 |
| -44 | n-C₃H₇ | 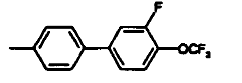 |
| -45 | n-C₃H₇ | 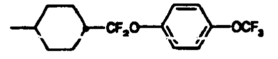 |
| -46 | n-C₃H₇ | 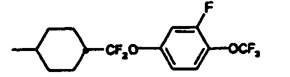 |
| -47 | n-C₃H₇ | 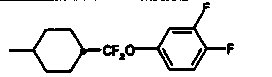 |
| -48 | n-C₃H₇ | 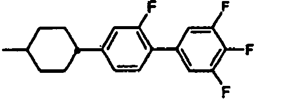 |
| -49 | C₂H₅ | 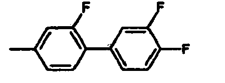 |
| -50 | C₂H₅ | 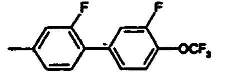 |
| -51 | C₂H₅ | 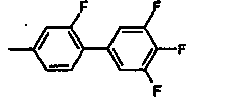 |
| -52 | n-C₃H₇ | 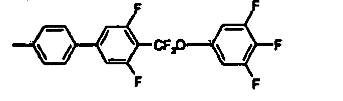 |
| -53 | n-C₃H₇ | 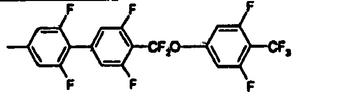 |
| -54 | n-C₃H₇ | 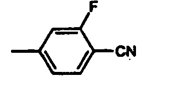 |
| -55 | n-C₃H₇ | 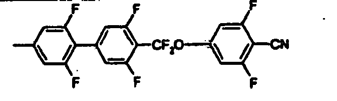 |
| -56 | n-C₃H₇ | 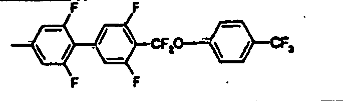 |
| -57 | 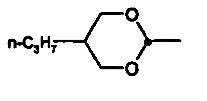 | 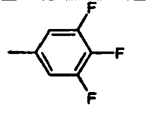 |
| -58 | n-C₃H₇ | 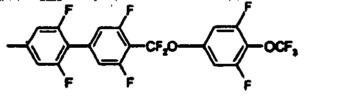 |
| -59 | n-C₃H₇ | 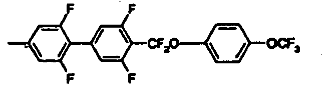 |
| -60 | n-C₃H₇ | 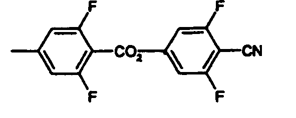 |
| -61 | n-C₃H₇ | 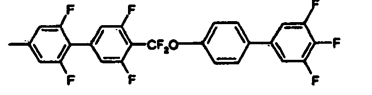 |
| -62 | CH₃ | 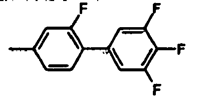 |
| -63 | 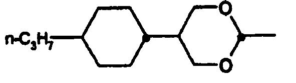 | 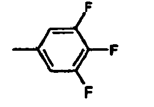 |
| -64 | n-C₃H₇ | 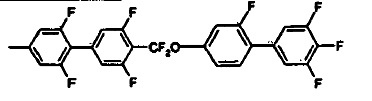 |
| -65 | n-C₃H₇ | 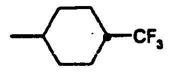 |
| -66 | 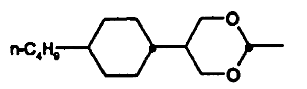 | 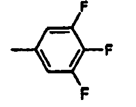 |
| -67 | n-C₃H₇ | 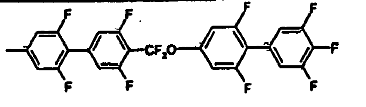 |
| -68 | n-C₃H₇ | 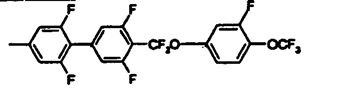 |
| -69 | n-C₅H₁₁ | 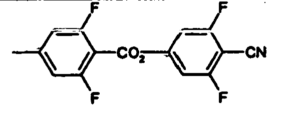 |
| -70 | C₂H₅ | 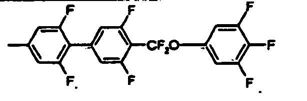 |
| -71 | C₂H₅ | 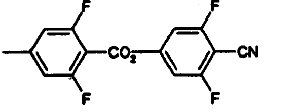 |
| -72 | n-C₄H₉ | 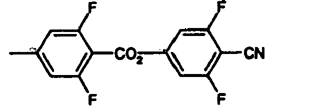 |
| -73 | n-C₃H₇ | 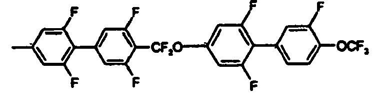 |
| -74 | n-C₃H₇ | 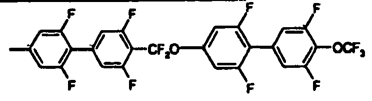 |
| -75 | C₂H₅ | 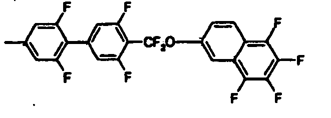 |
| -76 | C₂H₅ | 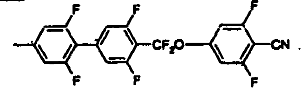 |
| -77 | n-C₅H₁₁ | 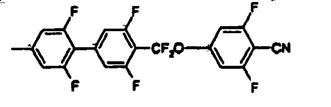 |
| -78 | n-C₄H₉ | 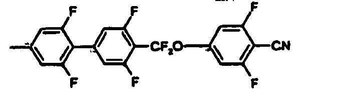 |
| -79 | n-C₇H₁₅ | 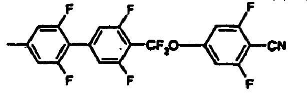 |
| -80 | n-C₄H₉ | 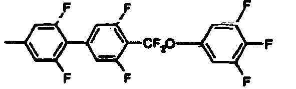 |
| -81 | n-C₅H₁₁ | 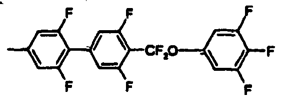 |
| -82 | n-C₇H₁₃ | 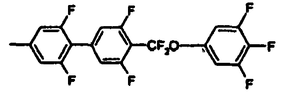 |
| -83 | CH₃ | 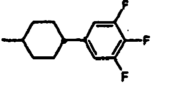 |
| -84 | n-C₄H₉ | 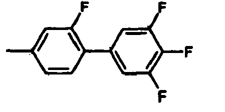 |
| -85 | n-C₃H₇ | 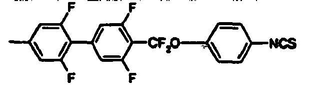 |
| -86 | n-C₃H₇ | 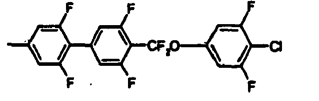 |
| -87 | n-C₆H₁₃ | 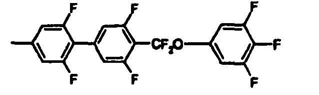 |
| -88 | n-C₃H₇ | 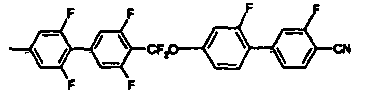 |

### Beispiel 3 - Synthese von Allylverbindungen der Formel IV

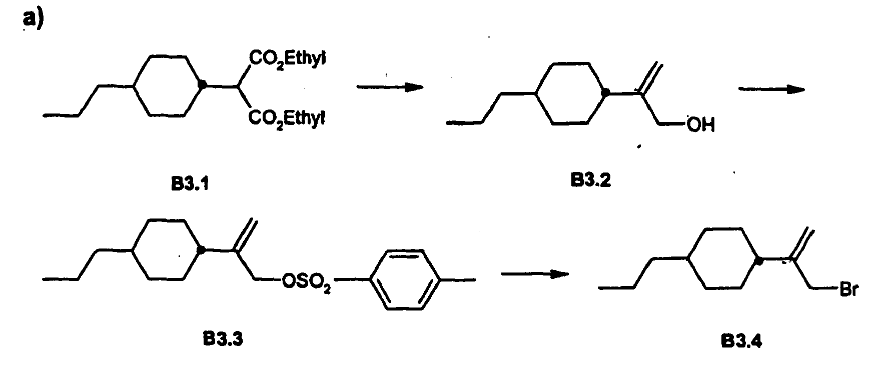

4-trans-Propylcyclohexyl-(1)-malonsäurediethylester B3.1 (0,2 mol) in wasserfreiem Dimethoxyethan wird zu einer Suspension von Natriumhydrid (0,24 mol) in Dimethoxyethan getropft. Es wird 5 Stunden zum Rückfluss erhitzt und dann Lithiumaluminiumhydrid (0,5 mol) bei 0 °C hinzugegeben. Anschließend wird weitere 3 Stunden zum Rückfluss erhitzt. Nach Abkühlen auf 0 °C wird Ameisensäureethylester, dann Wasser, wässrige Natronlauge und erneut Wasser hinzugegeben. Man filtriert und extrahiert die wässrige Phase mit tert.-Butylmethylether. Die vereinigten organischen Phasen werden getrocknet und eingedampft, und der Rückstand wird an Kieselgel filtriert. Nach Entfernen des Lösungsmittels wird der Allylalkohol B 3.2 erhalten. Ausbeute: 76%.

Zu einer Lösung von p-Toluolsulfonsäurechlorid (0,2 mol) und 2-(4-trans-Propylcyclohexyl-(1)-allylalkohol B3.2 (0,2 mol) in Tetrahydrofuran wird 4-Dimethylaminopyridin (0,4 mol) in Tetrahydrofuran unter Rühren bei 0 °C zugetropft. Nach vollendeter Zugabe lässt man auf Raumtemperatur kommen und erhitzt noch weitere 12 Stunden zum Rückfluss. Nach dem Abkühlen wird auf eine Mischung aus Eis und konzentrierter Salzsäure gegossen. Die organische Phase wird abgetrennt, die wässrige Phase wird dreimal mit tert.-Butylmethylether extrahiert. Die vereinigten organischen Phasen werden getrocknet und eingedampft, der Rückstand an Kieselgel chromatographiert. Der Allyltosylester B3.3 wird als Öl erhalten. Ausbeute: 87%. Analog lässt sich mit Methansulfonsäurechlorid der entsprechende Methansulfonsäureallylester und mit Trifluormethansulfonsäureanhydrid der entsprechende Trifluormethansulfonsäureallylester darstellen.

Der Allyltosylester B3.3 (0,1 mol) wird in Aceton aufgenommen und mit Lithiumbromid (0,1 mol) 36 Stunden zum Rückfluss erhitzt. Nach dem Abkühlen wird vom Niederschlag abfiltriert, eingedampft und der entstehende Rückstand über Kieselgel filtriert. Nach Einengen wird 2-(4-trans-Propylcyclohexyl-(1)-allylbromid B3.4 erhalten. Ausbeute: 68%.

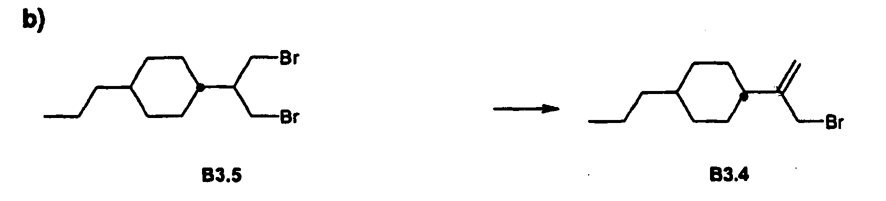

Verbindung B3.5 (0,553 mol) und Kalium-tert.-butylat (0,692 mol) werden in Toluol zusammengegeben und über Nacht bei 80 °C gerührt. Nach dem Abkühlen versetzt man mit Wasser und extrahiert mit Methyl-tert.-butylether. Die organische Phase wird abgetrennt, getrocknet und eingeengt. Der Rückstand wird in Pentan aufgenommen und an Kieselgel filtriert. Nach Einengen wird das Allylbromid B3.4 erhalten. Ausbeute: 73%.

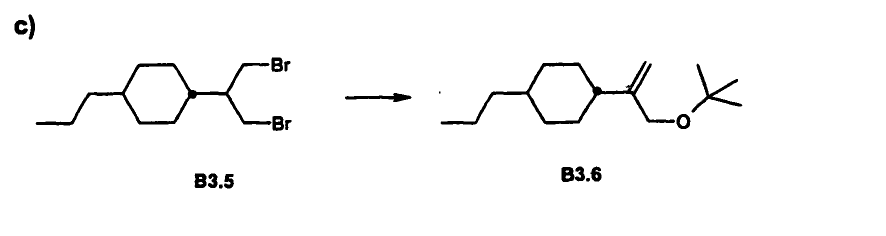

2-(4-trans-Propylcyclohexyl-(1))-1,3-dibrompropan B3.5 (0,5 mol) wird zusammen mit Kalium-tert-butylat (1,25 mol) in Toluol 16 Stunden bei 80 °C gerührt. Nach dem Abkühlen wird mit Wasser versetzt und unter Zugabe von tert.-Butylmethylether extrahiert. Die organische Phase wird getrocknet und eingedampft, der Rückstand über Kieselgel filtriert. 2-(4-trans-Propylcyclohexyl-(1))-allyl-tert.-butylether B3.6 wird als öliges Produkt erhalten. Ausbeute: 68%.

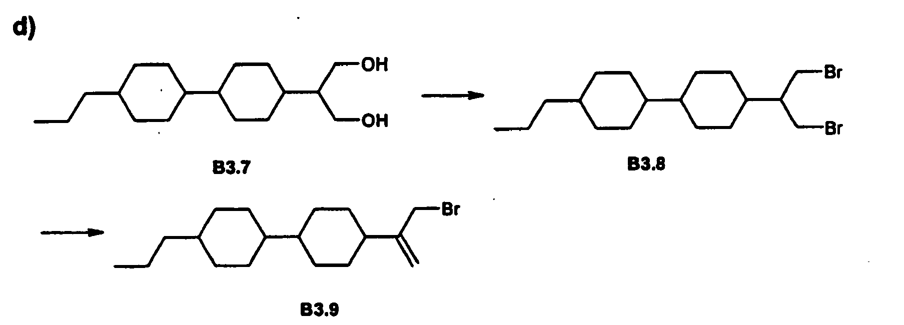

Triphenylphosphan (0,4 mol) wird in Acetonitril suspendiert, auf 10 °C gekühlt und bei dieser Temperatur mit Brom (0,39 mol) versetzt. Die Suspension wird noch 1 Stunde gerührt und dann mit Dicyclohexyl-propylpropandiol B3.7 (0,2 mol) versetzt. Man rührt über Nacht bei Raumtemperatur und erhitzt anschließend 4 Stunden zum Rückfluss. Nach Abkühlen auf Raumtemperatur wird mit Wasser versetzt und mit Petrolether extrahiert. Die organische Phase wird getrocknet und eingeengt, der Rückstand an Kieselgel filtriert. Nach dem Einengen wird Dicyclohexyl-propyl-propandibromid B3.8 erhalten. Ausbeute: 94%.

Das Dibromid B3.8 (0,402 mol) und Kalium-tert-butylat (0,503 mol) werden in Toluol gemischt und über Nacht bei 80°C gerührt. Man kühlt ab, versetzt mit Wasser und extrahiert mit Methyl-tert.-butylether. Die organische Phase wird getrocknet und eingeengt. Der Rückstand wird an Kieselgel filtriert. Nach dem Einengen erhält man das Allylbromid B3.9. Ausbeute: 90 %.

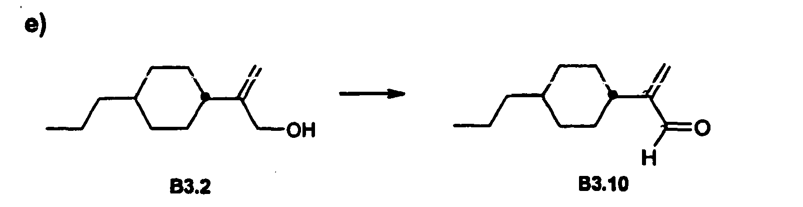

2-(4-trans-Propylcyclohexyl-(1))-allylalkohol (0,2 mol) werden mit 105 g aktivem Braunstein in 350 ml Dichlormethan 24 Stunden bei Raumtemperatur kräftig gerührt. Danach wird filtriert, mit Dichlormethan gewaschen, die organische Phase eingedampft und mit Toluol/Methyl-tert.-butylether über Kieselgel filtriert. Ausbeute des Acrolein B3.10: 64%.

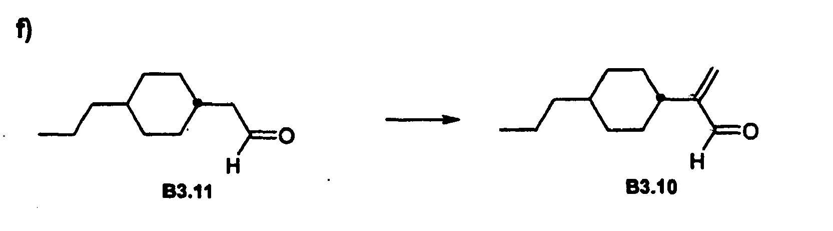

Der Aldehyd B3.11 (0,321 mol) und N,N-Dimethylmethyleniminiumchlorid (Eschenmosers Salz; 0,321 mol) werden in Dichlormethan suspendiert. Dann wird bei Raumtemperatur Triethylamin zugetropft und über Nacht gerührt. Man schüttelt mit Wasser aus, trennt die organische Phase ab, rotiert ein, nimmt in Heptan/Methyl-tert.-butylether auf und filtriert über Kieselgel. Ausbeute: 40%.

### Beispiel 4 - Synthese von Homoallylverbindungen der Formel V

### a) Allgemeine Arbeitsvorschrift B 4a (AAV B4a)

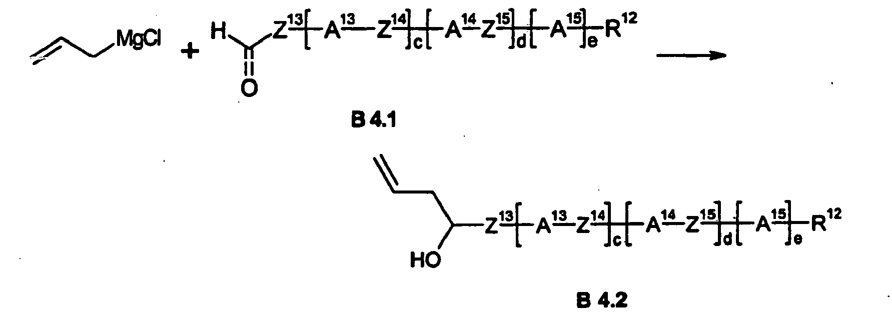

200 ml (2 mol) Allylmagnesiumchlorid in Diethylether (Aldrich Co.) werden unter schwacher Eiskühlung mit dem Aldehyd B 4.1, gelöst in 100 ml THF, tropfenweise versetzt und dann 4 Stunden bei Raumtemperatur gerührt. Die Redaktionsmischung wird anschließend in 100 ml 0,5 N HCl eingegossen und fünf Minuten gerührt. Die organische Phase wird abgetrennt, die wässrige Phase zweimal mit Methyl-tert.-butylether extrahiert. Die vereinigten organischen Extrakte werden mit Wasser nachgewaschen, getrocknet, filtriert und eingeengt. Das quantitativ erhaltene Rohprodukt kann aufgrund seiner Reinheit direkt in der nachfolgenden Umsetzung verwendet werden.

### b) Allgemeine Arbeitsvorschrift B 4b (AAV B 4b)

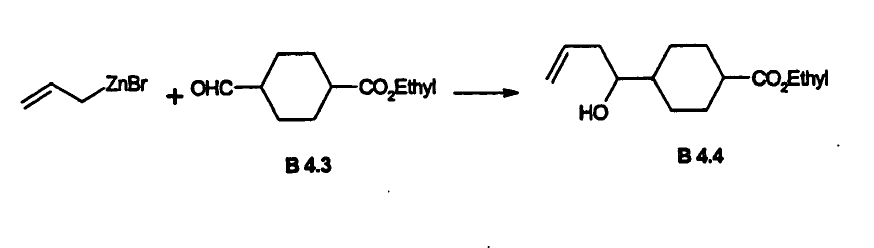

Allylmagnesiumchlorid (0,40 mol) in 400 ml Diethylether (Aldrich Co.) werden unter Außenkühlung zu einer Lösung von Zinkbromid (0,4 mol) in 200 ml THF getropft, so dass die Temperatur zwischen 10 bis 15°C liegt. Die entstandene Suspension wird bei dieser Temperatur noch 2 Stunden gerührt, bevor 0,4 mol des Aldehyds B 4.3, gelöst in 400 ml Diethylether, tropfenweise zugesetzt werden. Die Suspension wird weitere 16 Stunden bei Raumtemperatur gerührt und dann auf 600 ml 0,5N HCl gegossen. Die organische Phase wird isoliert und nach dem Trocknen mit Natriumsulfat eingeengt. Das Rohprodukt kann ohne weitere Reinigung eingesetzt werden. Anstelle von Zinkbromid kann zur Ummetallierung auch mit CITi(i-Propoxid)₃ verwendet werden.

Diese Reaktionsführung mit Ummetallierung eignet sich insbesondere für für die Umsetzung von Aldehyden, die neben der aldehydischen Carbonylfunktion eine gegenüber Grignard-Reagenzien reaktive funktionelle Gruppe wie eine Carbonsäureester oder Nitrilgruppe enthalten.

### Beispiel 5 - Synthese von Verbindungen der Formel 11

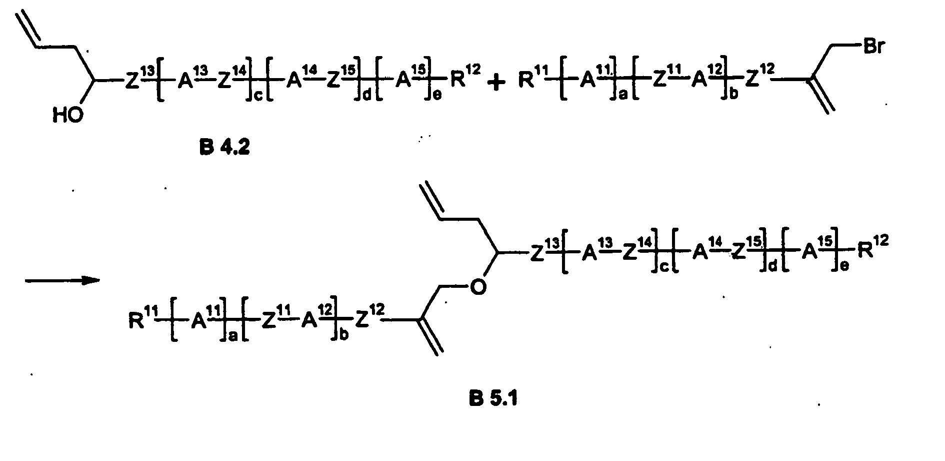

### a) Allgemeine Arbeitsvorschrift B 5a (AAV B5a)

### (Für Verbindungen B 4.2, für die Z¹³, Z¹⁴ und Z¹⁵ nicht CO₂ bedeuten)

0,4 mol granuliertes Natriumhydroxid werden bei Raumtemperatur in 200 ml THF suspendiert und mit 2 ml Wasser. 0.4 mol Homoallylalkohol B 4.2 und 0,02 mol N-Cetyl-N,N,N-trimethylammoniumbromid bei 25 °C unter Rühren versetzt. Darauf werden 0,4 mol des Allylbromids unter Rühren zugegeben. Das Gemisch wird 20 Stunden bei 40 bis 50 °C gerührt. Anschließend wird laut DC nicht umgesetzter Homoallylalkohol B4.2 mit weiteren 0,05 bis 0,1 mol Allylbromid versetzt, um die Reaktion nach fortgesetztem Rühren (12 Stunden) zu vervollständigen. Nach dem Abkühlen wird auf 500 ml Eiswasser gegossen und zweimal mit Methyl-tert.-butylether extrahiert. Die vereinigten organischen Extrakte werden nach dem Trocknen eingedampft und über Kieselgel mit Heptan/Toluol filtriert. Die Verbindungen B 5.1 werden dabei zumeist als Öle erhalten. Ausbeute: 70 bis 95%.

### b) Allgemeine Arbeitsvorschrift B 5b (AAV B5b)

0,46 mol NaH (60 %ige Suspension in Paraffinöl) werden in 200 ml THF vorgelegt. Hierzu wird der Homoallylalkohol B 4.2 (0,4 mol) in 200 ml THF bei einer Temperatur zwischen -10 und +20 °C zugetropft. Es wird solange zwischen 20 und 30 °C nachgerührt, bis die Wasserstoffentwicklung abgeschlossen ist. Dann werden 0,4 mol des Allylbromids in 200 ml THF bei Raumtemperatur zugetropft und je nach Ergebnis der DC-Kontrolle 12 bis 48 Stunden bei Raumtemperatur gerührt. Das Reaktionsgemisch wird auf Eiswasser gegossen, mit Methyl-tert.-butylether extrahiert, nach dem Trocknen und Eindampfen mit Heptan/Toluol über Kieselgel filtriert.

### c) Allgemeine Arbeitsvorschrift B 5c (AAV B5c)

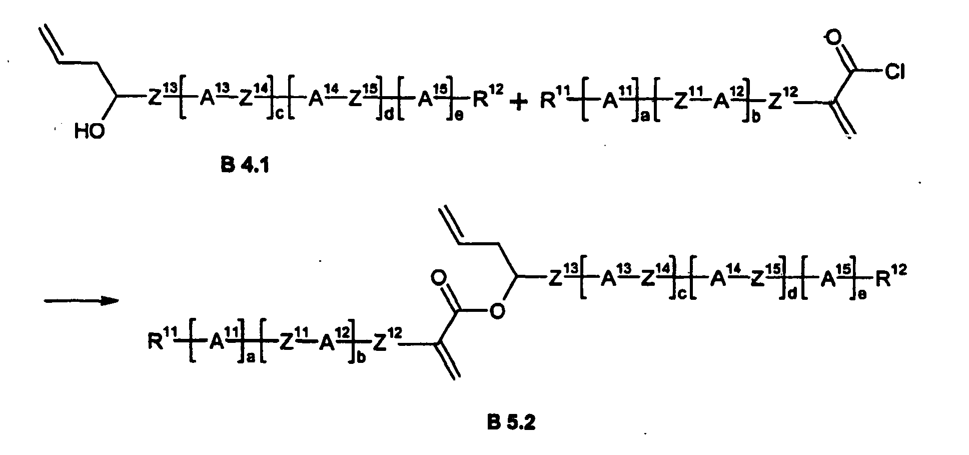

Die Herstellung von Arcylsäureestern B 5.2 erfolgt gemäß den Verfahren AAV B 5a oder AAV B 5b, wobei das Allylbromid durch das entsprechende Säurechlorid ersetzt wird.

### Beispiel 6 - Physikalische Parameter erfindungsgemäßer Verbindungen

In Tabelle B3 sind ausgewählte physikalische Parameter einiger erfindungsgemäßer Verbindungen wiedergegeben, die gemäß den Vorschriften der angehenden Beispiele hergestellt worden sind. (Die Angaben in der Spalte "Verbindung" beziehen sich auf die in den Beispielen 1 bis 5 wiedergegebenen Bezeichnungen.)

**Tabelle B3**

| **Verbindung** | **Δn** | **Δε** | **γ**_{**1**} **[mPa·s]** | **Fp. [°C]** | **Klp. [°C]** | **Phasenübergänge** |
|---|---|---|---|---|---|---|
| B1.2-1* | 0,0640 | 12,8 | 205 | 48 | 21*** | K 48 I |
| B1.2-2* | 0,0740 | 14,5 | 286 | 35 | 36*** | K 35 N (10,3) I |
| B1.2-3* | 0,1060 | 12,1 | 562 | 98 | 217 | K 98 N 2171 I |
| B1.2-4* | 0,0560 | 13.3 | 203 | 74 | 3*** | K 741 I |
| B1.2-7* | 0,0740 | 14,5 | 286 | 35 | 36*** | K 35 N (10,3) I |
| B1.2-8* | -0,0142 | 7,5 | | 62 | | K 62 I |
| B1.2-9* | 0,0520 | 14,8 | 99 | 89 | 25*** | K 89 I |
| B1.2-11* | 0,0760 | 8,2 | 137 | 84 | 63*** | K 84 SmB? 90 I |
| B1.2-12* | 0,0739 | 8,4 | | 79 | 37*** | K 79 SmB (74) I |
| B2.1-1* | 0,0710 | 12,2 | 182 | 57 | 62*** | K 57 N (50,9) I |
| B2.1-2* | 0,0770 | 13,5 | 325 | 69 | 68*** | K 69 N 70 I |
| B2.1-3* | 0,1020 | 11,8 | 582 | 113 | 256 | K 113 N256 I |
| B2.1-4* | 0,0599 | 14,1 | 136 | 71 | 32*** | K 71 I |
| B2.1-9* | 0,0490 | 34,9 | 142 | 54 | 16*** | K 54 I |
| B2.1-11* | 0,0810 | 8,5 | 149 | 37 | 106 | K 37 SmB 93 N 106 I |
| B2.1-13* | 0,0475 | 14,0 | 140 | 72 | 14*** | K 72 I |
| B2.1-14* | 0,1256 | 18,0 | 239 | 58 | 61 | K 58 SmA (37) N 61 I |
| B2.1-15* | 0,0570 | 13,4 | | 34 | 58 | K 34 N 58 I |
| B2.1-16* | 0,1493 | 27,3 | 287 | 77 | 91 | K 77 N 91 I |
| B2.1-17* | 0,0540 | 14,1 | 124 | 45 | 35*** | K 45 N (35) I |
| B2.1-18* | 0,1390 | 9,4 | | 84 | 232 | K 84 N 232 I |
| B2.1-19* | 0,1370 | 10,7 | | 47 | 238 | K 47 SmB 91 N 238 I |
| B2.1-20* | 0,1291 | 15,7 | 725 | 60 | 207 | K 60 SmB 81 N 207 I |
| B2.1-21* | 0,1071 | 17,3 | 176 | 64 | 13*** | K 64 I |
| B2.1-22* | 0,1310 | 29,9 | | 89 | 69*** | K 89 N (77) I |
| B2.1-23* | 0,1254 | 12,7 | 225 | 43 | 44 | K 43 N 44 I |
| B2.1-24* | 0,0568 | 11,8 | 216 | 39 | 75 | K 39 N 75 I |
| B2.1-25* | 0,1364 | 29,7 | | 70 | 102 | K 70 N 102 I |
| B2.1-26* | 0,0580 | 35,6 | 75 | 27 | -83*** | K 27 I |
| B2.1-27* | 0,1231 | 34,9 | 457 | 81 | 83 | K 81 N 83 I |
| B2.1-28* | 0,0623 | 11,5 | | | 106 | SmB 81 N 106 I |
| B2.1-29* | 0,0689 | 9,1 | | -54 | 117*** | K? -54 SmB 129 I |
| B2.1-30* | 0,1220 | 19,1 | 625 | 61 | 192 | K61 N 192 I |
| B2.1-31* | 0,1328 | 13,4 | 806 | 44 | 213 | K 44 Sm? 45 B 213 I |
| B2.1-32* | 0,1422 | 10,1 | 116 | 54 | 106*** | K 54 Sm? SmB 154 SmA 168 I |
| B2.1-33* | 0,1243 | 14,8 | 178 | 42 | 44*** | K 41 I |
| B2.1-34* | 0,1256 | 18,0 | 239 | 58 | 61 | K 58 SmA (37) N 61 I |
| B2.1-35* | 0,0599 | 14,1 | 136 | 71 | 32*** | K 71 I |
| B2.1-36* | 0,0570 | 13,4 | 160 | 35 | 66 | K 35 N 66 I |
| B2.1-37* | 0,1070 | 29,7 | 213 | 89 | 48*** | K 89 I |
| B2.1-38* | 0,1493 | 27,3 | 287 | 78 | 92 | K 78 N 92 I |
| B2.1-39* | 0,1159 | 14,5 | 130 | 52 | 17*** | K 52 I |
| B2.1-40* | 0,1243 | 14,8 | 178 | 41 | 44*** | K 41 I |
| B2.1-41* | 0,0475 | 14,0 | 140 | 72 | 14*** | K 72 I |
| B2.1-42* | 0,1159 | 14,5 | 130 | 52 | 17*** | K 52 I |
| B2.1-43* | 0,1430 | 9,6 | 189 | 50 | 99 | K 50 SmA-1 (32) SmA-2 64 N99 I |
| B2.1-44* | 0,1380 | 12,7 | 236 | 45 | 90*** | K 45 SmA-1 68 SmA-2 118 I |
| B2.1-45* | 0,0780 | 9,1 | 178 | -41 | 129 | K-41 SmB 123 N 129 I |
| B2.1-46* | 0,0701 | 11,7 | 199 | | 106 | SmB 74 N 106 |
| B2.1-47* | 0,0688 | 9,7 | 187 | 41 | 96 | K 41 SmB 51 N 96 I |
| B2.1-48* | 0,1384 | 19,7 | | 74 | 198 | K 74 N 198 I |
| B2.1-49* | 0,1157 | 12,3 | | 28 | 25*** | K 28 N (11) I |
| B2.1-50* | 0,1100 | 15,2 | 177 | 37 | 39 | K 37 SmA-(35) N 39 I |
| B2.1-52* | 0,1450 | 23,2 | 300 | 75 | 118 | K 75 N 118 I |
| B2.1-53** | 0,1190 | 40,8 | 529 | 91 | 47*** | K 91 I |
| B2.1-54* | 0,0800 | 28,2 | 128 | 32 | -50*** | K 32 I |
| B2.1-57* | 0,0550 | 23,7 | 241 | 87 | 20*** | K 87 I |
| B2.1-58* | 0,1158 | 35,6 | 541 | 74 | 101 | K 74 N 101 I |
| B2.1-59* | 0,1330 | 25,3 | 466 | 80 | 120 | K 80 N 120 I |
| B2.1-60* | 0,1230 | 61,4 | 625 | 79 | 91 | K 79 N 91 I |
| B2.1-61* | 0,1706 | 35,4 | | 115 | 198 | K 115 N 198 I |
| B2.1-62* | 0,0950 | 18,9 | 160 | 58 | -30*** | K 58 I |
| B2.1-63* | 0,0871 | 21,7 | | 106 | 207 | K 106 N 2071 I |
| B2.1-64** | 0,1649 | 37,9 | | 98 | 193 | K 98 N 193 I |
| B2.1-65* | 0,0049 | 7,7 | 76 | 7 | -78*** | K7 SmB 15 I |
| B2.1-66* | 0,0880 | 21,3 | | 91 | 203 | K 91 SmH (63) N 203 I |
| B2.1-67** | 0,1510 | 42,1 | | 144 | 181 | K 144 N 181 I |
| B2.1-68* | 0,1180 | 29,8 | 541 | 48 | 105 | K 48 SmA (46) N 105 I |
| B2.1-69* | 0,1279 | 57,4 | | 50 | 95 | K 50 N 95 I |
| B2.1-70* | 0,1149 | 35,0 | 356 | 91 | 38*** | K 91 N (59) I |
| B2.1-71* | 0,1202 | 64,0 | 536 | 77 | 38*** | K 77 N (66) I |
| B2.1-72* | 0,1270 | 58,0 | 534 | 76 | 90 | K 76 N 90 I |
| B2.1-73** | 0,1634 | 37,7 | | 121 | 205 | K 121 N 205 I |
| B2.1-74** | 0,1411 | 43,8 | | 128 | 201 | K 128 N 201 I |
| B2.1-75** | 0,1431 | 29,8 | | 113 | 149 | K 113 N 149 I |
| B2.1-76* | 0,1459 | 67,8 | | 98 | 128 | K 98 N 128 I |
| B2.1-77* | 0,1490 | 61,8 | | 65 | 142 | K 65 SmC (44) N 142 I |
| B2.1-78* | 0,1477 | 64,2 | | 79 | 139 | K 79 N 139 I |
| B2.1-79* | 0,1459 | 58,5 | | 64 | 131 | K 64 N 131 I |
| B2.1-80* | 0,1171 | 32,4 | 381 | 90 | 54*** | K 90 N (81) I |
| B2.1-81* | 0,1205 | 31,9 | 589 | 82 | 89 | K 82 N 89 I |
| B2.1-82* | 0,1130 | 30,3 | 662 | 83 | 86 | K 83 N 86 I |
| B2.1-83* | 0,0459 | 14,5 | 126 | 72 | -19*** | K 72 I |
| B2.1-84* | 0,0960 | 16,4 | 185 | 55 | 14*** | K 55 I |
| 82.1-85* | 0,2052 | 31,4 | | 107 | 186 | K 107 N 186 I |
| B2.1-86* | 0,1372 | 31,5 | 607 | 80 | 107 | K 80 N 1071 I |
| B2.1-87* | 0,1116 | 30,6 | 473 | 89 | 59*** | K 89 N (83) I |
| B2.1-88* | 0,2125 | 59,4 | | 121 | 269 | K 121 N 269 |

| | | | | | | |
|---|---|---|---|---|---|---|
| *Die Bestimmung der Parameter Δn, Δε und γ₁ erfolgte durch Messung einer Mischung von 10 Gew.% der Verbindung in dem Host ZLI-4792 (Merck KGaA, Darmstadt) und nachfolgende Extrapolation. ** Die Bestimmung der Parameter Δn, Δε und γ₁ erfolgte mit 5 Gew.% der Verbindung in ZLI-4792. *** Die Bestimmung des Klärpunkts erfolgte durch Messung einer Mischung von 10 beziehungsweise 5 Gew.% der Verbindung in dem Host ZLI-4792 und nachfolgende Extrapolation. | | | | | | |

## Patentansprüche

1. Verbindung der allgemeinen Formel I 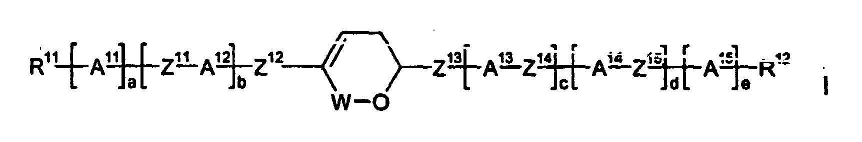 wobei
a, b, c, d und e jeweils unabhängig voneinander 0 oder 1 sind;
W -CH₂- oder -C(=O)- bedeutet;
R¹¹ H, einen unsubstituierten oder einfach oder mehrfach gleich oder verschieden mit Halogen oder -CN substituierten Alkylrest mit 1 bis 15 Kohlenstoffatomen bedeutet, wobei in diesem Rest auch eine oder mehrere CH₂-Gruppen durch -C≡C-, -CH=CH-, -O-, -S-, -C(O)-O- und/oder -O-C(O)- so ersetzt sein können, dass Heteroatome (O, S) nicht direkt miteinander verknüpft sind;
R¹² H, Halogen, -CN, -NCS, Aralkyl, -O-Aralkyl oder einen unsubstituierten oder einfach oder mehrfach gleich oder verschieden mit Halogen oder -CN substituierten Alkylrest mit 1 bis 15 Kohlenstoffatomen bedeutet, wobei in diesem Rest auch eine oder mehrere CH₂-Gruppen durch -C≡C-, -CH=CH-, -O-, -S-, -C(O)-O- und/oder-O-C(O)- so ersetzt sein können, dass Heteroatome (O, S) nicht direkt miteinander verknüpft sind;
Z¹¹ eine Einfachbindung, -CH₂-, -CH₂CH₂-, -CF₂CH₂, -CH₂CF₂-, -CF₂CF₂-, -CH=CH- oder -C≡C- bedeutet;
Z¹² eine Einfachbindung, -CH₂-, -CH₂CH₂-, -CF₂CH₂-, -CH₂CF₂- oder -CF₂CF₂- bedeutet;
Z¹³, Z¹⁴ und Z¹⁵ jeweils unabhängig voneinander eine Einfachbindung, -CH₂CH₂-, -CF₂CH₂-, -CH₂CF₂-, -CF₂CF₂-, -CH=CH-, -C≡C-, -CH₂O-, -CF₂O-, -C(O)- oder -C(O)-O- bedeuten;
A¹¹ und A¹² unabhängig voneinander für 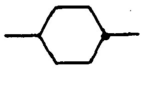 oder 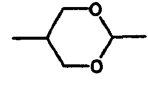 stehen;
A¹³ und A¹⁴ unabhängig voneinander für 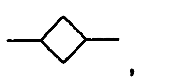 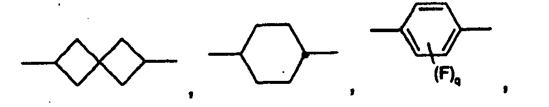 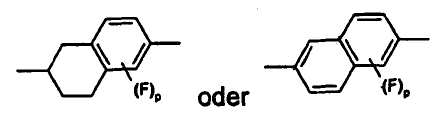 stehen;
A¹⁵ für 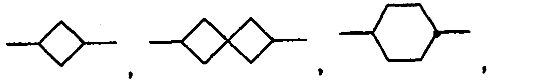 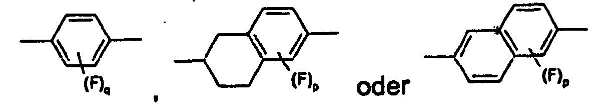 steht oder
A¹⁵-R¹² zusammen für 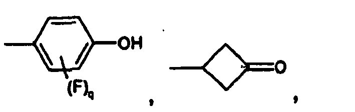 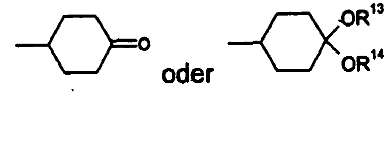 steht;
oder
Z¹³-[-A¹³-Z¹⁴-]_{c}-[-A¹⁴-Z¹⁵-]_{d}-[-A¹⁵-]ₑ-R¹² für 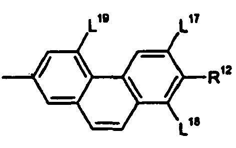 oder 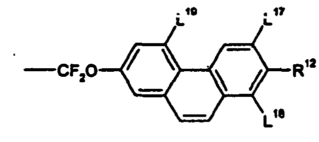 steht, wobei R¹² wie oben definiert ist und L¹⁷, L¹⁸ und L¹⁹ unabhängig voneinander H oder F bedeuten;
q 0, 1, 2, 3 oder 4 ist;
p 0, 1, 2 oder 3 ist;
R¹³ und R¹⁴ unabhängig voneinander einen Alkanylrest mit 1 bis 7 Kohlenstoffatomen bedeuten oder zusammen für eine Alkylenbrücke mit 2 bis 7 Kohlenstoffatomen stehen;
mit der Maßgabe,
dass für den Fall der direkten Verknüpfung von Z¹³ und R¹² zu -Z¹³-R¹² R¹² H, Aralkyl, Alkanyl oder Alkenyl bedeutet, wenn Z¹³ -C(=O)-O- oder -C(=O)- bedeutet, und Z¹³ nicht -CH₂O- oder -CF₂O-bedeutet;
dass für den Fall der direkten Verknüpfung von Z¹⁴ und R¹² zu -Z¹⁴-R¹² R¹² H, Aralkyl, Alkanyl oder Alkenyl bedeutet, wenn Z¹⁴ -C(=O)-O- oder -C(=O)- bedeutet, und Z¹⁴ nicht -CH₂O- oder -CF₂O-bedeutet;
dass für den Fall der direkten Verknüpfung von Z¹⁵ und R¹² zu -Z¹⁵-R¹² R¹² H, Aralkyl, Alkanyl oder Alkenyl bedeutet, wenn Z¹⁵ -C(=O)-O- oder -C(=O)- bedeutet, und Z¹⁵ nicht -CH₂O- oder -CF₂O-bedeutet;
dass R¹¹ nicht H bedeutet, wenn zugleich a und b beide null sind und Z¹² eine Einfachbindung bedeutet;
dass R¹¹ nicht CH₃ bedeutet, wenn zugleich a und b beide null sind, Z¹² und Z¹³ jeweils eine Einfachbindung bedeuten, W -CH₂- bedeutet und -[A¹³-Z¹⁴-]_{c}-[-A¹⁴-Z¹⁵-]_{d}-[-A¹⁵]ₑ-R¹² für unsubstituiertes Phenyl steht.

2. Verbindung gemäß Anspruch 1, **dadurch gekennzeichnet, dass** a+b+c+d+e ≤ 3.

3. Verbindung gemäß wenigstens einem der vorangehenden Ansprüche,
**dadurch gekennzeichnet, dass**
W -C(=O)- bedeutet.

4. Verbindung gemäß wenigstens einem der Ansprüche 1 oder 2,
**dadurch gekennzeichnet, dass**
W -CH₂- bedeutet.

5. Verbindung gemäß wenigstens einem der vorangehenden Ansprüche,
**dadurch gekennzeichnet, dass**
Z¹² eine Einfachbindung bedeutet.

6. Verbindung gemäß wenigstens einem der vorangehenden Ansprüche,
**dadurch gekennzeichnet, dass**
b 1 ist;
Z¹¹ eine Einfachbindung bedeutet; und
A¹² für 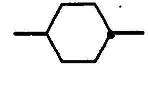 steht.

7. Verbindung gemäß wenigstens einem der vorangehenden Ansprüche,
**dadurch gekennzeichnet, dass**
a 1 ist; und
A¹¹ für 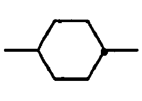 steht.

8. Verbindung gemäß wenigstens einem der vorangehenden Ansprüche,
**dadurch gekennzeichnet, dass**
Z¹³ eine Einfachbindung, -C(O)-O- oder -CF₂O- bedeutet.

9. Verbindung gemäß wenigstens einem der vorangehenden Ansprüche,
**dadurch gekennzeichnet, dass**
e 1 ist;
A¹⁵ für 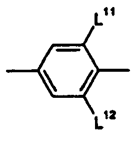 steht; und
L¹¹ und L¹² unabhängig voneinander H oder F bedeuten.

10. Verbindung gemäß wenigstens einem der Ansprüche 1 bis 8, **dadurch**
**gekennzeichnet, dass**
e 1 ist;
A¹⁵-R¹² für 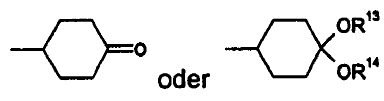 steht; und
R¹³ und R¹⁴ wie in Anspruch 1 definiert sind.

11. Verbindung gemäß wenigstens einem der vorangehenden Ansprüche,
**dadurch gekennzeichnet, dass**
c 1 ist;
Z¹⁴ eine Einfachbindung, -C(O)-O- oder -CF₂O- bedeutet;
A¹³ für 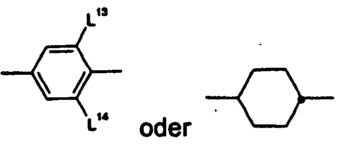 steht; und
L¹³ und L¹⁴ unabhängig voneinander für H oder F stehen.

12. Verbindung gemäß wenigstens einem der vorangehenden Ansprüche,
**dadurch gekennzeichnet, dass**
d 1 ist;
Z¹⁵ -CF₂O- bedeutet:
A¹⁴ für 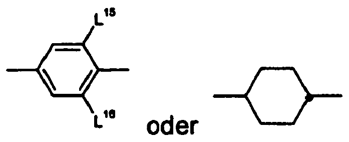 steht; und
L¹⁵ und L¹⁶ unabhängig voneinander H oder F bedeuten.

13. Verfahren zur Herstellung von Pyranderivaten umfassend einen Verfahrensschritt, der eine ringschließende Metathese-Reaktion einer Verbindung der allgemeinen Formel II 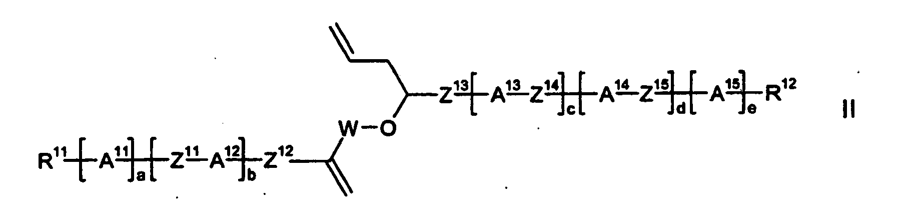 zu einem Pyranderivat der Formel I 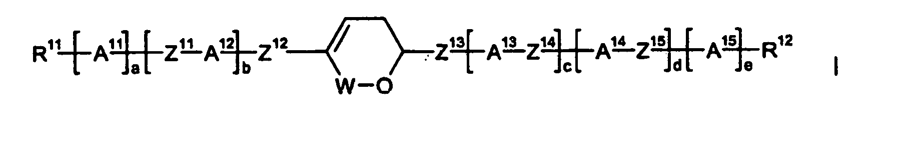 wobei a, b, c, d, e, W, R¹¹, R¹², Z¹¹, Z¹², Z¹³, Z¹⁴, Z¹⁵, A¹¹, A¹², A¹³, A¹⁴ und A¹⁵ in den Formeln und II die gleiche Bedeutung wie in Anspruch 1 für Formel I haben;
mit der Maßgabe,
dass für den Fall der direkten Verknüpfung von Z¹³ und R¹² zu -Z¹³-R¹² R¹² H, Aralkyl oder Alkanyl bedeutet, wenn Z¹³ -C(=O)-bedeutet, R¹² Aralkyl oder Alkanyl bedeutet, wenn Z¹³ -C(=O)-O-bedeutet, und Z¹³ nicht -CH₂O- oder-CF₂O- bedeutet; dass für den Fall der direkten Verknüpfung von Z¹⁴ und R¹² zu -Z¹⁴-R¹² R¹² H, Aralkyl oder Alkanyl bedeutet, wenn Z¹⁴ -C(=O)-bedeutet, R¹² Aralkyl oder Alkanyl bedeutet, wenn Z¹⁴ -C(=O)-O-bedeutet, und Z¹⁴ nicht -CH₂O- oder -CF₂O- bedeutet; dass für den Fall der direkten Verknüpfung von Z¹⁵ und R¹² zu -Z¹⁵-R¹² R¹² H, Aralkyl oder Alkanyl bedeutet, wenn Z¹⁵ -C(=O)-bedeutet, R¹² Aralkyl oder Alkanyl bedeutet, wenn Z¹⁵ -C(=O)-O-bedeutet, und Z¹⁵ nicht -CH₂O- oder -CF₂O- bedeutet; dass R¹¹ nicht H bedeutet, wenn a und b beide zugleich null sind und Z¹² eine Einfachbindung bedeutet;
dass R¹¹ nicht CH₃ bedeutet, wenn zugleich a und b beide null sind, Z¹² und Z¹³ jeweils eine Einfachbindung bedeuten, W -CH₂- bedeutet und -[-A¹³-Z¹⁴-]_{c}-[-A¹⁴-Z¹⁵-]_{d}-[-A¹⁵-]ₑ-R¹² für unsubstituiertes Phenyl steht;
in Gegenwart eines Metathese-Katalysators umfaßt.

14. Verfahren gemäß Anspruch 13, **dadurch gekennzeichnet, dass** der Metathese-Katalysator ein Ruthenium-Alkyliden-Komplex ist.

15. Verfahren gemäß wenigstens einem der Ansprüche 13 und 14 umfassend nach dem Metathese-Reaktionsschritt als einen weiteren Reaktionsschritt eine Reduktionsreaktion zur Überführung des Pyranderivats der Formel I, worin W -C(=O)- bedeutet und Z¹³, Z¹⁴ und Z¹⁵ nicht -C(O)- bedeuten, in ein Pyranderivat der Formel I, worin W -CH₂- bedeutet;
R¹¹ H, einen unsubstituierten oder einfach oder mehrfach gleich oder verschieden mit Halogen oder -CN substituierten Alkylrest mit 1 bis 15 Kohlenstoffatomen bedeutet, wobei in diesem Rest auch eine oder mehrere CH₂-Gruppen durch -C≡C-, -CH=CH-, -O- und/oder -S- so ersetzt sein können, dass Heteroatome (O, S) nicht direkt miteinander verknüpft sind;
R¹² H, Halogen, -CN, -NCS, Aralkyl, -O-Aralkyl oder einen unsubstituierten oder einfach oder mehrfach gleich oder verschieden mit Halogen oder -CN substituierten Alkylrest mit 1 bis 15 Kohlenstoffatomen bedeutet, wobei in diesem Rest auch eine oder mehrere CH₂-Gruppen durch -C≡C-, -CH=CH-, -O- und/oder -S- so ersetzt sein können, dass Heteroatome (O, S) nicht direkt miteinander verknüpft sind;
Z¹³, Z¹⁴ und Z¹⁵ jeweils unabhängig voneinander eine Einfachbindung, -CH₂CH₂-, -CF₂CH₂-, -CH₂CF₂-, -CF₂CF₂-, -CH=CH-, -C≡C, -CH₂O- oder -CF₂O- bedeuten;
und a, b, c, d, e, Z¹¹, Z¹², A¹¹, A¹², A¹³, A¹⁴ und A¹⁵ wie in Anspruch 1 definiert sind.

16. Verfahren gemäß wenigstens einem der Ansprüche 13 bis 15 umfassend nach dem Metathese-Reaktionsschritt und nach dem gegebenenfalls durchzuführenden Reduktionsreaktionsschritt als einen weiteren Reaktionsschritt eine katalytische Hydrierung zu einem Pyranderivat der allgemeinen Formel III: 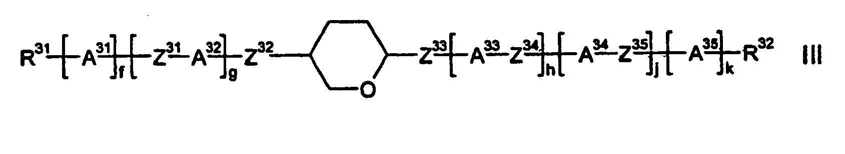 worin
f, g, h, j und k jeweils unabhängig voneinander 0 oder 1 sind;
R³¹ H, einen unsubstituierten oder einfach oder mehrfach gleich oder verschieden mit Halogen oder -CN substituierten Alkylrest mit 1 bis 15 Kohlenstoffatomen bedeutet, wobei in diesem Rest auch eine oder mehrere CH₂-Gruppen durch -O-, -S-, -C(O)-O- und/oder -O-C(O)- so ersetzt sein können, dass Heteroatome (O, S) nicht direkt miteinander verknüpft sind;
R³² H, Halogen, -CN, -NCS, Aralkyl oder einen unsubstituierten oder einfach oder mehrfach gleich oder verschieden mit Halogen oder -CN substituierten Alkylrest mit 1 bis 15 Kohlenstoffatomen bedeutet, wobei in diesem Rest auch eine oder mehrere CH₂-Gruppen durch -O-, -S-, -C(O)-O- und/oder -O-C(O)- so ersetzt sein können, dass Heteroatome (O, S) nicht direkt miteinander verknüpft sind;
Z³¹ und Z³² unabhängig voneinander eine Einfachbindung, -CH₂-, -CH₂CH₂-, -CF₂CH₂-, -CH₂CF₂- oder -CF₂CF₂- bedeuten;
Z³³, Z³⁴ und Z³⁵ jeweils unabhängig voneinander eine Einfachbindung, -CH₂CH₂-, CF₂CH₂-, -CH₂CF₂-, -CF₂CF₂-, -CH₂O-, -CF₂O-, -C(O)- oder -C(O)-O- bedeuten;
A³¹ und A³² unabhängig voneinander für 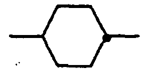 oder 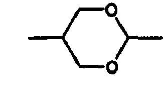 stehen;
A³³ und A³⁴ unabhängig voneinander für 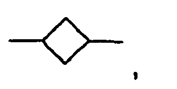 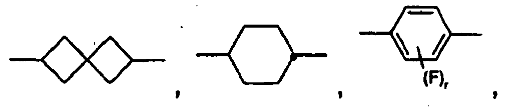 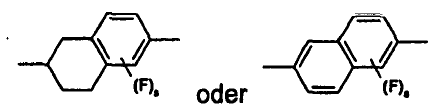 stehen;
A³⁵ unabhängig voneinander für 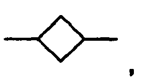 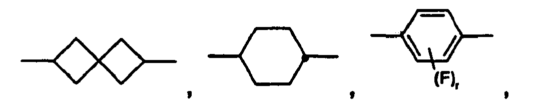 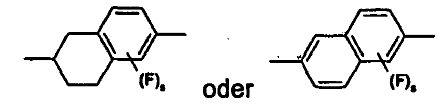 steht oder
A³⁵-R³² zusammen für 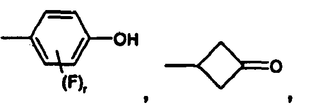 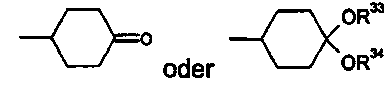 steht;
oder
Z³³-[-A³³-Z³⁴-]ₕ-[-A³⁴-Z³⁵-]ⱼ-[-A³⁵-]ₖ-R³² für 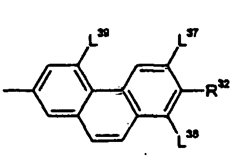 oder 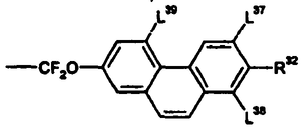 steht, wobei R³² wie oben definiert ist und L³⁷, L³⁸ und L³⁹ unabhängig voneinander H oder F bedeuten;
r 0, 1, 2, 3 oder 4 ist;
s 0, 1, 2 oder 3 ist;
R³³ und R³⁴ unabhängig voneinander einen Alkanylrest mit 1 bis 7 Kohlenstoffatomen bedeutet oder zusammen für eine Alkylenbrücke mit 2 bis 7 Kohlenstoffatomen stehen;
mit der Maßgabe,
dass für den Fall der direkten Verknüpfung von Z³³ und R³² zu -Z³³-R³² R³² H, Aralkyl oder Alkanyl bedeutet, wenn Z³³ -C(=O)-bedeutet, R³² Aralkyl oder Alkanyl bedeutet, wenn Z³³ -C(=O)-O-bedeutet, und Z³³ nicht -CH₂O- oder -CF₂O- bedeutet; dass für den Fall der direkten Verknüpfung von Z³⁴ und R³² zu -Z³⁴-R³² R³² H, Aralkyl oder Alkanyl bedeutet, wenn Z³⁴ -C(=O)-bedeutet, R³² Aralkyl oder Alkanyl bedeutet, wenn Z³⁴ -C(=O)-O-bedeutet, und Z³⁴ nicht -CH₂O- oder -CF₂O- bedeutet; dass für den Fall der direkten Verknüpfung von Z³⁵ und R³² zu -Z³⁵-R³² R³² H, Aralkyl oder Alkanyl bedeutet, wenn Z³⁵ -C(=O)-bedeutet, R³² Aralkyl oder Alkanyl bedeutet, wenn Z³⁵ -C(=O)-O-bedeutet, und Z³⁵ nicht -CH₂O- oder -CF₂O- bedeutet;
dass R³¹ nicht H bedeutet, wenn zugleich f und g beide null sind und Z³² eine Einfachbindung bedeutet.

17. Verfahren gemäß wenigstens einem der Ansprüche 13 bis 16 umfassend vor dem Metathese-Reaktionsschritt als einen weiteren Reaktionsschritt die Umsetzung einer Verbindung der Formel IV mit einer Homoallylverbindung der Formel V 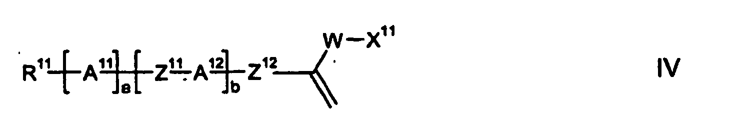 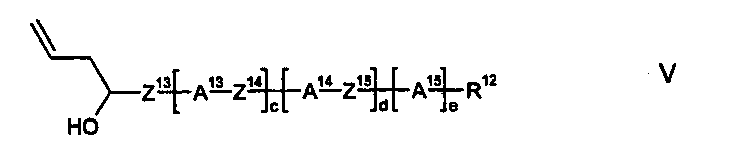 unter Bildung der Verbindung der Formel II 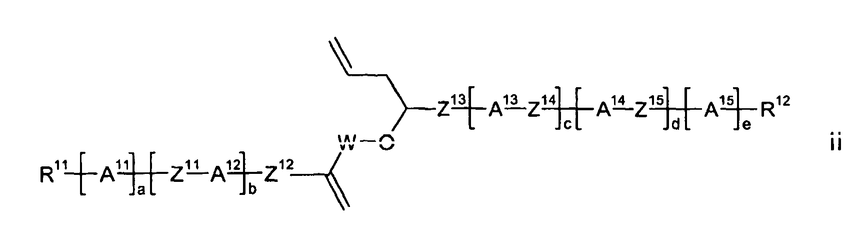 wobei X¹¹ eine Abgangsgruppe bedeutet und a, b, c, d, e, W, R¹¹, R¹², Z¹¹, Z¹², Z¹³, Z¹⁴, Z¹⁵, A¹¹, A¹², A¹³, A¹⁴ und A¹⁵ in den Formeln II, IV und V die gleiche Bedeutung wie in Anspruch 1 für Formel I haben.

18. Verfahren gemäß Anspruch 17, **dadurch gekennzeichnet, dass**
W -CH₂- bedeutet; und
X¹¹ Chlor, Brom, Iod oder einen Sulfonsäurerest bedeutet.

19. Allylverbindung der Formel IV-Bb 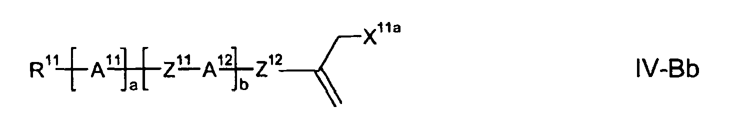 wobei
a null und b 1 oder a 1 und b null oder a und b beide 1 sind;
X^{11a} Chlor, Brom, Iod, einen Sulfonsäurerest, -OH, Alkoxy oder O-Aralkyl bedeutet
oder
-X^{11a} für =O steht, im Sinn von Aldehyden;
R¹¹, Z¹¹ und Z¹² wie in Anspruch 1 definiert sind; und
A¹¹ und A¹² wie in Anspruch 1 definiert sind und bevorzugt 1,4-Cyclohexylen bedeuten.

20. Verwendung eines Pyranderivats der Formel I gemäß Anspruch 1 als Bestandteil eines flüssigkristallinen Mediums insbesondere in einer elektrooptischen Anzeigevorrichtung.

## Claims

1. Compound of the general formula I 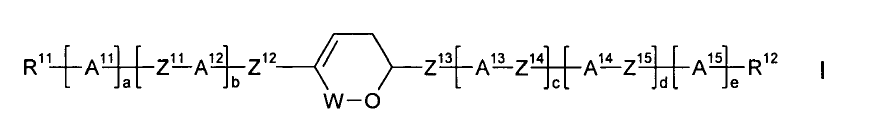 where
a, b, c, d and e are each, independently of one another, 0 or 1;
W denotes -CH₂- or -C(=O)-;
R¹¹ denotes H, an alkyl radical having 1 to 15 carbon atoms which is unsubstituted or mono- or polysubstituted, identically or differently, by halogen or -CN, where, in addition, one or more CH₂ groups in this radical may be replaced by -C≡C-, -CH=CH-, -O-, -S-, -C(O)-O- and/or -O-C(O)- in such a way that heteroatoms (O, S) are not linked directly to one another;
R¹² denotes H, halogen, -CN, -NCS, aralkyl, -O-aralkyl or an alkyl radical having 1 to 15 carbon atoms which is unsubstituted or mono- or polysubstituted, identically or differently, by halogen or -CN, where, in addition, one or more CH₂ groups in this radical may be replaced by -C≡C-, -CH=CH-, -O-, -S-, -C(O)-O- and/or -O-C(O)- in such a way that heteroatoms (O, S) are not linked directly to one another;
Z¹¹ denotes a single bond, -CH₂-, -CH₂CH₂-, -CF₂CH₂-, -CH₂CF₂-, -CF₂CF₂-, -CH=CH- or -C≡C-;
Z¹² denotes a single bond, -CH₂-, -CH₂CH₂-, -CF₂CH₂-, -CH₂CF₂- or -CF₂CF₂-;
Z¹³, Z¹⁴ and Z¹⁵ each, independently of one another, denote a single bond, -CH₂CH₂-, -CF₂CH₂-, -CH₂CF₂-, -CF₂CF₂-, -CH=CH-, -C≡C-, -CH₂O-, -CF₂O-, -C(O)- or -C(O)-O-;
A¹¹ and A¹², independently of one another, stand for 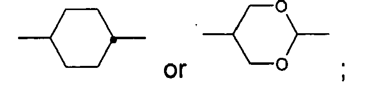
A¹³ and A¹⁴, independently of one another, stand for 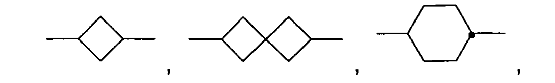 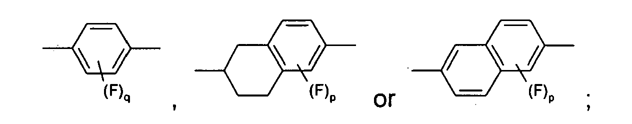
A¹⁵ stands for 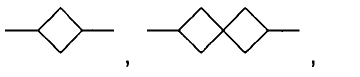 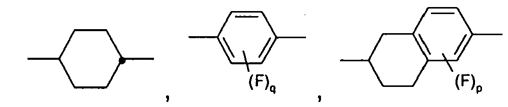 or 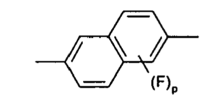 or
A¹⁵-R¹² together stand for 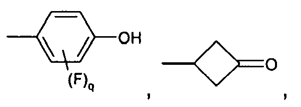 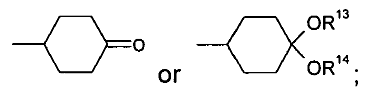 or
Z¹³-[-A¹³-Z¹⁴-]_{c}-[-A¹⁴-Z¹⁵-]_{d}-[-A¹⁵-]ₑ-R¹² stands for 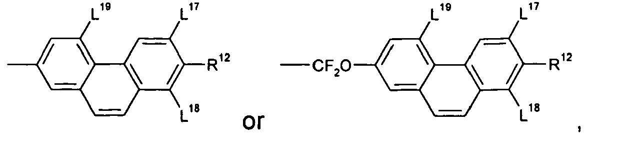 where R¹² is as defined above, and L¹⁷, L¹⁸ and L¹⁹, independently of one another, denote H or F;
q is 0, 1, 2, 3 or 4;
p is 0, 1, 2 or 3;
R¹³ and R¹⁴, independently of one another, denote an alkanyl radical having 1 to 7 carbon atoms or together stand for an alkylene bridge having 2 to 7 carbon atoms;
with the proviso
that, in the case of direct linking of Z¹³ and R¹² to give -Z¹³-R¹², R¹² denotes H, aralkyl, alkanyl or alkenyl if Z¹³ denotes -C(=O)-O- or -C(=O)-, and Z¹³ does not denote -CH₂O- or -CF₂O-;
that, in the case of direct linking of Z¹⁴ and R¹² to give -Z¹⁴-R¹², R¹² denotes H, aralkyl, alkanyl or alkenyl if Z¹⁴ denotes -C(=O)-O- or -C(=O)-, and Z¹⁴ does not denote -CH₂O- or -CF₂O-;
that, in the case of direct linking of Z¹⁵ and R¹² to give -Z¹⁵-R¹², R¹² denotes H, aralkyl, alkanyl or alkenyl if Z¹⁵ denotes -C(=O)-O- or -C(=O)-, and Z¹⁵ does not denote -CH₂O- or -CF₂O-;
that R¹¹ does not denote H if simultaneously a and b are both zero and Z¹² denotes a single bond;
that R¹¹ does not denote CH₃ if simultaneously a and b are both zero, Z¹² and Z¹³ each denote a single bond, W denotes -CH₂- and -[-A¹³-Z¹⁴-]_{c}-[-A¹⁴-Z¹⁵-]_{d}-[-A¹⁵-]ₑ-R¹² stands for unsubstituted phenyl.

2. Compound according to Claim 1, **characterised in that** a+b+c+d+e ≤ 3.

3. Compound according to at least one of the preceding claims, **characterised in that**
W denotes -C(=O)-.

4. Compound according to at least one of Claims 1 and 2, **characterised in that**
W denotes -CH₂-.

5. Compound according to at least one of the preceding claims, **characterised in that**
Z¹² denotes a single bond.

6. Compound according to at least one of the preceding claims, **characterised in that**
b is 1;
Z¹¹ denotes a single bond; and
A¹² stands for

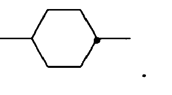

7. Compound according to at least one of the preceding claims, **characterised in that**
a is 1; and
A¹¹ stands for

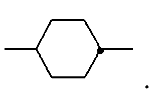

8. Compound according to at least one of the preceding claims, **characterised in that**
Z¹³ denotes a single bond, -C(O)-O- or -CF₂O-.

9. Compound according to at least one of the preceding claims, **characterised in that**
e is 1;
A¹⁵ stands for 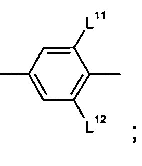 and
L¹¹ and L¹², independently of one another, denote H or F.

10. Compound according to at least one of Claims 1 to 8, **characterised in that**
e is 1;
A¹⁵-R¹² stands for 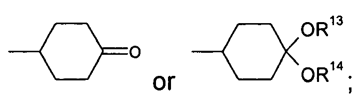 and
R¹³ and R¹⁴ are as defined in Claim 1.

11. Compound according to at least one of the preceding claims, **characterised in that**
c is 1;
Z¹⁴ denotes a single bond, -C(O)-O- or -CF₂O-;
A¹³ stands for 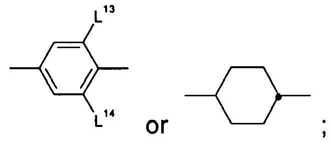 and
L¹³ and L¹⁴, independently of one another, stand for H or F.

12. Compound according to at least one of the preceding claims, **characterised in that**
d is 1;
Z¹⁵ denotes -CF₂O-;
A¹⁴ stands for 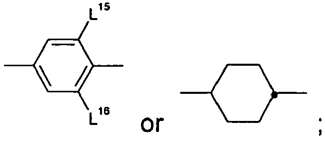 and
L¹⁵ and L¹⁶, independently of one another, denote H or F.

13. Process for the preparation of pyran derivatives including a process step which includes a ring-closing metathesis reaction of a compound of the general formula II 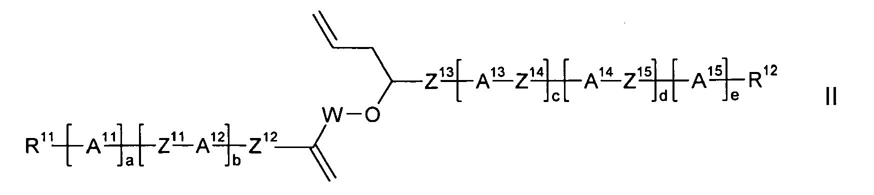 to give a pyran derivative of the formula I 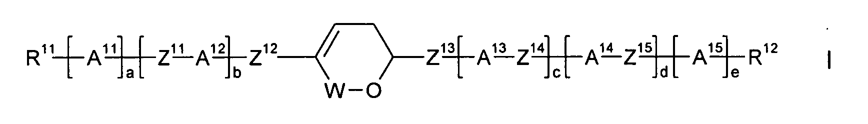 where a, b, c, d, e, W, R¹¹, R¹², Z¹¹, Z¹², Z¹³, Z¹⁴, Z¹⁵, A¹¹, A¹², A¹³, A¹⁴ and A¹⁵ in the formulae I and II have the same meaning as in Claim 1 for formula I;
with the proviso
that, in the case of direct linking of Z¹³ and R¹² to give -Z¹³-R¹², R¹² denotes H, aralkyl or alkanyl if Z¹³ denotes -C(=O)-, R¹² denotes aralkyl or alkanyl if Z¹³ denotes -C(=O)-O-, and Z¹³ does not denote -CH₂O- or -CF₂O-;
that, in the case of direct linking of Z¹⁴ and R¹² to give -Z¹⁴-R¹², R¹² denotes H, aralkyl or alkanyl if Z¹⁴ denotes -C(=O)-, R¹² denotes aralkyl or alkanyl if Z¹⁴ denotes -C(=O)-O-, and Z¹⁴ does not denote -CH₂O- or -CF₂O-;
that, in the case of direct linking of Z¹⁵ and R¹² to give -Z¹⁵-R¹², R¹² denotes H, aralkyl or alkanyl if Z¹⁵ denotes -C(=O)-, R¹² denotes aralkyl or alkanyl if Z¹⁵ denotes -C(=O)-O-, and Z¹⁵ does not denote -CH₂O- or -CF₂O-;
that R¹¹ does not denote H if a and b are both simultaneously zero and Z¹² denotes a single bond;
that R¹¹ does not denote CH₃ if simultaneously a and b are both zero, Z¹² and Z¹³ each denote a single bond, W denotes -CH₂- and -[-A¹³-Z¹⁴]_{c}-[-A¹⁴-Z¹⁵-]_{d}-[-A¹⁵-]ₑ-R¹² stands for unsubstituted phenyl; in the presence of a metathesis catalyst.

14. Process according to Claim 13, **characterised in that** the metathesis catalyst is a ruthenium/alkylidene complex.

15. Process according to at least one of Claims 13 and 14 including, after the metathesis reaction step, as a further reaction step, a reduction reaction for conversion of the pyran derivative of the formula I in which W denotes -C(=O)- and Z¹³, Z¹⁴ and Z¹⁵ do not denote -C(O)-, into a pyran derivative of the formula I, in which
W denotes -CH₂-;
R¹¹ denotes H, an alkyl radical having 1 to 15 carbon atoms which is unsubstituted or mono- or polysubstituted, identically or differently, by halogen or -CN, where, in addition, one or more CH₂ groups in this radical may be replaced by -C≡C-, -CH=CH-, -O- and/or -S- in such a way that heteroatoms (O, S) are not linked directly to one another;
R¹² denotes H, halogen, -CN, -NCS, aralkyl, -O-aralkyl or an alkyl radical having 1 to 15 carbon atoms which is unsubstituted or mono- or polysubstituted, identically or differently, by halogen or -CN, where, in addition, one or more CH₂ groups in this radical may be replaced by -C≡C-, -CH=CH-, -O- and/or -S- in such a way that heteroatoms (O, S) are not linked directly to one another;
Z¹³, Z¹⁴ and Z¹⁵ each, independently of one another, denote a single bond, -CH₂CH₂-, -CF₂CH₂-, -CH₂CF₂-, -CF₂CF₂-, -CH=CH-, -C≡C-, -CH₂O- or -CF₂O-;
and a, b, c, d, e, Z¹¹, Z¹², A¹¹, A¹², A¹³, A¹⁴ and A¹⁵ are as defined in Claim 1.

16. Process according to at least one of Claims 13 to 15 including, after the metathesis reaction step and after any reduction reaction step to be carried out, as a further reaction step, a catalytic hydrogenation to give a pyran derivative of the general formula III: 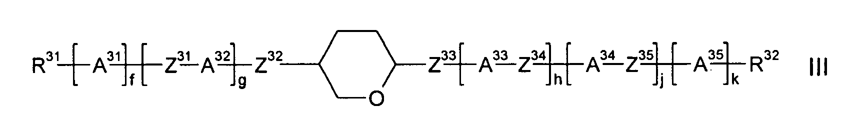 in which
f, g, h, j and k are each, independently of one another, 0 or 1;
R³¹ denotes H, an alkyl radical having 1 to 15 carbon atoms which is unsubstituted or mono- or polysubstituted, identically or differently, by halogen or -CN, where, in addition, one or more CH₂ groups in this radical may be replaced by -O-, -S-, -C(O)-O- and/or -O-C(O)- in such a way that heteroatoms (O, S) are not linked directly to one another;
R³² denotes H, halogen, -CN, -NCS, aralkyl or an alkyl radical having 1 to 15 carbon atoms which is unsubstituted or mono- or polysubstituted, identically or differently, by halogen or -CN, where, in addition, one or more CH₂ groups in this radical may be replaced by -O-, -S-, -C(O)-O- and/or -O-C(O)- in such a way that heteroatoms (O, S) are not linked directly to one another;
Z³¹ and Z³², independently of one another, denote a single bond, -CH₂-, -CH₂CH₂-, -CF₂CH₂-, -CH₂CF₂- or -CF₂CF₂-;
Z³³, Z³⁴ and Z³⁵ each, independently of one another, denote a single bond, -CH₂CH₂-, -CF₂CH₂-, -CH₂CF₂-, -CF₂CF₂-, -CH₂O-, -CF₂O-, -C(O)- or -C(O)-O-;
A³¹ and A³², independently of one another, stand for 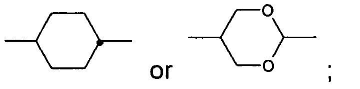
A³³ and A³⁴, independently of one another, stand for 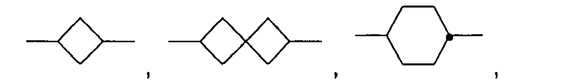 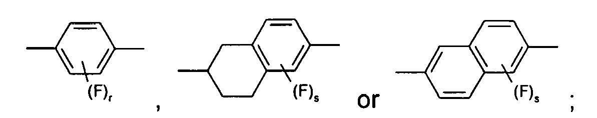
A³⁵ stands for 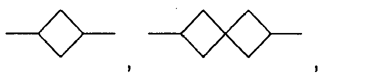 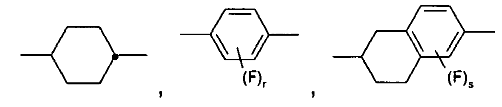 or 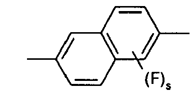 or
A³⁵-R³² together stand for 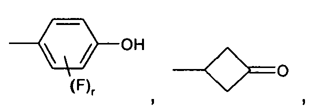 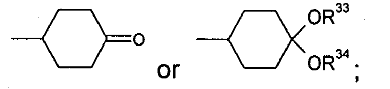 or
Z³³-[-A³³-Z³⁴-]ₕ-[-A³⁴-Z³⁵-]ⱼ-[-A³⁵-]ₖ-R³² stands for 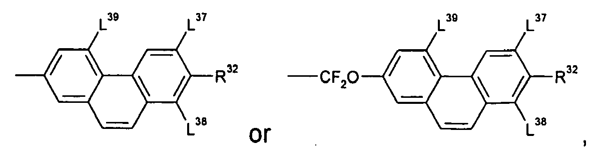 where R³² is as defined above and L³⁷, L³⁸ and L³⁹, independently of one another, denote H or F;
r is 0, 1, 2, 3 or 4;
s is 0, 1, 2 or 3;
R³³ and R³⁴, independently of one another, denote an alkanyl radical having 1 to 7 carbon atoms or together stand for an alkylene bridge having 2 to 7 carbon atoms;
with the proviso
that, in the case of direct linking of Z³³ and R³² to give -Z³³-R³², R³² denotes H, aralkyl or alkanyl if Z³³ denotes -C(=O)-, R³² denotes aralkyl or alkanyl if Z³³ denotes -C(=O)-O-, and Z³³ does not denote -CH₂O- or -CF₂O-;
that, in the case of direct linking of Z³⁴ and R³² to give -Z³⁴-R³², R³² denotes H, aralkyl or alkanyl if Z³⁴ denotes -C(=O)-, R³² denotes aralkyl or alkanyl if Z³⁴ denotes -C(=O)-O-, and Z³⁴ does not denote -CH₂O- or -CF₂O-;
that, in the case of direct linking of Z³⁵ and R³² to give -Z³⁵-R³², R³² denotes H, aralkyl or alkanyl if Z³⁵ denotes -C(=O)-, R³² denotes aralkyl or alkanyl if Z³⁵ denotes -C(=O)-O-, and Z³⁵ does not denote -CH₂O- or -CF₂O-;
that R³¹ does not denote H if simultaneously f and g are both zero and Z³² denotes a single bond.

17. Process according to at least one of Claims 13 to 16 including, before the metathesis reaction step, as a further reaction step, the reaction of a compound of the formula IV with a homoallyl compound of the formula V 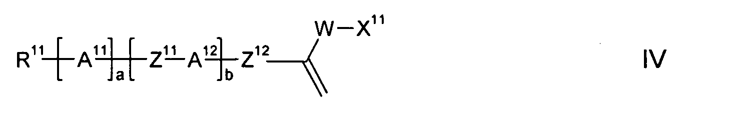 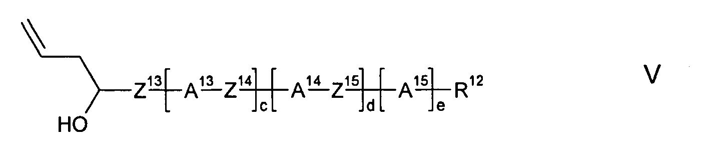 with formation of the compound of the formula II 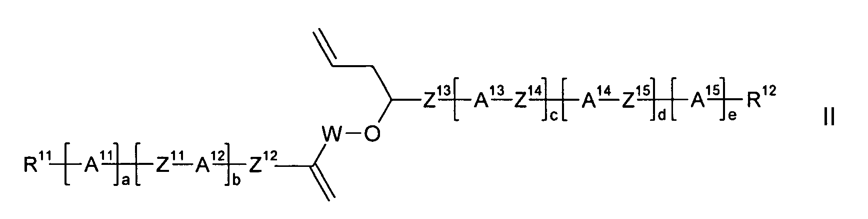 where X¹¹ denotes a leaving group and a, b, c, d, e, W, R¹¹, R¹², Z¹¹, Z¹², Z¹³, Z¹⁴, Z¹⁵, A¹¹, A¹², A¹³, A¹⁴ and A¹⁵ in the formulae II, IV and V have the same meaning as in Claim 1 for formula I.

18. Process according to Claim 17, **characterised in that**
W denotes -CH₂-; and
X¹¹ denotes chlorine, bromine, iodine or a sulfonic acid radical.

19. Allyl compound of the formula IV-Bb 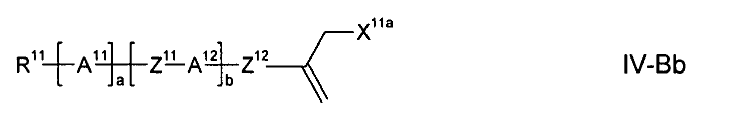 where
a is zero and b is 1 or a is 1 and b is zero or a and b are both 1;
X^{11a} denotes chlorine, bromine, iodine, a sulfonic acid radical, -OH, alkoxy or O-aralkyl
or
-X^{11a} stands for =O, in the sense of aldehydes;
R¹¹, Z¹¹ and Z¹² are as defined in Claim 1; and
A¹¹ and A¹² are as defined in Claim 1 and preferably denote 1,4-cyclohexylene.

20. Use of a pyran derivative of the formula I according to Claim 1 as constituent of a liquid-crystalline medium, in particular in an electro-optical display device.

## Revendications

1. Composé de la formule générale I 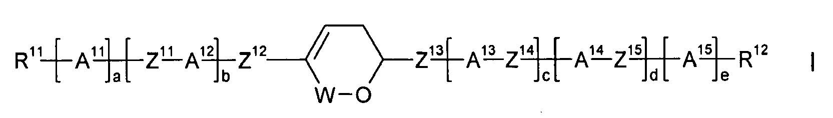 dans laquelle
a, b, c, d et e sont, chacun indépendamment les uns des autres, 0 ou 1;
W signifie -CH₂- ou -C(=O)- ;
R¹¹ signifie H, un radical alkyle comportant de 1 à 5 atomes de carbone, qui est non substitué ou mono- ou polysubstitué, de manière identique ou différente, par halogène ou -CN, où, en plus, un ou plusieurs groupes CH₂ dans ce radical peuvent être remplacés par -C≡C-, -CH=CH-, -O-, -S-, -C(O)-O- et/ou -O-C(O)- de telle sorte que des hétéroatomes (O, S) ne soient pas liés directement les uns aux autres ;
R¹² signifie H, halogène, -CN, -NCS, aralkyle, -O-aralkyle ou un radical alkyle comportant de 1 à 15 atomes de carbone, qui est non substitué ou mono- ou polysubstitué, de manière identique ou différente, par halogène ou -CN, où, en plus, un ou plusieurs groupes CH₂ dans ce radical peuvent être remplacés par -C≡C-, -CH=CH-, -O-, -S-, -C(O)-O- et/ou -O-C(O)- de telle sorte que des hétéroatomes (O, S) ne soient pas liés directement les uns aux autres ;
Z¹¹ signifie une liaison simple, -CH₂-, -CH₂CH₂-, -CF₂CH₂-, -CH₂CF₂-, -CF₂CF₂-, -CH=CH- ou -C≡C- ;
Z¹² signifie une liaison simple, -CH₂-, -CH₂CH₂-, -CF₂CH₂-, -CH₂CF₂- ou -CF₂CF₂- ;
Z¹³, Z¹⁴ et Z¹⁵ chacun indépendamment les uns des autres, signifient une liaison simple, -CH₂CH₂-, -CF₂CH₂-, -CH₂CF₂-, -CF₂CF₂-, -CH=CH-, -C≡C-, -CH₂O-, -CF₂O-, -C(O)- ou C(O)-O- ;
A¹¹ et A¹² indépendamment l'un de l'autre, représentent 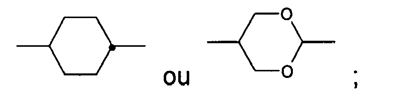
A¹³ et A¹⁴ indépendamment l'un de l'autre, représentent 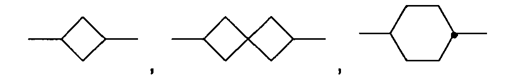 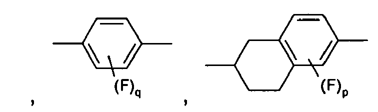 ou 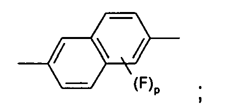
A¹⁵ représente 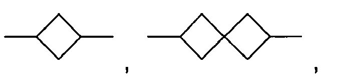 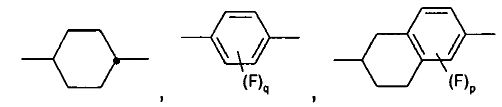 ou 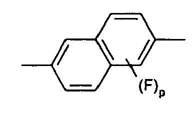 ou
A¹⁵-R¹² représentent ensemble 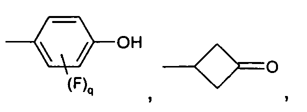 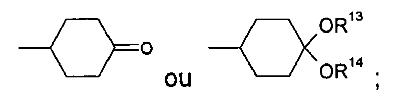 ou
Z¹³-[-A¹³-Z¹⁴-]_{c}-[-A¹⁴-Z¹⁵]_{d}-[-A¹⁵-]ₑ-R¹² représente 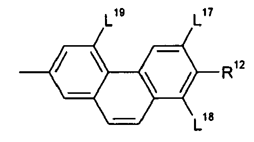 ou 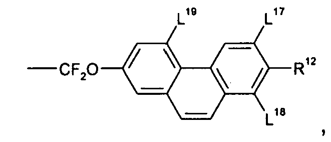 où R¹² est comme défini ci-avant, et L¹⁷, L¹⁸ et L¹⁹, indépendamment les uns des autres, signifient H ou F ;
q est 0, 1, 2, 3 ou 4 ;
p est 0, 1,2 ou 3;
R¹³ et R¹⁴ indépendamment l'un de l'autre, signifient un radical alkanyle comportant de 1 à 7 atomes de carbone ou représentent ensemble un pont alkylène comportant de 2 à 7 atomes de carbone ;
étant entendu que,
dans le cas d'une liaison directe de Z¹³ et de R¹² pour obtenir -Z¹³⁻R¹², R¹² signifie H, aralkyle, alkanyle ou alkényle si Z¹³ signifie -C(=O)-O- ou -C(=O)-, et Z¹³ ne signifie pas -CH₂O- ou -CF₂O- ;
dans le cas d'une liaison directe de Z¹⁴ et de R¹² pour obtenir -Z¹⁴⁻R¹², R¹² signifie H, aralkyle, alkanyle ou alkényle si Z¹⁴ signifie C(=O)-O- ou -C(=O)-, et Z¹⁴ ne signifie pas -CH₂O- ou -CF₂O- ;
dans le cas d'une liaison directe de Z¹⁵ et de R¹² pour obtenir -Z¹⁵⁻R¹², R¹² signifie H, aralkyle, alkanyle ou alkényle si Z¹⁵ signifie -C(=O)-O- ou -C(=O)-, et Z¹⁵ ne signifie pas -CH₂O- ou -CF₂O- ;
R¹¹ ne signifie pas H si simultanément a et b sont tous deux à zéro et Z¹² signifie une liaison simple ;
R¹¹ ne signifie pas CH₃ si simultanément a et b sont tous deux à zéro, Z¹² et Z¹³ signifient chacun une liaison simple, W signifie -CH₂- et -[-A¹³-Z¹⁴-]_{c}-[-A¹⁴-Z¹⁵-]_{d}-[-A¹⁵-]ₑ-R¹² représente un phényle non substitué.

2. Composé selon la revendication 1, **caractérisé en ce que**
a+b+c+d+e ≤ 3.

3. Composé selon au moins l'une des revendications précédentes, **caractérisé en ce que**
W signifie -C(=O)-.

4. Composé selon au moins l'une des revendications 1 et 2, **caractérisé en ce que**
W signifie -CH₂-.

5. Composé selon au moins l'une des revendications précédentes, **caractérisé en ce que**
Z¹² signifie une liaison simple.

6. Composé selon au moins l'une des revendications précédentes, **caractérisé en ce que**
b est 1 ;
Z¹¹ signifie une liaison simple ; et
A¹² représente

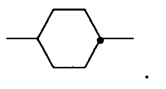

7. Composé selon au moins l'une des revendications précédentes, **caractérisé en ce que**
a est 1 ; et
A¹¹ représente

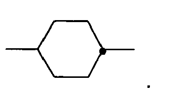

8. Composé selon au moins l'une des revendications précédentes, **caractérisé en ce que**
Z¹³ signifie une liaison simple, -C(O)-O- ou -CF₂O-.

9. Composé selon au moins l'une des revendications précédentes,
**caractérisé en ce que**
e est 1 ;
A¹⁵ représente 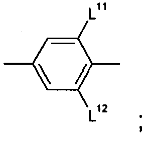 et
L¹¹ et L¹² indépendamment l'un de l'autre, signifient H ou F.

10. Composé selon au moins l'une des revendications 1 à 8, **caractérisé en ce que**
e est 1 ;
A¹⁵-R¹² représente 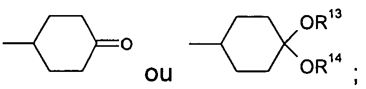 et
R¹³ et R¹⁴ sont comme défini selon la revendication 1.

11. Composé selon au moins l'une des revendications précédentes, **caractérisé en ce que**
c est 1 ;
Z¹⁴ signifie une liaison simple, -C(O)-O- ou -CF₂O- ;
A¹³ représente 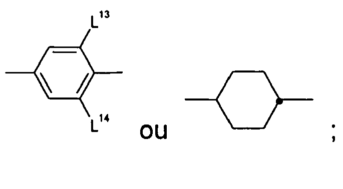 et
L¹³ et L¹⁴ indépendamment l'un de l'autre, représentent H ou F.

12. Composé selon au moins l'une des revendications précédentes, **caractérisé en ce que**
d est 1 ;
Z¹⁵ signifie -CF₂O- ;
A¹⁴ représente 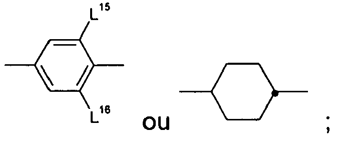 et
L¹⁵ et L¹⁶ indépendamment l'un de l'autre, signifient H ou F.

13. Procédé pour la préparation de dérivés de pyranne incluant une étape de processus qui inclut une réaction de métathèse à fermeture d'anneau d'un composé de la formule générale II 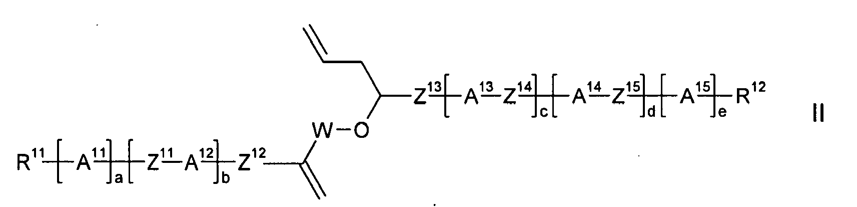 pour obtenir un dérivé de pyranne de la formule I 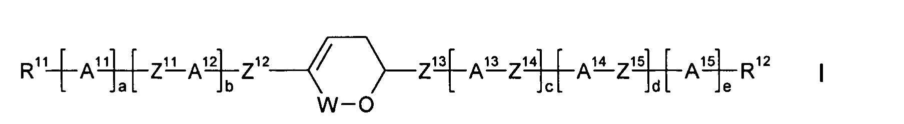 où a, b, c, d, e, W, R¹¹, R¹², Z¹¹, Z¹², Z¹³, Z¹⁴, Z¹⁵, A¹¹, A¹², A¹³, A¹⁴ et A¹⁵ dans les formules I et II présentent la même signification que selon la revendication 1 pour la formule I ;
étant entendu que,
dans le cas d'une liaison directe de Z¹³ et de R¹² pour obtenir -Z¹³⁻R¹², R¹² signifie H, aralkyle ou alkanyle si Z¹³ signifie -C(=O)-,
R¹² signifie aralkyle ou alkanyle siZ¹³ signifie -C(=O)-O-, et Z¹³ ne signifie pas -CH₂O- ou -CF₂O- ;
dans le cas d'une liaison directe de Z¹⁴ et de R¹² pour obtenir -Z¹⁴⁻R¹², R¹² signifie H, aralkyle ou alkanyle si Z¹⁴ signifie -C(=O)-,
R¹² signifie aralkyle ou alkanyle si Z¹⁴ signifie -C(=O)-O-, et Z¹⁴ ne signifie pas -CH₂O- ou -CF₂O- ;
dans le cas d'une liaison directe de Z¹⁵ et de R¹² pour obtenir -Z¹⁵-R¹², R¹² signifie H, aralkyle ou alkanyle si Z¹⁵ signifie -C(=O)-, R¹² signifie aralkyle ou alkanyle si Z¹⁵ signifie -C(=O)-O-, et Z¹⁵ ne signifie pas -CH₂O- ou -CF₂O- ;
R¹¹ ne signifie pas H si a et b sont tous deux simultanément à zéro et Z¹² signifie une liaison simple ;
R¹¹ ne signifie pas CH₃ si simultanément a et b sont tous deux à zéro, Z¹² et Z¹³ signifient chacun une liaison simple, W signifie -CH₂- et -[-A¹³-Z¹⁴-]_{c}-[-A¹⁴-Z¹⁵-]_{d}-[-A¹⁵-]ₑ-R¹² représente un phényle non substitué ;
en présence d'un catalyseur de métathèse.

14. Procédé selon la revendication 13, **caractérisé en ce que** le catalyseur de métathèse est un complexe ruthénium/alkylidène.

15. Procédé selon au moins l'une des revendications 13 et 14 , incluant, après l'étape de réaction de métathèse, en tant qu'étape de réaction supplémentaire, une réaction de réduction pour la conversion du dérivé de pyranne de la formule I où W signifie -C(=O)- et Z¹³, Z¹⁴ et Z¹⁵ ne signifient pas -C(O)-, selon un dérivé de pyranne de la formule l, dans laquelle
W signifie -CH₂- ;
R¹¹ signifie H, un radical alkyle comportant de 1 à 15 atomes de carbone, qui est non substitué ou mono- ou polysubstitué, de manière identique ou différente, par halogène ou -CN, où, en plus, un ou plusieurs groupes CH₂ dans ce radical peuvent être remplacés par -C≡C-, -CH=CH-, -O-, et/ou -S- de telle sorte que des hétéroatomes (O, S) ne soient pas liés directement les uns aux autres ;
R¹² signifie H, halogène, -CN, -NCS, aralkyle, -O-aralkyle ou un radical alkyle comportant de 1 à 15 atomes de carbone, qui est non substitué ou mono- ou polysubstitué, de manière identique ou différente, par halogène ou -CN, où, en plus, un ou plusieurs groupes CH₂ dans ce radical peuvent être remplacés par -C≡C-, -CH=CH-, -O-, et/ou -S- de telle sorte que des hétéroatomes (O, S) ne soient pas liés directement les uns aux autres ;
Z¹³, Z¹⁴ et Z¹⁵ chacun indépendamment les uns des autres, signifient une liaison simple, -CH₂CH₂-, -CF₂CH₂-, -CH₂CF₂-, -CF₂CF₂-, -CH=CH-, -C≡C-, -CH₂O-, -CF₂O- ;
et a, b, c, d, e, Z¹¹, Z¹², A¹¹, A¹², A¹³, A¹⁴ et A¹⁵ sont comme défini selon la revendication 1.

16. Procédé selon au moins l'une des revendications 13 à 15 incluant, après l'étape de réaction de métathèse et après une quelconque étape de réaction de réduction destinées à être mises en oeuvre, en tant qu'étape de réaction supplémentaire, une hydrogénation catalytique pour obtenir un dérivé de pyranne de la formule générale III : 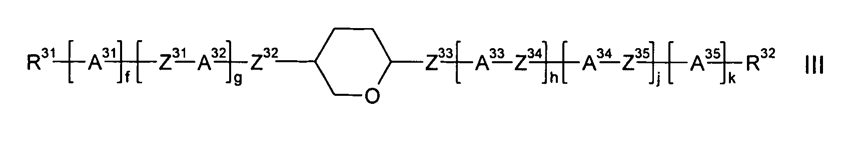 dans laquelle
f, g, h, j et k sont, chacun indépendamment les uns des autres, 0 ou 1 ;
R³¹ signifie H, un radical alkyle comportant de 1 à 15 atomes de carbone, qui est non substitué ou mono- ou polysubstitué, de manière identique ou différente, par halogène ou -CN, où, en plus, un ou plusieurs groupes CH₂ dans ce radical peuvent être remplacés par -O-, -S-, -C(O)-O- et/ou -O-C(O)- de telle sorte que des hétéroatomes (O, S) ne soient pas liés directement les uns aux autres ;
R³² signifie H, halogène, -CN, -NCS, aralkyle ou un radical alkyle comportant de 1 à 15 atomes de carbone, qui est non substitué ou mono- ou polysubstitué, de manière identique ou différente, par halogène ou -CN, où, en plus, un ou plusieurs groupes CH₂ dans ce radical peuvent être remplacés par -O-, -S-, -C(O)-O- et/ou -O-C(O)- de telle sorte que des hétéroatomes (O, S) ne soient pas liés directement les uns aux autres,
Z³¹ et Z³² indépendamment l'un de l'autre, signifient une liaison simple, -CH₂-, -CH₂CH₂-, -CF₂CH₂-, -CH₂CF₂- ou -CF₂CF₂- ;
Z³³, Z³⁴ et³⁵ chacun indépendamment les uns des autres, signifient une liaison simple, -CH₂CH₂-, -CF₂CH₂-, -CH₂CF₂-, -CF₂CF₂-, -CH₂O-, -CF₂O-, -C(O)- ou -C(O)-O- ;
A³¹ et A³² indépendamment l'un de l'autre représentent 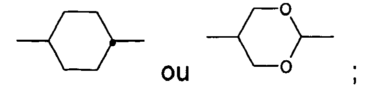
A³³ et A³⁴ indépendamment l'un de l'autre, représentent 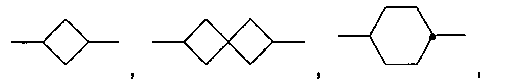 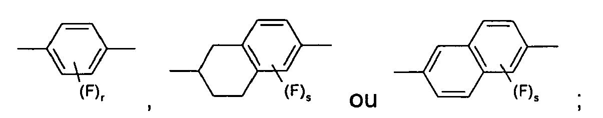
A³⁵ représente 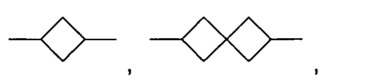 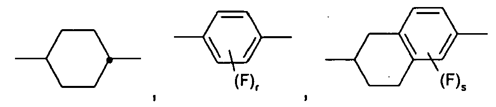 ou 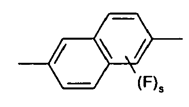 ou
A³⁵-R³² représentent ensemble 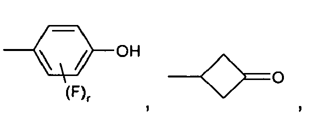 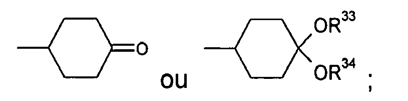 ou
Z³³-[-A³³-Z³⁴-]ₕ-[-A³⁴-Z³⁵-]ⱼ-[-A³⁵-]ₖ-R³² représente 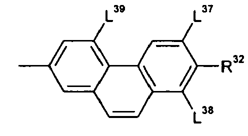 ou 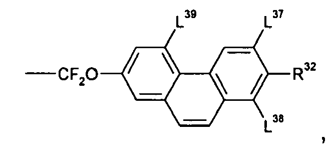 où R³² est comme défini ci-avant et L³⁷, L³⁸ et L³⁹, indépendamment les uns des autres, signifient H ou F ;
r est 0, 1, 2, 3 ou 4 ;
s est 0, 1, 2 ou 3 ;
R³³ et R³⁴ indépendamment l'un de l'autre, signifient un radical alkanyle comportant de 1 à 7 atomes de carbone ou représentent ensemble un pont alkylène comportant de 2 à 7 atomes de carbone ;
étant entendu que
dans le cas d'une liaison directe de Z³³ et de R³² pour obtenir -Z³³-R³², R³² signifie H, aralkyle ou alkanyle si Z³³ signifie -C(=O)-, R³² signifie aralkyle ou alkanyle si Z³³ signifie -C(=O)-O-, et Z³³ ne signifie pas -CH₂O- ou -CF₂O- ;
dans le cas d'une liaison directe de Z³⁴ et de R³² pour obtenir -Z³⁴-R³², R³² signifie H, aralkyle ou alkanyle si Z³⁴ signifie -C(=O)-, R³² signifie aralkyle ou alkanyle si Z³⁴ signifie -C(=O)-O-, et Z³⁴ ne signifie pas -CH₂O- ou -CF₂O- ;
dans le cas d'une liaison directe de Z³⁵ et de R³² pour obtenir -Z³⁵-R³², R³² signifie H, aralkyle ou alkanyle si Z³⁵ signifie -C(=O)-, R³² signifie aralkyle ou alkanyle si Z³⁵ signifie -C(=O)-O-, et Z³⁵ ne signifie pas -CH₂O- ou -CF₂O- ;
R³¹ ne signifie pas H si simultanément f et g sont tous deux à zéro et Z³² signifie une liaison simple.

17. Procédé selon au moins l'une des revendications 13 à 16, incluant, avant l'étape de réaction de métathèse, en tant qu'étape de réaction supplémentaire, la réaction d'un composé de la formule IV avec un composé homoallyle de la formule V 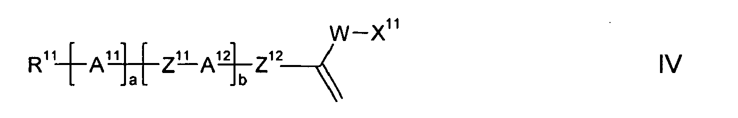 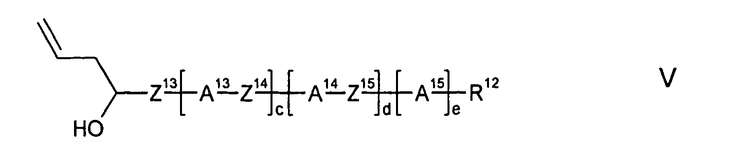 moyennant la formation du composé de la formule II 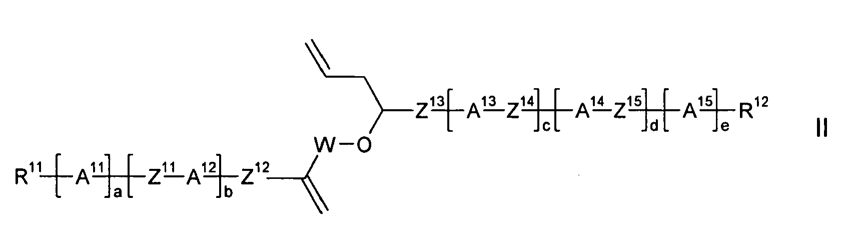 où X¹¹ signifie un groupe partant et a, b, c, d, e, W, R¹¹, R¹², Z¹¹, Z¹², Z¹³, Z¹⁴, Z¹⁵, A¹¹, A¹², A¹³, A¹⁴ et A¹⁵ selon les formules II, IV et V présentent la même signification que selon la revendication1 pour la formule I.

18. Procédé selon la revendication 17, **caractérisé en ce que** :
W signifie -CH₂- ; et
X¹¹ signifie chlore, brome, iode ou un radical acide sulfonique.

19. Composé allyle de la formule IV-Bb 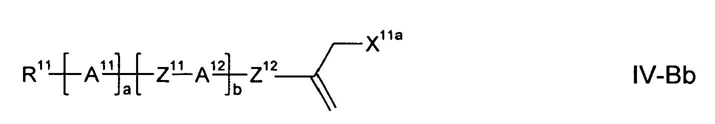 dans laquelle
a est zéro et b est 1 ou a est 1 et b est zéro ou a et b sont tous deux 1 ;
X^{11a} signifie chlore, brome, iode, un radical acide sulfonique, -OH, alkoxy ou O-aralkyle
ou
-X^{11a} représente =O, dans le sens des aldéhydes ;
R¹¹, Z¹¹ et Z¹² sont comme défini selon la revendication 1 ; et
A¹¹ et A¹² sont comme défini selon la revendication 1 et signifient de préférence 1,4-cyclohexylène.

20. Utilisation d'un dérivé de pyranne de la formule I selon la revendication 1 en tant que constituant d'un milieu de cristal liquide, en particulier dans un dispositif d'affichage électro-optique.
